# EUROPEAN PATENT APPLICATION

(11) **EP 4 389 746 A2**
(43) Date of publication of application: **26.06.2024**
(21) Application number: 22215635.8
(22) Date of filing: 21.12.2022
(51) Int. Cl.: C07D 471/04, C07D 487/04, A61P 25/02, A61K 31/5025

(54) **P2X3 RECEPTOR ANTAGONISTS**

(71) Applicant: Recordati Industria Chimica E Farmaceutica SPA, 20148 Milano (IT)
(72) Inventor: GRAZIANI, Davide, 20148 Milano (IT); RIVA, Carlo, 20148 Milano (IT); PIRONA, Lorenza, 20148 Milano (IT); MENEGON, Sergio, 20148 Milano (IT); TAZZARI, Valerio, 20148 Milano (IT); FRIGERIO, Fabio, 20148 Milano (IT)
(74) Representative: Houghton, Mark Phillip

(57) **Abstract**

This invention relates to compounds of formula I; 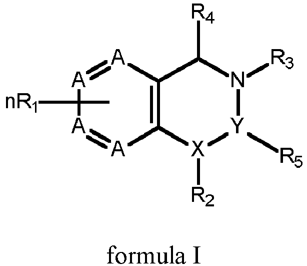 and their use as antagonists of P2X₃ and P2X_{2/3} receptor activity, pharmaceutical compositions comprising such compounds, and methods of treatment therewith. Compounds of the invention can be used for the treatment and/or prevention of pain and chronic pain and tolerance to analgesic, respiratory disorders and dysfunctions, and treatment of overactive bladder, bladder pain syndrome, dysuria and in general in genitourinary diseases, cardiovascular disorders and more in general for the potential treatment of visceral organ diseases and disorders characterized by the involvement of P2X₃ and P2X_{2/3} receptors.

## Description

### FIELD OF THE INVENTION

This application is related to the Patent Application Number PCT/EP2019/068681 filed the 11 July 2019, with priority data 12 July 2018, which describe the invention relates to fused heterocyclic derivatives, including 4-imino-1H-pyrido[3,2-d]pyrimidin-2-one, 8-imino-5H-pyrimido[5,4-c]pyridazin-6-one and 7H-pyrido[2,3-d]pyridazin-8-imine derivatives, and their use as antagonists of P2X₃ and P2X_{2/3} receptor activity and their pharmaceutical compositions comprising such compounds, and methods of treatment therewith. Some of the described compounds are characterized by high selectivity versus P2X_{2/3} receptor, which will result useful for the limitation of some of the reported adverse effects linked to this class of molecules.

### BACKGROUND TO THE INVENTION

Adenosine-5'-triphosphate (ATP) acts as an extracellular signalling molecule after release from healthy or damaged cells (G. Burnstock, "Discovery of purinergic signalling, the initial resistance and current explosion of interest", Br. J. Pharmacol. (2012), No. 167, pp.238-55) on two different classes of purinergic receptors: the ionotropic P2X receptors and the G-protein-coupled P2Y receptors.

P2X receptors are ion channels resulting by seven P2X₁₋₇ subunits association as homo- or hetero- trimers (R.A. North, "Molecular physiology of P2X receptors", Physiol. Rev. (2002), No.82, pp. 1013-67).

The homo trimer P2X₃ receptor and the hetero-trimer P2X_{2/3} receptor are predominantly localized on small- to medium-diameter C- and Aδ-fiber sensory neurons within the dorsal root ganglion and cranial sensory ganglia, and on their peripheral nerve terminals in tissues comprising skin, joints, and viscera. The P2X₃ receptor is also present on central projections of sensory neurons within the dorsal horn of the spinal cord and in the brainstem, where it plays a role in augmenting the release of glutamate and substance P. Because of its specific and limited location, the P2X₃ receptor subtype thus offers unique opportunity to investigate sensory and nociceptive mechanisms (C. Volontè, G. Burnstock, "P2X3 receptor - a novel 'CASKade' of signalling", J. Neurochem. (2013), No. 126, pp. 1-3).

The P2X₃ receptors is also involved in many conditions where pain symptoms originate from chronic sensitization of peripheral afferent pathways (e.g., overactive bladder, irritable bowel syndrome, chronic itch and cough, airways hyperreactivity).

"Cough is a forced expulsive manoeuvre, usually against a closed glottis, which is associated with a characteristic sound" which serves as a crucial protective reflex for upper airway, but can be interpreted as sign of disease. (EAACI Position Paper on assessment of cough in the workplace; G. Moscato et al. Allergy 69 (2014) 292-304) Chronic cough (CC), is defined as a cough lasting more than 8 weeks, is associated with different medical conditions (asthma, gastroesophageal reflux disease (GERD), non-asthmatic eosinophilic bronchitis, and upper-airway cough syndrome) and affects 8-10% of population of development countries. Cough that persists despite guideline-based treatment is considered refractory to the presumed associated common and uncommon condition. The management of the patients affected from refractory chronic cough (RCC) is difficult and their treatment response often limited, and refractory chronic cough (RCC) represents a relevant unmet medical need. (Management of chronic refractory cough; Peter G Gibson, Anne E Vertigan, BMJ 2015;351).

The afferent fibres that evoke cough are almost completely confined to the vagus nerve and preclinical studies suggest key roles for both C fibres (chemoreceptors) and Aδ fibres (mechanoreceptors). P2X₃ receptors are ATP-gated ion channels selectively localized on populations of primary afferent nerves arising from both cranial and dorsal root ganglia.

In guinea pigs, vagal C fibres innervating the airways express P2X₃ receptors, and can be activated by ATP released into the airways. Moreover, when guinea pigs are exposed to ATP and histamine or citric acid aerosols, cough responses to these tussive stimuli are increased. P2X3-receptor antagonists have been proved to reduce this effect. The P2X₃R is also involved in many conditions where pain symptoms originate from chronic sensitization of peripheral afferent pathways (e.g., overactive bladder, irritable bowel syndrome, chronic itch and cough, airways hyperreactivity).

P2X₃ ion channel receptors are expressed by a subpopulation of small-diameter primary nociceptors in the trigeminal nervous system and when activated by adenosine triphosphate (ATP) they can evoke a sensation of burning pain. P2X₃ receptors, coupled with the transient receptor potential subfamily member V 1 (TRPV1) ion channel, and of nerve growth factor NGF are upregulated in Burning Mouth Syndrome. For this reason, compounds acting on the P2X₃ receptors may have a potential role in the treatment of Burning Mouth Syndrome ("Burning Mouth Syndrome: Aetiopathogenesis and Principles of Management", L. Feller, J. Fourie, M. Bouckaert, R. A. G. Khammissa, R. Ballyram, and J. Lemmer, Pain Research and Management, Vol.2017, Article ID 1926269, 6 pages).

Daily systemic injection of an orthosteric P2X₃ receptor antagonist attenuated the morphine-induced antinociceptive tolerance to von Frey and thermal stimuli, in comparison with morphine alone, showing that a P2X₃ receptor antagonist is able to reverse morphine tolerance and it may be a new therapeutic target in the prevention of tolerance to morphine-induced antinociception ("Blockade and reversal of spinal morphine tolerance by P2X₃ receptor antagonist", Ma X and Xu T, Xu H, Jiang W, Behavioural Pharmacology, (2015), Vol.26(3), pp.260-267). P2X₃ receptor antagonist morphine tolerance attenuation may be attributed to down-regulation of N-methyl-D-aspartate receptor subunits NR1 and NR2B expression in the synaptosomal membrane and inhibition of excitatory amino acids release in morphine-tolerant rats ("Purinergic P2X Receptor Regulates N-Methyl-D-aspartate Receptor Expression and Synaptic Excitatory Amino Acid Concentration in Morphine-tolerant Rats", Yueh-Hua Tai, Pao-Yun Cheng, Ru-Yin Tsai, Yuh-Fung Chen, Chih-Shung Wong, Anesthesiology, (2010), Vol. 113(5), pp. 1163-75).

Currently, the carotid body is under consideration as a therapeutic target for hypertension because sympathoexcitatory response is potentiated in hypertensive rats and human. Moreover, the aberrant signalling that contributes to high blood pressure may be normalized by carotid body denervation in rats. P2X₃ receptor mRNA expression is upregulated in chemoreceptive petrosal ganglion neurons in hypertensive rats. These neurons generate both tonic drive and hyperreflexia in hypertensive rats, and both phenomena are normalized by P2X₃ receptor antagonists. Antagonism of P2X₃ receptors also reduces arterial pressure and basal sympathetic activity and normalizes carotid body hyperreflexia in conscious rats with hypertension. The purinergic receptors present in the carotid body can be considered as a potential new target for the control of human hypertension (Wioletta Pijacka, Davi J A Moraes, Laura E K Ratcliffe, Angus K Nightingale, Emma C Hart, Melina P da Silva, Benedito H Machado, Fiona D McBryde, Ana P Abdala, Anthony P Ford & Julian F R Paton).

Endometriosis is a common gynecological disease characterized by the presence of functional endometrium outside the uterine cavity, resulting in dysmenorrhea, dyspareunia, pelvic pain, and infertility, with lack of effective clinical treatment (Strathy JH, Molgaard CA, Coulam CB, Melton LJ 3rd. "Endometriosis and infertility: a laparoscopic study of endometriosis among fertile and infertile women", Fertility and sterility, (1982), Vol.38(6), pp.667-72). Endometriosis is considered as a kind of inflammatory and neuropathic pain with increasing evidence indicating the importance of adenosine triphosphate (ATP) and P2X₃ receptors in endometriosis pain sensitization and transduction. P2X₃ are expressed on endometrial epithelial cells and on endometrial stromal cells. P2X₃ are overexpressed in the endometriosis endometrium and endometriotic lesions and both significantly higher as compared with control endometrium, and both positively correlated with pain, and with the severity of pain in women affected with endometriosis. The expression levels of phosphorylated ±ERK (p-ERK), phosphorylated-cAMP-response element binding protein (p-CREB), and P2X₃ in endometriotic stromal cells (ESCs) were all significantly increased in comparison to the initial levels after treated with interleukin (IL)-1β or adenosine triphosphate (ATP), respectively, and did not increase after the ESCs were pre-treated with ERK_{1/2} inhibitor. P2X₃ receptor may represent a highly innovative target for the non-hormonal treatment of endometriosis ("P2X3 receptor involvement in endometriosis pain via ERK signaling pathway", Shaojie Ding, Libo Zhu, Yonghong Tian, Tianhong Zhu, Xiufeng Huang, Xinmei Zhang; PLoS ONE, (2017), Vol. 12(9): e0184647).

P2X₃ receptors present in visceral afferent fibers undergo sensitization following visceral inflammation. In a rat model of esophagitis, the expression of P2X₃ receptor was discovered significantly upregulated both in the vagus nerve and spinal afferent nerve. Therefore, P2X₃ ion channel receptor antagonists may represent a potential target for the treatment of oesophageal hypersensitivity (Pathophysiological Role of Purinergic P2X Receptors in Digestive System Diseases; An Q, Yue G, Yang X, Lou J, Shan W, Ding J, Jin Z, Hu Y, Du Q, Liao Q, Xie R and Xu J (2021) Front. Physiol. 12:781069).

Several P2X receptor subtypes, including P2X₂, P2X₃, P2X₄, and P2X₇, have been shown to play diverse roles in the pathogenesis of central pain including the mediation of fast transmission in the peripheral nervous system and modulation of neuronal activity in the central nervous system. P2X₃ receptors play a significant role in neuropathic and inflammatory pain. Long-lasting allodynia that is produced by intrathecal administration of ATP likely occurs through P2X_{2/3} receptors. Spinal P2X₂ and P2X₃ receptors have been reported to be involve in neuropathic pain in a mouse model of chronic constriction injury ("Nociceptive transmission and modulation via P2X receptors in central pain syndrome.", Kuan, Y. H., and Shyu, B. C. Mol. Brain (2016), Vol.9, pp.58). P2X₃ receptors show a combination of fast desensitization onset and slow recovery. P2X₃ receptors represent an attractive target for development of new analgesic drugs via promotion of desensitization aimed at suppressing chronic pain, such as: Inflammatory and Neuropathic Pain, Migraine and Trigeminal Pain, and Cancer Pain ("Desensitization properties of P2X3 receptors shaping pain signalling, Rashid Giniatullin and Andrea Nistri", Front. Cell. Neurosci., (2013), Vol.7, pp.245).

### SUMMARY OF THE INVENTION

The invention provides a compound according to general formula I: 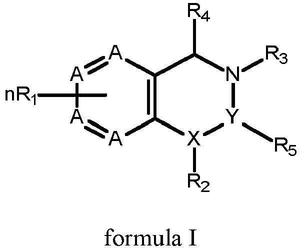 or an enantiomer, diastereomer, N-oxide, or a pharmaceutically acceptable salt or combinations thereof, wherein:
each A independently represents an atom selected from C, N, S or O;
X and Y are selected from C and N atoms, wherein the unit X-Y represents either a N-C group, or a C=N group respectively;
each R₁ independently represents hydrogen, a halogen atom, or an, optionally substituted, hydroxy, carbonyl, carboxyl, amino, amido, C₁-C₆ alkyl or C₁-C₆ alkoxy group, an, optionally substituted, mono-, bi- or tricyclic C₆-C₁₄ aryl group or an, optionally substituted, mono-, bi- or tricyclic C₁-C₁₃ heterocyclic group containing from 1 to 5 heteroatoms selected from N, O or S;
R₂ represents hydrogen or an, optionally substituted, C₁-C₆ alkyl group, C₁-C₆ alkoxy group, C₄-C₁₄ arylalkyl group, C₄-C₁₄ heteroarylalkyl group, C₃-C₇ cycloalkyl group, a mono-, bi- or tricyclic C₆-C₁₄ aryl group or a mono-, bi- or tricyclic C₁-C₁₃ heterocyclic group containing from 1 to 5 heteroatoms selected from N, O or S;
groups R₃ and R₄, or alternatively groups R₃ and R₅, are linked to each other to form a five- or six-membered heterocyclic ring containing from 2 to 3 heteroatoms atoms selected from N, O and S, optionally substituted with one or more groups nR₆, with the proviso that the remainder of R₄ or R₅ not linked with group R₃ to form the heterocyclic ring is absent, or is an atom independently selected from N, O or S which is double-bonded directly to the X-Y containing ring;
each R₆ independently represents hydrogen, a halogen atom selected from F, Cl, Br or I; or an, optionally substituted, carbonyl, oxo, C₁-C₆ alkyl, C₁-C₆ alkoxy or C₃-C₇ cycloalkyl group, an, optionally substituted, mono-, bi- or tricyclic C₆-C₁₄ aryl group or an, optionally substituted, mono-, bi- or tricyclic C₁-C₁₃ heterocyclic group containing from 1 to 5 heteroatoms selected from N, O or S or alternatively, two R₆ groups are linked to each other to form a group of the formula -(Zp)- wherein p is an integer of from 3 to 5 and each Z independently represents an oxygen atom or an optionally substituted methylene group, provided that no two adjacent Y moieties represent oxygen atoms; and
n is an integer independently selected from 0 to 3.

Preferably, compounds of the invention can be used for the treatment and/or prevention of pain and chronic pain and tolerance to analgesic, respiratory disorders and dysfunctions, and treatment of overactive bladder, bladder pain syndrome, dysuria and in general in genitourinary diseases, cardiovascular disorders and more in general for the potential treatment of visceral organ diseases and disorders characterized by the involvement of P2X₃ and P2X_{2/3} receptors.

Preferably, the optional substituents are independently selected from the group consisting of halogen atoms, C₁-C₆ alkyl, C₁-C₆ alkoxy, hydroxy, mercapto, nitro, cyano, oxo, halo(C₁-C₆)alkyl, halo(C₁-C₆)alkoxy, C₁-C₆ alkylthio, C₁-C₆ alkylsulphonyl, C₁-C₆ alkylcarbonyl, sulphamoyl, C₁-C₆ alkylsulphamoyl, di(C₁-C₆)alkylsulphamoyl, (C₁-C₆)alkoxycarbonyl and (C₁-C₆)alkylcarbonyl(C₁-C₆)alkyl groups, and from groups of the formulae -NR*R*, -C(=O)-NR*R*, -D, -O-D, -C(=O)-D, -(CH₂)q-D, -NR**-D, -C(=O)-NR**-D, -NR**C(=O)-D and -O-C(=O)-D wherein each R* independently represents a hydrogen atom or a C₁-C₆ alkyl, C₁-C₆ alkoxy, C₁-C₆ alkylcarbonyl, phenyl or benzyl group, R** represents a hydrogen atom or a C₁-C₆ alkyl group, q is an integer from 1 to 6 and D represents a phenyl group or a C₁-C₈ heterocyclic group containing from 1 to 3 heteroatoms selected from N, O and S; a C₁-C₆ cycloalkyl group; each group D being further optionally substituted with from 1 to 3 groups independently selected from halo, hydroxy, cyano, nitro and C₁-C₆ alkyl, preferably wherein the optional substituents are selected from the groups consisting of halogen atoms and C₁-C₆ alkyl groups.

Preferred compounds of the invention are those in which one of the A groups comprises a heteroatom and the remaining three A groups comprise carbons atoms. A non-limiting example includes the situation where one of the A groups comprises a nitrogen atom, and the remaining three A groups each comprise carbon atoms, such that the heterocyclic ring so formed is a pyridine ring.

Preferred compounds of the invention are those in which two of the A groups comprise heteroatoms and the two remaining A groups comprise carbon atoms. Non-limiting examples include the situation where two of the A groups comprise nitrogen atoms, and the remaining two A groups each comprise carbon atoms, such that the heterocyclic ring so formed is a pyridazine, pyrimidine or pyrazine ring.

Preferred compounds of the invention are those in which three of the A groups comprise heteroatoms and the remaining A group comprises a carbon atom. Non-limiting examples include the situation where three of the A groups comprise nitrogen atoms, and the remaining A group comprises a carbon atom, such that the heterocyclic ring so-formed is a 1,2,3-triazine or 1,2,4-triazine ring.

Preferred compounds of the invention are those in which all four of the A groups comprise heteroatoms. A non-limiting example includes the situation where all four of the A groups comprises nitrogen atoms, such that the heterocyclic ring so-formed is a 1,2,3,4-tetrazine ring.

The skilled person will appreciate that for each of the examples described above, each A group comprising a carbon or other heterocyclic atom, or the heterocyclic ring so formed, may further comprise one or more hydrogen atoms directly attached to one or more of the ring atoms, and/or n groups of R₁ (as defined above) to satisfy the usual rules relating to atomic bonding and valences.

The invention also provides for other such combinations of heteroatoms including, but not limited to, heterocyclic rings formed from each A group being independently represented by an atom selected from C, N, S or O, such that the resulting heterocyclic ring so formed is a piperidine, pyridine, tetrahydropyran, pyran, thiane, thiopyran, morpholine, oxazine, thiomorpholine, thiazine, dioxane, dioxine, dithiane, dithiin, trioxane or trithiane derivative.

Preferred non-limiting examples include the resulting heterocyclic ring so-formed being a 2H-1,2-oxazine, 4H-1,2-oxazine, 6H-1,2-oxazine, 2H-1,3-oxazine, 4H-1,3-oxazine, 6H-1,3-oxazine, 2H-1,4-oxazine, 4H-1,4-oxazine, thiomorpholine, 1,2-thiazine, 1,3-thiazine, 1,4-thiazine, 1,4-dioxane, 1,2-dioxin, 1,4-dioxin, 1,2-dithiane, 1,3-dithiane, 1,4-dithiane, 1,2-dithiin and 1,4-dithiin, 1,2,3-trioxane or 1,2,4-trioxane derivative.

Preferred compounds of the invention are those in which group X-Y represents a N-C group, such that the six-membered central heterocyclic ring so-formed is a pyrimidine ring.

Preferred compounds of the invention are those in which group X-Y represents a C=N group, such that the six-membered central heterocyclic ring so-formed is a pyradizine ring.

Preferred compounds of the invention are those in which group X-Y represents a N-C group, groups R₃ and R₄ are linked to each other to form a five- or six-membered heterocyclic ring containing from 2 to 3 nitrogen heteroatoms atoms, optionally substituted with one or more groups nR₆, as defined above, and R₅ is a carbonyl group.

Preferred compounds of the invention are those in which group X-Y represents a N-C group, and groups R₃ and R₄ are linked to each other to form a five- or six-membered heterocyclic ring containing from 2 to 3 nitrogen heteroatoms atoms (e.g. 2-imidazoline, imidazole, 1,2,4-triazole or pyrimidine), selected from but not limited to a imidazo[1,2-c]pyrido[2,3-e]pyrimidine, imidazo[1',2':1,6]pyrimido[5,4-c]pyridazine, pyrido[2,3-e][1,2,4]triazolo[4,3-c]pyrimidine or [1,2,4]triazolo[4',3':1,6]pyrimido[5,4-c]pyridazine derivative, and R₅ is a carbonyl group.

Preferred compounds of the invention are those in which group X-Y represents a N-C group, groups R₃ and R₅ are linked to each other to form a five-membered heterocyclic ring containing from 2 to 3 nitrogen heteroatoms atoms, optionally substituted with one or more groups nR₆, as defined above, and R₄ is a carbonyl group.

Preferred compounds of the invention are those in which group X-Y represents a N-C group, and groups R₃ and R₅ are linked to each other to form a five-membered heterocyclic ring containing from 2 to 3 nitrogen heteroatoms atoms (*e.g*. 1,2,4-triazole), selected from but not limited to a pyrido[2,3-e]1,2,4-triazolo[2,1-b]pyrimidine derivative, and R₄ is a carbonyl group.

Preferred compounds of the invention are those in which group X-Y represents a C=N group, groups R₃ and R₄ are linked to each other to form a five- or six-membered heterocyclic ring containing from 2 to 3 nitrogen heteroatoms atoms, optionally substituted with one or more groups nR₆, as defined above, and R₅ is absent.

Preferred compounds of the invention are those in which group X-Y represents a C=N group, and groups R₃ and R₄ are linked to each other to form a five- or six-membered heterocyclic ring containing from 2 to 3 nitrogen heteroatoms atoms (*e.g*. 1,2,4-triazole), selected from but not limited to a pyrido[3,2-d] 1,2,4-triazolo[1,2-d] 1,4,5,6-tetrahydropyridazine derivative, and R₅ is a carbonyl group.

Preferred compounds of the invention are those in which R₁ is selected from the group comprising H, Br, hydroxy, carboxyl, methoxy, methoxyethylamino, 2-hydroxyethylamino, tertiarybutoxycarbonylamino, 2-hydroxyethylaminocarbonyl, an optionally substituted azetidinyl, morpholinyl, oxetanyl, piperazinyl, piperidinyl, pyranyl or pyrrolidinyl moiety or derivative thereof, or an optionally substituted, spiro-fused bi- or tricyclic C₁-C₁₃ heterocyclic group containing from 1 to 5 heteroatoms selected from N, O or S.

Highly preferred compounds of the invention are those in which R₁ is selected from the group comprising 2-oxa-6-azaspiro[3.3]heptan-6-yl, 3-methoxymethylazetidin-1-yl, 3-methoxypyrrolidin-1-yl, 4-acetylpiperazin-1-yl, 4-aminopiperidin-1-yl, 4-hydroxypiperidin-1-yl, 4-hydroxypiperidin-1-yl-carbonyl, 4-methoxypiperidin-1-yl, 4-morpholinyl, dimethylaminopiperidin-1-yl, hydroxymethylpiperidin-1-yl, morpholin-4-ylcarbonyl, tetrahydro-2H-pyran-4-ylamino or tetrahydro-2H-pyran-4-ylaminocarbonyl.

Preferred compounds of the invention are those in which R₂ is a hydrogen atom or an optionally substituted benzyl group or derivative thereof.

Highly preferred compounds of the invention are those in which R₂ is a hydrogen atom or is selected from the group comprising 3,5-dimethoxybenzyl, 4-methoxybenzyl, 4-methylbenzyl, 4-chlorobenzyl or 4-chloro-2,6-difluorobenzyl.

Preferred compounds of the invention are those in which R₆ is selected from the group comprising phenyl, (1-phenyl)ethyl, 1-ethyl-1H-pyrazol-3-yl, 1-ethyl-1H-pyrazol-5-yl, (tetrahydro-2H-pyran-4-yl)methyl, (tetrahydro-2H-pyran-4-yloxy)methyl, (tetrahydro-2H-pyran-4-yl)ethyl, 3,5-dimethyl-1,2oxazol-4-yl, 2-hydroxypyridin-3-yl, 2-methylpyridin-3-yl, morpholin-4-yl-carbonyl, pyridin-3-yl-methyl, oxo, methyl, ethyl, iso-propyl, tertiary-butyl, methylpropyl, 2,2-dimethylpropyl, 3-methylbutyl, 2,2,2-trifluoroethyl, methoxymethyl, methoxyethyl, (propan-2-yloxy)methyl, tertiary-butoxymethyl, prop-1-en-2-yl, propan-2-yl-acetamide, cyclopropyl, cyclobutyl, cyclohexyl, 1-methylcyclopropyl.

Preferred compounds of the invention are those in which -(Zp)- represents a group selected from -O-(CH₂)₂-O-, -O-(CH₂)₃-O-, -O-(CH₂)₂-, -O-(CH₂)₃-, -CH₂-O-CH₂- or -(CH₂)₂-O-(CH₂)₂.

Preferred compounds of the invention are those in which one of the A groups is a nitrogen atom and the three remaining A groups are carbon atoms, the X-Y unit is a N-C group, groups R₃ and R₄ are linked to each other to form a five-membered heterocyclic ring containing 2 nitrogen heteroatoms atoms and R₅ is an oxygen atom double-bonded directly to the X-Y containing ring (carbonyl group), such that the compound so formed is a 2,6-dihydroimidazo[1,2-*c*]pyrido[2,3-*e*]pyrimidin-5(3*H*)-one derivative and has a structure in accordance with formulae **1a** below: 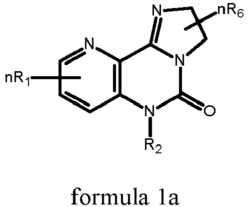 wherein groups R₁, R₂, R₆ and n are as defined for formula I above.

Preferred compounds of the invention are those in which two of the A groups are nitrogen atoms and the two remaining A groups are carbon atoms, the X-Y unit is a N-C group, groups R₃ and R₄ are linked to each other to form a five-membered heterocyclic ring containing 2 nitrogen heteroatoms atoms and R₅ is an oxygen atom double-bonded directly to the X-Y containing ring (carbonyl group), such that the compound so formed is a 8,9-dihydroimidazo[1',2':1,6]pyrimido[5,4-*c*]pyridazin-6(5*H*)-one (derivative and has a structure in accordance with formulae **1b** below: 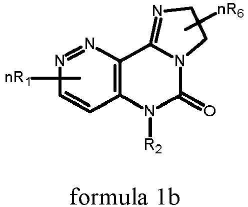 wherein groups R₁, R₂, R₆ and n are as defined for formula I above.

Preferred compounds of the invention are those in which one of the A groups is a nitrogen atom and the three remaining A groups are carbon atoms, the X-Y unit is a N-C group, groups R₃ and R₄ are linked to each other to form a five-membered heterocyclic ring containing a C=N-C=O group, a nitrogen and the remaining carbon atoms and R₅ is an oxygen atom double-bonded directly to the X-Y containing ring (carbonyl group), such that the compound so formed is a imidazo[1,2-*c*]pyrido[2,3-*e*]pyrimidine-2,5(3*H*,6*H*)-dione derivative and has a structure in accordance with formulae **1c** below: 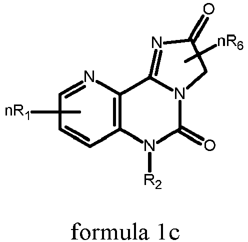 wherein groups R₁, R₂, R₆ and n are as defined for formula I above.

Preferred compounds of the invention are those in which two of the A groups are nitrogen atoms and the two remaining A groups are carbon atoms, the X-Y unit is a N-C group, groups R₃ and R₄ are linked to each other to form a five-membered heterocyclic ring containing a C=N-C=O group, a nitrogen and the remaining is a carbon atom and R₅ is an oxygen atom double-bonded directly to the X-Y containing ring (carbonyl group), such that the compound so formed is a imidazo[1',2':1,6]pyrimido[5,4-*c*]pyridazine-6,9(5*H*,8*H*)-dione derivative and has a structure in accordance with formulae 1d below: 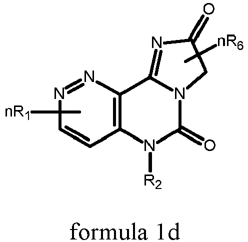 wherein groups R₁, R₂, R₆ and n are as defined for formula I above.

Preferred compounds of the invention are those in which one of the A groups is a nitrogen atom and the three remaining A groups are carbon atoms, the X-Y unit is a N-C group, groups R₃ and R₄ are linked to each other to form a five-membered heterocyclic ring containing 3 nitrogen heteroatoms atoms and R₅ is an oxygen atom double-bonded directly to the X-Y containing ring (carbonyl group), such that the compound so formed is a pyrido[2,3-*e*][1,2,4]triazolo[4,3-*c*]pyrimidin-5(6*H*)-one derivative and has a structure in accordance with formulae 1e below: 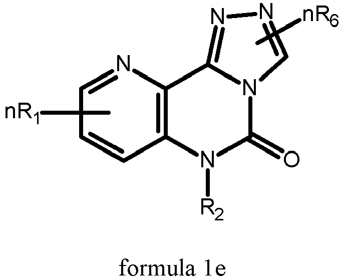 wherein groups R₁, R₂, R₆ and n are as defined for formula I above.

Preferred compounds of the invention are those in which two of the groups are two nitrogen atoms and the two remaining A groups are carbon atoms, the X-Y unit is a N-C group, groups R₃ and R₄ are linked to each other to form a five-membered heterocyclic ring containing 3 nitrogen heteroatoms atoms and R₅ is an oxygen atom double-bonded directly to the X-Y containing ring (carbonyl group), such that the compound so formed is a [1,2,4]triazolo[4',3':1,6]pyrimido[5,4-c]pyridazin-5(6*H*)-one derivative and has a structure in accordance with formulae **1f** below: 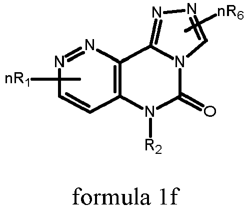 wherein groups R₁, R₂, R₆ and n are as defined for formula I above.

More preferred compounds of the invention are those represented by the general structure:

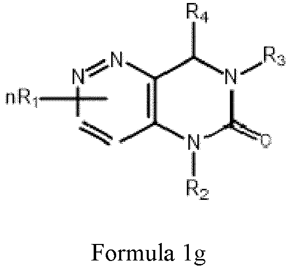

And still more preferred compounds of the invention are those represented by one or more of the general structures: 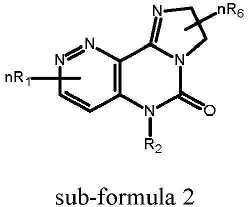 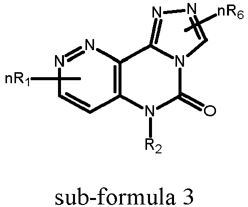 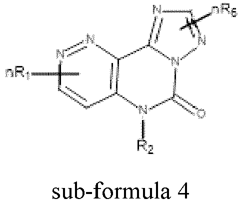 wherein groups R₁, R₂, R₃, R₄, R₅, R₆ and n are as defined for formula I above.
Still more preferred may be compounds of sub-formula 2.
Still more preferred may be compounds of sub-formula 3.
Still more preferred may be compounds of sub-formula 4.

Preferred compounds according to the invention are compounds or an enantiomer, diastereomer, N-oxide, or a pharmaceutically acceptable salt or combinations thereof, is provided according to general formula 1a selected from the compounds in **Table 1** below:

**Table 1: Selected compounds of the invention according to formula 1a**

| **Example** | **Structure** | **Name** |
|---|---|---|
| **1** | 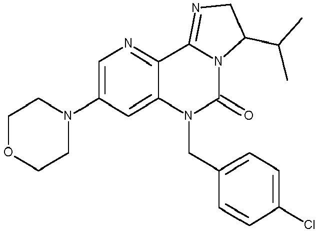 | 6-(4-Chlorobenzyl)-8-(morpholin-4-yl)-3-(propan-2-yl)-2,6-dihydroimidazo[1,2-c]pyrido[2,3-e]pyrimidin-5(3H)-one |
| **2** | 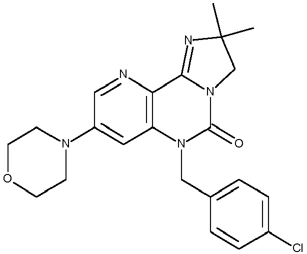 | 6-(4-Chlorobenzyl)-2,2-dimethyl-8-(morpholin-4-yl)-2,6-dihydroimidazo[1,2-c]pyrido[2,3-e]pyrimidin-5(3H)-one |
| **3** | 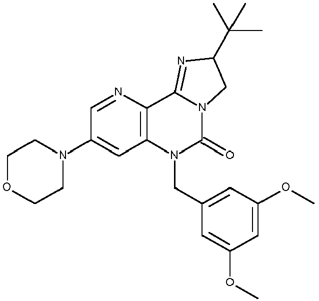 | 2-tert-Butyl-6-(3,5-dimethoxybenzyl)-8-(morpholin-4-yl)-2,6-dihydroimidazo[1,2-c]pyrido[2,3-e]pyrimidin-5(3H)-one |
| **4** | 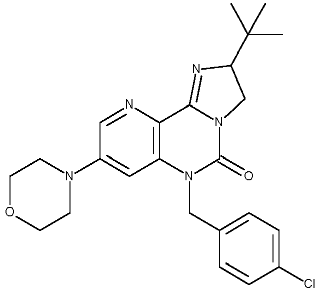 | 2-tert-Butyl-6-(4-chlorobenzyl)-8-(morpholin-4-yl)-2,6-dihydroimidazo[1,2-c]pyrido[2,3-e]pyrimidin-5(3H)-one |
| **5** | 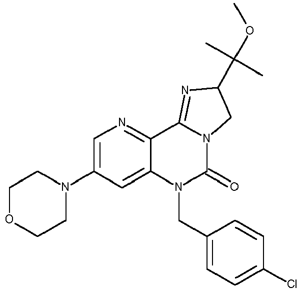 | 6-(4-Chlorobenzyl)-2-(2-methoxypropan-2-yl)-8-(morpholin-4-yl)-2,6-dihydroimidazo[1,2-c]pyrido[2,3-e]pyrimidin-5(3H)-one |
| **6** | 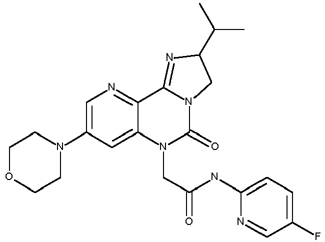 | N-(5-Fluoropyridin-2-yl)-2-[8-(morpholin-4-yl)-5-oxo-2-(propan-2-yl)-2,3-dihydroimidazo[1,2-c]pyrido[2,3-e]pyrimidin-6(5H)-yl]acetamide |
| **7** | 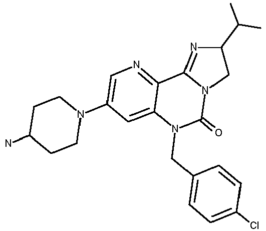 | 8-(4-Aminopiperidin-1-yl)-6-[(4-chlorophenyl)methyl]-2-(propan-2-yl)-2,6-dihydroimidazo[1,2-c]pyrido[2,3-e]pyrimidin-5(3H)-one |
| **8** | 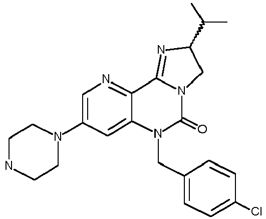 | 6-[(4-Chlorophenyl)methyl]-8-(piperazin-1-yl)-2-(propan-2-yl)-2,6-dihydroimidazo[1,2-c]pyrido[2,3-e]pyrimidin-5(3H)-one |
| **9** | 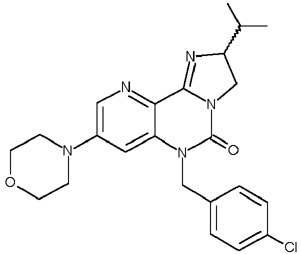 | 6-[(4-Chlorophenyl)methyl]-8-(morpholin-4-yl)-2-(propan-2-yl)-2,6-dihydroimidazo[1,2-c]pyrido[2,3-e]pyrimidin-5(3H)-one |
| **10** | 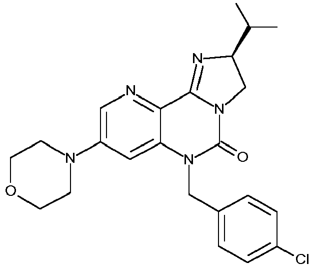 | (2S)-6-(4-Chlorobenzyl)-8-(morpholin-4-yl)-2-(propan-2-yl)-2,6-dihydroimidazo[1,2-c]pyrido[2,3-e]pyrimidin-5(3H)-one |
| **11** | 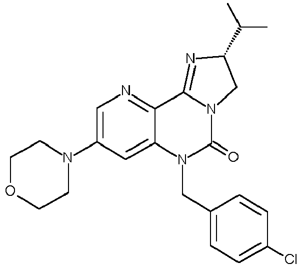 | (2R)-6-(4-Chlorobenzyl)-8-(morpholin-4-yl)-2-(propan-2-yl)-2,6-dihydroimidazo[1,2-c]pyrido[2,3-e]pyrimidin-5(3H)-one |
| **12** | 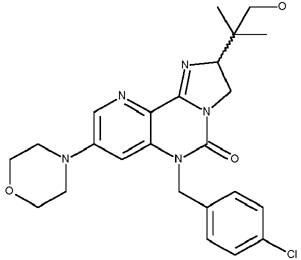 | 6-(4-Chlorobenzyl)-2-(1-hydroxy-2-methylpropan-2-yl)-8-(morpholin-4-yl)-2,6-dihydroimidazo[1,2-c]pyrido[2,3-e]pyrimidin-5(3H)-one |
| **13** | 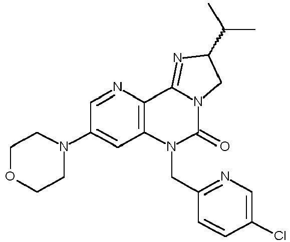 | 6-[(5-Chloropyridin-2-yl)methyl]-8-(morpholin-4-yl)-2-(propan-2-yl)-2,6-dihydroimidazo[1,2-c]pyrido[2,3-e]pyrimidin-5(3H)-one |
| **14** | 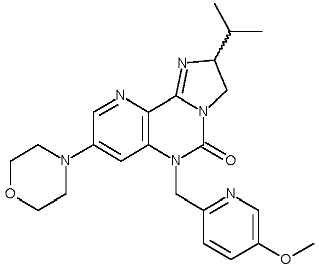 | 6-[(5-Methoxypyridin-2-yl)methyl]-8-(morpholin-4-yl)-2-(propan-2-yl)-2,6-dihydroimidazo[1,2-c]pyrido[2,3-e]pyrimidin-5(3H)-one |
| **15** | 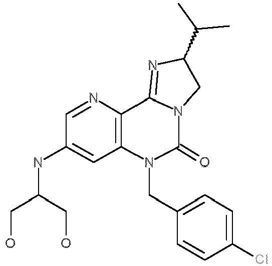 | 6-[(4-Chlorophenyl)methyl]-8-[(1,3-dihydroxypropan-2-yl)amino]-2-(propan-2-yl)-2,6-dihydroimidazo[1,2-c]pyrido[2,3-e]pyrimidin-5(3H)-one |
| **16** | 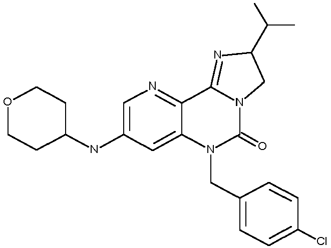 | 6-[(4-Chlorophenyl)methyl]-8-[(oxan-4-yl)amino]-2-(propan-2-yl)-2,6-dihydroimidazo[1,2-c]pyrido[2,3-e]pyrimidin-5(3H)-one |

Preferred compounds according to the invention are compounds or an enantiomer, diastereomer, N-oxide, or a pharmaceutically acceptable salt or combinations thereof, is provided according to general formula **1b** selected from the compounds in **Table 2** below:

**Table 2: Selected compounds of the invention according to formula 1b and for compounds 17 to 19 and 27 and 28 more preferred sub-formula 2**

| | | |
|---|---|---|
| 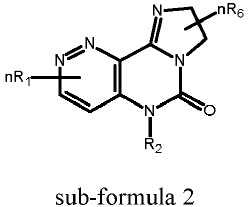 | | |
| wherein the substituents and their variants are as described herein. | | |

| **Example** | **Structure** | **Name** |
|---|---|---|
| **17** | 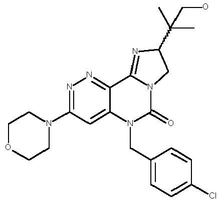 | 5-[(4-Chlorophenyl)methyl]-9-(1-hydroxy-2-methylpropan-2-yl)-3-(morpholin-4-yl)-8,9-dihydroimidazo[1',2':1,6]pyrimido[5,4-c]pyridazin-6(5H)-one |
| **18** | 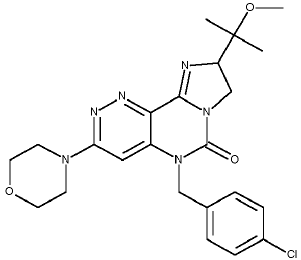 | 5-[(4-Chlorophenyl)methyl]-9-(2-methoxypropan-2-yl)-3-(morpholin-4-yl)-8,9-dihydroimidazo[1',2':1,6]pyrimido[5,4-c]pyridazin-6(5H)-one |
| **19** | 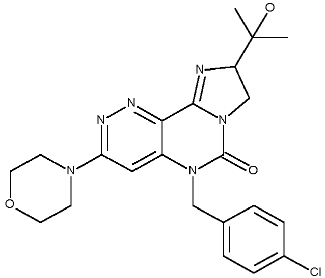 | 5-[(4-Chlorophenyl)methyl]-9-(2-hydroxypropan-2-yl)-3-(morpholin-4-yl)-8,9-dihydroimidazo[1',2':1,6]pyrimido[5,4-c]pyridazin-6(5H)-one |

Preferred compounds according to the invention are compounds or an enantiomer, diastereomer, N-oxide, or a pharmaceutically acceptable salt or combinations thereof, is provided according to general formula **1c** selected from the compounds in **Table 3** below:

**Table 3: Selected compounds of the invention according to formula 1c.**

| **Example** | **Structure** | **Name** |
|---|---|---|
| **20** | 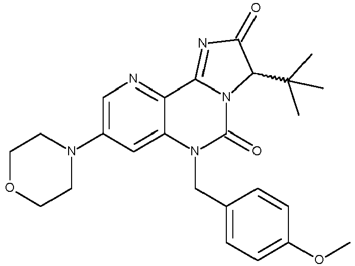 | 3-tert-Butyl-6-[(4-methoxyphenyl)methyl] -8-(morpholin-4-yl)imidazo[1,2-c]pyrido[2,3-e]pyrimidine-2,5(3H,6H)-dione |
| **21** | 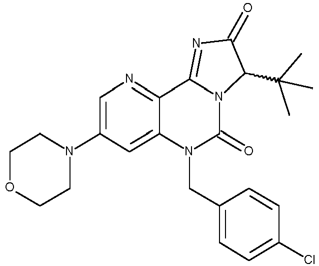 | 3-tert-Butyl-6-[(4-chlorophenylmethyl]-8-(morpholin-4-yl)imidazo[1,2-c]pyrido[2,3-e]pyrimidine-2,5(3H,6H)-dione |
| **22** | 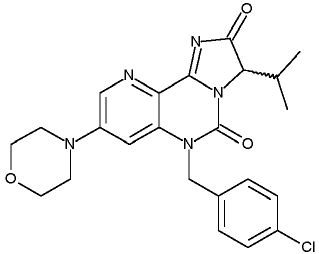 | 6-[(4-Chlorophenyl)methyl]-8-(morpholin-4-yl)-3-(propan-2-yl)imidazo[1,2-c]pyrido[2,3-e]pyrimidine-2,5(3H,6H)-dione |
| **23** | 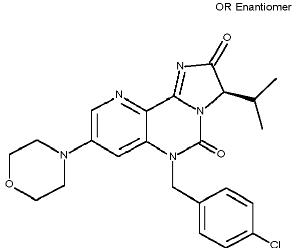 | (R or S)-6-[(4-Chlorophenyl)methyl]-8-(morpholin-4-yl)-3-(propan-2-yl)imidazo[1,2-c]pyrido[2,3-e]pyrimidine-2,5(3H,6H)-dione |
| **24** | 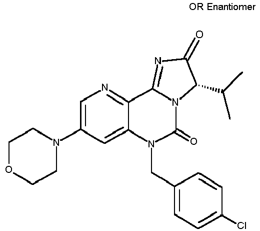 | (S or R)-6-[(4-Chlorophenyl)methyl]-8-(morpholin-4-yl)-3-(propan-2-yl)imidazo[1,2-c]pyrido[2,3-e]pyrimidine-2,5(3H,6H)-dione |
| **25** | 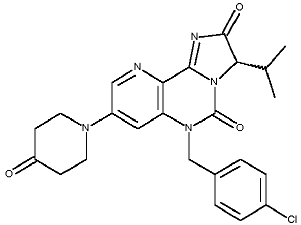 | 6-[(4-Chlorophenyl)methyl]-8-(4-oxopiperidin-1-yl)-3-(propan-2-yl)imidazo[1,2-c]pyrido[2,3-e]pyrimidine-2,5(3H,6H)-dione |
| **26** | 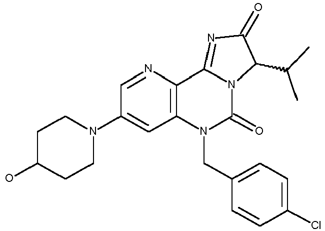 | 6-[(4-Chlorophenyl)methyl]-8-(4-hydroxypiperidin-1-yl)-3-(propan-2-yl)imidazo[1,2-c]pyrido[2,3-e]pyrimidine-2,5(3H,6H)-dione |

Preferred compounds according to the invention are compounds or an enantiomer, diastereomer, N-oxide, or a pharmaceutically acceptable salt or combinations thereof, is provided according to general formula **1d** selected from the compounds in **Table 4** below:

**Table 4: Selected compounds of the invention according to formula 1d.**

| **Example** | **Structure** | **Name** |
|---|---|---|
| 27 | 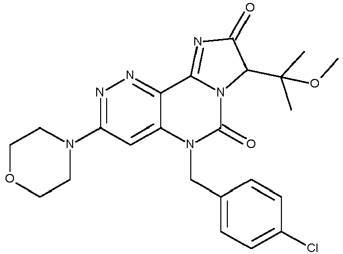 | 5-[(4-Chlorophenyl)methyl]-8-(2-methoxypropan-2-yl)-3- (morpholin-4-yl)imidazo[1',2':1,6]pyrimido[5,4-c]pyridazine-6,9(5H,8H)-dione |
| **28** | 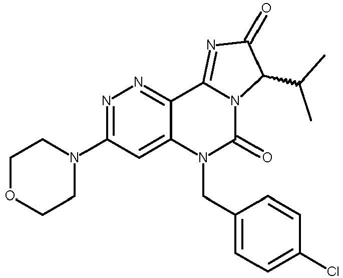 | 5-[(4-Chlorophenyl)methyl]-3-(morpholin-4-yl)-8-(propan-2-yl)imidazo[1',2':1,6]pyrimido[5,4-c]pyridazine-6,9(5H,8H)-dione |

Preferred compounds according to the invention are compounds or an enantiomer, diastereomer, N-oxide, or a pharmaceutically acceptable salt or combinations thereof, is provided according to general formula **1e** selected from the compounds in **Table 5** below:

**Table 5: Selected compounds of the invention according to formula 1d.**

| **Example** | **Structure** | **Name** |
|---|---|---|
| **29** | 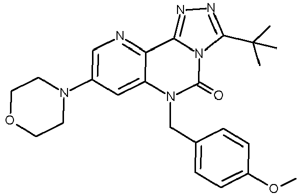 | 3-tert-Butyl-6-(4-methoxybenzyl)-8-(morpholin-4-yl)pyrido[2,3-e][1,2,4]triazolo[4,3-c]pyrimidin-5(6H)-one |
| **30** | 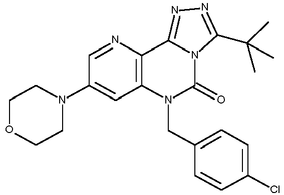 | 3-tert-Butyl-6-[(4-chlorophenyl)methyl]-8-(morpholin-4-yl)pyrido[2,3-e][1,2,4]triazolo[4,3-c]pyrimidin-5(6H)-one |
| **31** | 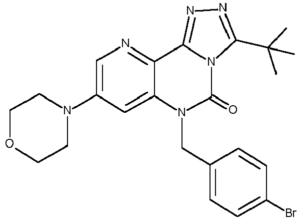 | 6-[(4-Bromophenyl)methyl] -3 -tert-butyl-8-(morpholin-4-yl)pyrido[2,3-e][1,2,4]triazolo[4,3-c]pyrimidin-5(6H)-one |
| **32** | 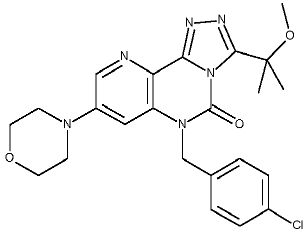 | 6-(4-Chlorobenzyl)-3 -(2-methoxypropan-2-yl)-8-(morpholin-4-yl)pyrido[2,3-e][1,2,4]triazolo[4,3-c]pyrimidin-5(6H)-one |
| **33** | 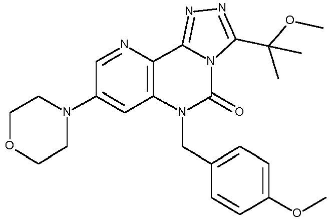 | 6-(4-Methoxybenzyl)-3 -(2-methoxypropan-2-yl)-8-(morpholin-4-yl)pyrido[2,3-e][1,2,4]triazolo[4,3-c]pyrimidin-5(6H)-one |
| **34** | 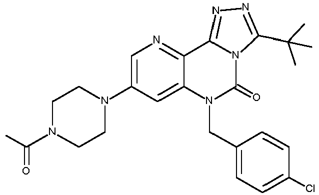 | 8-(4-Acetylpiperazin-1-yl)-3-*tert-*butyl-6-[(4-chlorophenyl)methyl]pyrido[2,3-*e*][1,2,4]triazolo[4,3-*c*]pyrimidin-5(6*H*)-one |
| **35** | 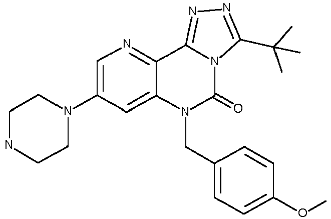 | 3-tert-Butyl-6-[(4-methoxyphenyl)methyl] -8-(piperazin-1-yl)pyrido[2,3-e][1,2,4]triazolo[4,3-c]pyrimidin-5(6H)-one |
| **36** | 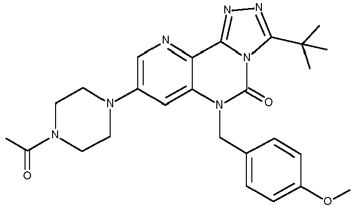 | 8-(4-Acetylpiperazin-1-yl)-3-tert-butyl-6-[(4-methoxyphenyl)methyl]pyrido[2,3-e][1,2,4]triazolo[4,3-c]pyrimidin-5(6H)-one |
| **37** | 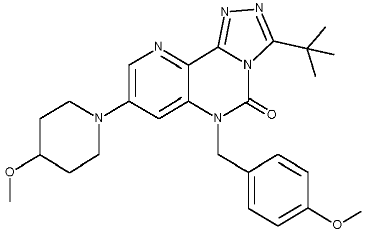 | 3-*tert*-Butyl-6-[(4-methoxyphenyl)methyl] -8-(4-methoxypiperidin-1-yl)pyrido[2,3-*e*][1,2,4]triazolo[4,3-c]pyrimidin-5(6*H*)-one |
| **38** | 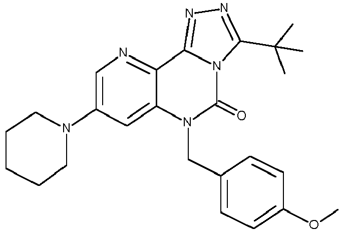 | 3-*tert*-Butyl-6-[(4-methoxyphenyl)methyl]-8-(piperidin-1-yl)pyrido[2,3-*e*][1,2,4]triazolo[4,3-*c*]pyrimidin-5(6*H*)-one |
| **39** | 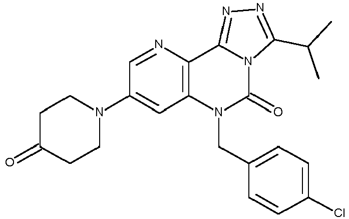 | 6-[(4-Chlorophenyl)methyl]-8-(4-oxopiperidin-1-yl)-3-(propan-2-yl)pyrido[2,3-*e*][1,2,4]triazolo[4,3-*c*]pyrimidin-5(6*H*)-one |
| **40** | 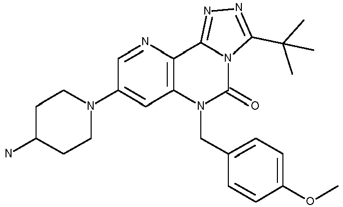 | 8-(4-Aminopiperidin-1-yl)-3-*tert-*butyl-6-[(4-methoxyphenyl)methyl]pyrido[2,3-*e*][1,2,4]triazolo[4,3-*c*]pyrimidin-5(6*H*)-one |
| **41** | 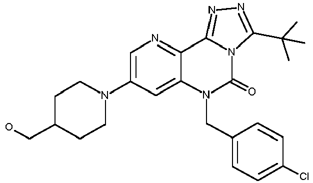 | 3-tert-Butyl-6-[(4-chlorophenyl)methyl] -8-[4-(hydroxymethyl)piperidin-1-yl]pyrido[2,3-e][1,2,4]triazolo[4,3-c]pyrimidin-5(6H)-one |
| **42** | 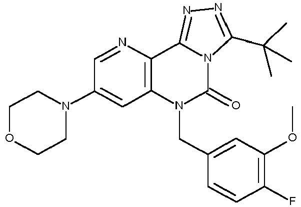 | 3-*tert*-Butyl-6-[(4-fluoro-3- methoxyphenyl)methyl] -8-(morpholin-4-yl)pyrido[2,3-*e*][1,2,4]triazolo[4,3-*c*]pyrimidin-5(6*H*)-one |
| 43 | 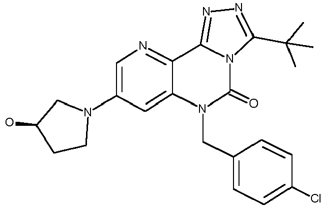 | 3-tert-Butyl-6-[(4-chlorophenyl)methyl]-8-[(3R)-3-hydroxypyrrolidin-1-yl]pyrido[2,3-e][1,2,4]triazolo[4,3-c]pyrimidin-5(6H)-one |

Preferred compounds according to the invention are compounds or an enantiomer, diastereomer, N-oxide, or a pharmaceutically acceptable salt or combinations thereof, is provided according to general formula **1f** selected from the compounds in **Table 6** below:

**Table 6: Selected compounds of the invention according to formula 1f and for compounds 45-50 and 55 more preferred sub-formula 3**

| | | |
|---|---|---|
| 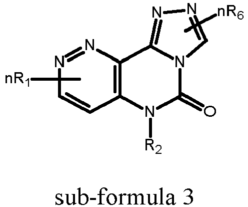 | | |
| wherein the substituents and their variants are as described herein. | | |

| **Exampl e** | **Structure** | **Name** |
|---|---|---|
| **44** | 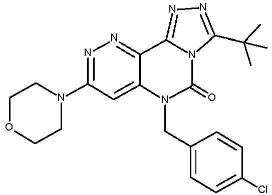 | 3-tert-Butyl-6-[(4-chlorophenyl)methyl]-8-(morpholin-4-yl)[1,2,4]triazolo[4',3': 1,6]pyrimido[5,4-c]pyridazin-5(6H)-one |
| **45** | 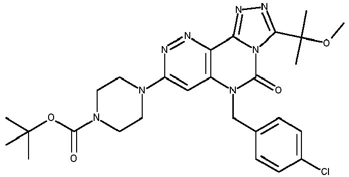 | tert-Butyl 4-{6-[(4-chlorophenyl)methyl]-3-(2-methoxypropan-2-yl)-5-oxo-5,6-dihydro[1,2,4]triazolo[4',3':1,6]pyrimido[5 ,4-c]pyridazin-8-yl}piperazine-1-carboxylate |
| **46** | 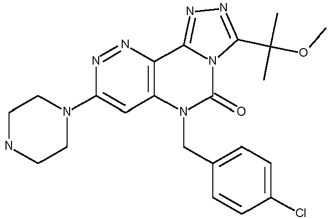 | 6-[(4-Chlorophenyl)methyl]-3-(2-methoxypropan-2-yl)-8-(piperazin-1-yl)[1,2,4]triazolo[4',3': 1,6]pyrimido[5,4-c]pyridazin-5(6H)-one |
| **47** | 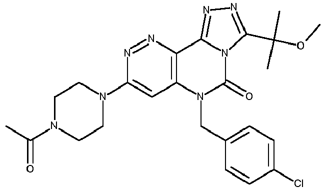 | 8-(4-Acetylpiperazin-1-yl) -6- [(4-chlorophenyl)methyl]-3-(2-methoxypropan-2-yl)[1,2,4]triazolo[4',3': 1,6]pyrimido[5,4-c]pyridazin-5(6H)-one |
| **48** | 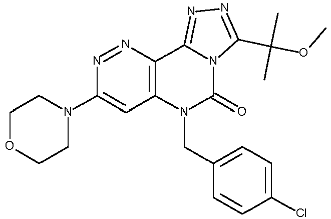 | 6-[(4-Chlorophenyl)methyl]-3-(2-methoxypropan-2-yl)-8-(morpholin-4-yl)[1,2,4]triazolo[4',3': 1,6]pyrimido[5,4-c]pyridazin-5(6H)-one |
| **49** | 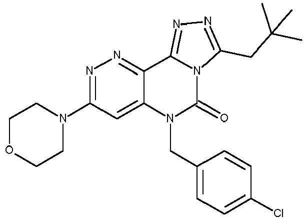 | 6-[(4-Chlorophenyl)methyl]-3-(2,2-dimethylpropyl)-8-(morpholin-4-yl)[1,2,4]triazolo[4',3':1,6]pyrimido[5,4-*c*]pyridazin-5(6*H*)-one |
| **50** | 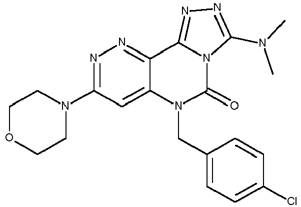 | 6-[(4-Chlorophenyl)methyl] -3 - (dimethylamino)-8-(morpholin-4-yl)[1,2,4]triazolo[4',3': 1,6]pyrimido[5,4-*c*]pyridazin-5(6*H*)-one |
| **55** | 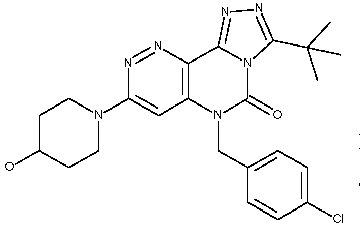 | 3-*tert*-Butyl-6-[(4-chlorophenyl)methyl]-8-(4-hydroxypiperidin-1-yl)[1,2,4]triazolo[4',3': 1,6]pyrimido[5,4-*c*]pyridazin-5(6*H*)-one |

The following invention are compounds or an enantiomer, diastereomer, N-oxide, or a pharmaceutically acceptable salt or combinations thereof, that do not respond to the general formula 1(a-f) but belongs to the general formula I, and are listed into the Table 7 below:

**Table 7: Compounds outside the specific examples 1(a-f) or the general formula I and for compounds 53 and 54 more preferred sub-formula 4**

| | | |
|---|---|---|
| 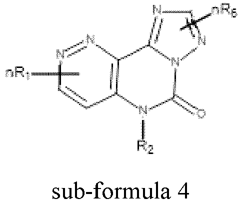 | | |
| wherein the substituents and their variants are as described herein. | | |

| **Example** | **Structure** | **Name** |
|---|---|---|
| **51** | 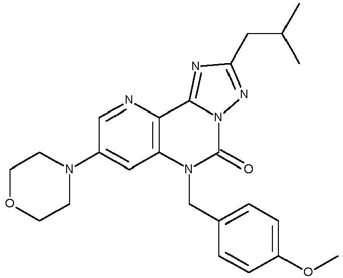 | 6-[(4-Methoxyphenyl)methyl] -2-(2-methylpropyl)-8-(morpholin-4-yl)pyrido[2,3-e][1,2,4]triazolo[1,5-c]pyrimidin-5(6H)-one |
| **52** | 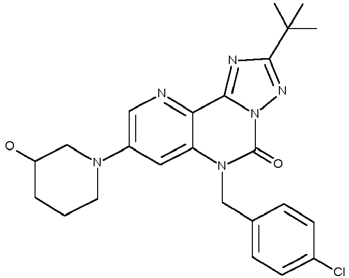 | 2-tert-Butyl-6-[(4-chlorophenyl)methyl]-8-(3-hydroxypiperidin-1-yl)pyrido[2,3-e][1,2,4]triazolo[1,5-c]pyrimidin-5(6H)-one |
| **53** | 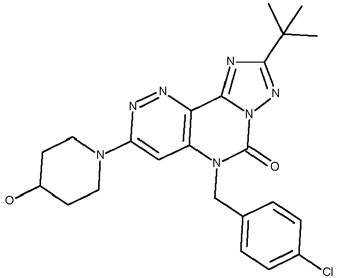 | 9-*tert*-Butyl-5-[(4-chlorophenyl)methyl]-3-(4-hydroxypiperidin-1-yl)[1,2,4]triazolo[1',5':1,6]pyrimido[5,4 -*c*]pyridazin-6(5*H*)-one |
| **54** | 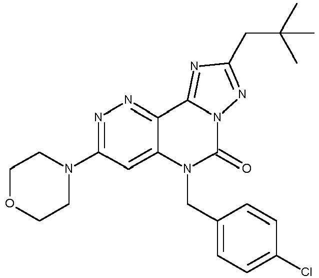 | 5-[(4-Chlorophenyl)methyl]-9-(2,2-dimethylpropyl)-3-(morpholin-4-yl)[1,2,4]triazolo[1',5':1,6]pyrimido[5,4 -*c*]pyridazin-6(5*H*)-one |

The invention also provides for a pharmaceutical composition comprising a compound of formula I: 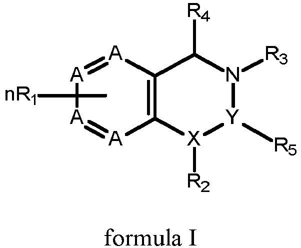 or an enantiomer, diastereomer, N-oxide, or a pharmaceutically acceptable salt or combinations thereof, and a pharmaceutically acceptable carrier, wherein A, X, Y, R₁ through R₆ and n have the meanings ascribed to them above, for use in the treatment and/or prevention of pain, chronic pain and tolerance to analgesic, respiratory disorders and dysfunctions, overactive bladder, bladder pain syndrome, dysuria and in general in genitourinary diseases, cardiovascular disorders and more in general for the potential treatment of visceral organ diseases and disorders characterized by the involvement of P₂X₃ and P₂X₂/₃. Compounds of the invention are suitable and can be used for the treatment and/or prevention of the itch and pruritus derived by different causes as for example but not exhaustive cases: atopic dermatitis, psoriasis or lupus erythematosus, or pruritus derived by viral infection or cancer (leukaemia or lymphomas).
The invention also provides for a pharmaceutical composition comprising a compound of any of formulae 1a to 1f or reported in Table 7: 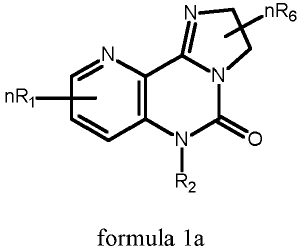 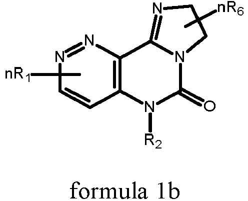 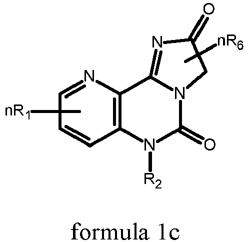 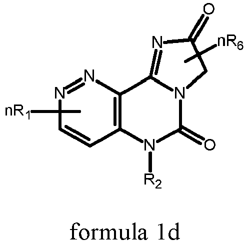 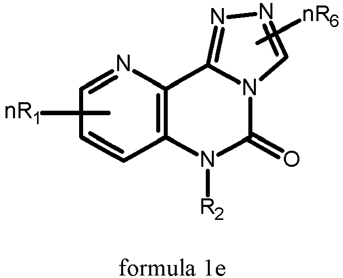 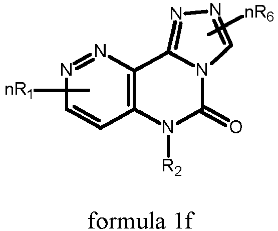 or an enantiomer, diastereomer, N-oxide, or a pharmaceutically acceptable salt or combinations thereof, and a pharmaceutically acceptable carrier, wherein A, X, Y, R₁ through R₆ and n have the meanings ascribed to them above, for use in the treatment and/or prevention of pain, chronic pain and tolerance to analgesic, respiratory disorders and dysfunctions, genitourinary diseases and cardiovascular disorders, in general, for the potential treatment of visceral organ diseases and disorders characterized by the involvement of P₂X₃ and P₂X₂/₃. Compounds of the invention are suitable and can be used for the treatment and/or prevention of the itch and pruritus derived by different causes as for example but not exhaustive cases: atopic dermatitis, psoriasis or lupus erythematosus, or pruritus derived by viral infection or cancer (leukaemia or lymphomas).

The invention also provides for compounds according to any of formula I or formulae 1a to 1f shown above, which is used in the treatment and/or prevention of, dysfunction including without any limitation involving ATP release, and in general, for the potential treatment of sensory and visceral organ diseases and disorders characterized by the involvement of P2X₃ and P2X_{2/3} receptors; for the treatment and/or prevention of pain, chronic pain and cancer pain, addiction and tolerance to analgesic; for the treatment of asthma, cough, COPD and refractory chronic cough and in general of respiratory disorders and dysfunctions; for the treatment of overactive bladder, urinary incontinence, bladder pain syndrome, dysuria and endometriosis and in general in genitourinary diseases; for treatment of cardiovascular disorders, irritable bowel syndrome (IBS), oesophageal hypersensitivity, Burning Mouth Syndrome (BMS) of migraine and itch.

### Terms and Definitions Used

Except where stated otherwise, the following definitions apply throughout the present specification and claims. These definitions apply regardless of whether a term is used by itself or in combination with other terms. For example, the definition of "alkyl" applies not only to alkyl groups *per se,* but also to the alkyl portions of alkoxy, alkylamino, alkylthio or alkylcarbonyl groups *etc.* Furthermore, all ranges described for a chemical group, for example "from 1 to 13 carbon atoms" or "C₁-C₆ alkyl" include all combinations and sub-combinations of ranges and specific numbers of carbon atoms therein.

The skilled person will be aware that groups A, X, Y, R₁ to R₆ and n all have the meanings given to them as described herein. For example, "groups X and Y are selected from C and N atoms, wherein the unit X-Y represents either a N-C group, or a C=N group respectively.

"Alkyl" means a straight chain or branched chain aliphatic hydrocarbon group having from 1 to 20 carbon atoms in the chain. Preferred alkyl groups have from 1 to 12 carbon atoms in the chain. More preferred alkyl groups have from 1 to 6 carbon atoms in the chain. "Lower alkyl" means an alkyl group having about 1 to about 6 carbon atoms in the chain which may be straight or branched. Examples of suitable alkyl groups include methyl, ethyl, n-propyl, isopropyl, sec-butyl, n-butyl, and t-butyl.

"Alkenyl" means a straight chain or branched chain aliphatic hydrocarbon group having at least one carbon-carbon double bond and having from 2 to 15 carbon atoms in the chain. Preferred alkenyl groups have from 2 to 12 carbon atoms in the chain. More preferred alkenyl groups have from 2 to 6 carbon atoms in the chain. "Lower alkenyl" means an alkenyl group having 2 to about 6 carbon atoms in the chain, which may be straight or branched. Examples of suitable alkenyl groups include ethenyl, propenyl, isopropenyl, n-butenyl, 1-hexenyl and 3-methylbut-2-enyl.

"Alkynyl" means a straight chain or branched chain aliphatic hydrocarbon group having at least one carbon-carbon triple bond and having from 2 to 15 carbon atoms in the chain. Preferred alkynyl groups have from 2 to 12 carbon atoms in the chain. More preferred alkynyl groups have from 2 to 6 carbon atoms in the chain. "Lower alkynyl" means an alkynyl group having 2 to about 6 carbon atoms in the chain, which may be straight or branched. Examples of suitable alkynyl groups include ethynyl, propynyl and 2-butynyl.

"Mono-, bi-, or tricyclic heterocyclic" means an aromatic or non-aromatic saturated mono- bi- or tricyclic ring system having from 2 to 14 ring carbon atoms and containing from 1 to 5 ring atoms selected from N, O and S, alone or in combination. Bi- and tricyclic heterocyclic groups are fused at 2 or 4 points or joined at one point *via* a bond or a heteroatom linker (O, S, NH, or N(C₁-C₆ alkyl). The "mono- bi- or tricyclic heterocyclic" can be optionally substituted on the ring by replacing an available hydrogen on the ring by one or more substituents which may be the same or different. The nitrogen or sulphur atom of the heterocyclic can be optionally oxidized to the corresponding N-oxide, S-oxide or S-dioxide. Examples of suitable heterocyclics include furanyl, imidazolyl, isoxazolyl, oxadiazolyl, oxazolyl, pyrrolyl, pyridyl, pyrimidyl, pyridazinyl, thiazolyl, triazolyl, tetrazolyl, thienyl, carbazolyl, benzimidazolyl, benzothienyl, benzofuranyl, indolyl, quinolinyl, benzotriazolyl, benzothiazolyl, benzooxazolyl, benzimidazolyl, isoquinolinyl, isoindolyl, acridinyl and benzoisoxazolyl, aziridinyl, piperidinyl, pyrrolidinyl, piperazinyl, tetrahydropyranyl, tetrahydrofuranyl, tetrahydrothiophenyl, morpholinyl and thiomorpholinyl.

Heterocyclics with aromatic characteristics may be referred to as heteroaryls or heteroaromatics. Examples of suitable heteroaromatics include furanyl, imidazolyl, isoxazolyl, oxadiazolyl, oxazolyl, pyrrolyl, pyridyl, pyrimidyl, pyridazinyl, thiazolyl, triazolyl, tetrazolyl, thienyl, carbazolyl, benzimidazolyl, benzothienyl, benzofuranyl, indolyl, quinolinyl, benzotriazolyl, benzothiazolyl, benzooxazolyl, benzimidazolyl, isoquinolinyl, isoindolyl, acridinyl, benzoisoxazolyl, tetrahydroquinolinyl, tetrahydroisoquinolinyl, 3-phenylpyridine, 3-cyclohexylpyridine, 3-(pyridin-3-yl) morpholine, 3-phenylisoxazole and 2-(piperidin-1-yl)pyrimidine.

"Mono-, bi- or tricyclic aryl" means an aromatic monocyclic, bicyclic or tricyclic ring system comprising 6 to 14 carbon atoms. Bi- and tricyclic aryl groups are fused at 2 or 4 points or joined at one point *via* a bond or a heteroatom linker (O, S, NH, or N(C₁-C₆ alkyl) (e.g., biphenyl, 1-phenylnapthyl). The aryl group can be optionally substituted on the ring with one or more substituents, preferably 1 to 6 substituents, which may be the same or different. Examples of suitable aryl groups include phenyl and naphthyl.

"Cycloalkyl" means a monocyclic or bicyclic carbon ring system having from 3 to 14 carbon atoms, preferably from 3 to 6 carbon atoms. The cycloalkyl can be optionally substituted on the ring by replacing an available hydrogen on the ring by one or more substituents which may be the same or different. Examples of suitable monocyclic cycloalkyls include cyclopropyl, cyclopentyl, cyclohexyl and cycloheptyl. Examples of suitable multicyclic cycloalkyls include 1-decalinyl, norbornyl and adamantyl.

"Cycloalkenyl" has a meaning corresponding to that of cycloalkyl, but with one or two double bonds within the ring (*e.g*., cyclohexenyl, cyclohexadiene).

"Amines" are derivatives of ammonia, wherein one or more hydrogen atoms have been replaced by a substituent such as an alkyl or aryl group. These may respectively be called alkylamines and arylamines; amines in which both types of substituent are attached to one nitrogen atom may be called alkylarylamines.

Amines can be further organized into four sub-categories. Primary amines arise when one of the three hydrogen atoms in ammonia is replaced by an alkyl or aromatic group (an N-alkylamino or N-arylamino respectively). Examples of suitable primary alkyl amines include methylamine or ethanolamine, or aniline (phenylamine) as an example of an aromatic amine. Secondary amines have two organic substituents (independently alkyl or aryl groups) bound to the nitrogen atom together with one hydrogen (or no hydrogen if one of the substituent bonds is double). Examples of suitable secondary amines include dimethylamine and methylethanolamine, while an example of an aromatic amine would be diphenylamine. Such compounds may also be referred to as "N,N-dialkylamino", "N,N-diarylamino" or "N,N-alkylarylamino" groups depending on the nature of the substituents. A secondary amine substituted by an alkoxy group, as defined herein, would be termed an "N-alkyl-N-alkoxyamino" compound for example. In tertiary amines, all three hydrogen atoms are replaced by organic substituents, such as trimethylamine. The final sub-category is cyclic amines which are either secondary or tertiary amines. Examples of suitable cyclic amines include the 3-member ring aziridine and the six-membered ring piperidine. N-methylpiperidine and N-phenylpiperidine are suitable examples of cyclic tertiary amines.

"Amides" are compounds with a nitrogen atom attached to a carbonyl group, thus having the structure R-CO-NR'R", with groups R' and R" being independently selected from alkyl or aromatic groups as defined herein. For example, when R' is hydrogen and R" is a 3-pyridyl group, the resulting amide has a 3-pyridylamino substituent. Alternatively, when R' is hydrogen and R" is a cyclopentyl group, the resulting amide has a cyclopentylamino substituent.

"Halogen", "halide" or "halo" means fluorine, chlorine, bromine or iodine. Preferred halogens are fluorine, chlorine or bromine, and most preferred are fluorine and chlorine.

The term "acyl", whether used alone, or within a term such as "acylamino", denotes a radical provided by the residue after removal of hydroxyl from an organic acid. The term "acylamino" refers to an amino radical substituted with an acyl group. An example of an "acylamino" radical is CH₃C(=O)-NH- where the amine may be further substituted with alkyl, aryl or aralkyl groups.

The term "condensed ring" refers to a polycyclic ring system in a molecule in which two rings share two or more common atoms. Two rings that have only two atoms and one bond in common are said to be ortho-fused, *e.g*. naphthalene. In a polycyclic compound, a ring ortho-fused to different sides of two other rings that are themselves ortho-fused together (*i.e.* there are three common atoms between the first ring and the other two) is said to be ortho- and peri-fused to the other two rings. Phenalene is considered as being composed of three benzene rings, each of which is ortho- and peri-fused to the other two. Fusion nomenclature is concerned with a two-dimensional representation of a polycyclic ring system with the maximum number of non-cumulative double bonds. In addition, this system may be bridged, or involved in assemblies or spiro- systems (see below). For ring systems any ring fused to other rings on all sides must be itself named (i.e. it is not treated as a hole). For nomenclature purposes two rings which have two atoms and one bond in common may be regarded as being derived from the two rings as separate entities. The process of joining rings in this way is termed fusion. Any fusion compound illustrated or described herein, is named in accordance and with reference to *"*Nomenclature of fused and bridged fused ring systems" (IUPAC Recommendations 1998)", IUPAC, Pure Appl. Chem., (1999), Vol.70, pp.143-216.

A spiro compound has two (or three) rings which have only one atom in common and the two (or three) rings are not linked by a bridge. The rings may form part of other ring systems (fused ring, bridged fused ring, system named by von Baeyer nomenclature, *etc.).* The common atom is known as a spiro atom, and spiro-fusion has also been termed spiro union. Monospiro hydrocarbons with two monocyclic rings are named by the prefix spiro before a von Baeyer descriptor (indicating the numbers of carbon atoms linked to the spiro atom in each ring in ascending order and separated by a full stop) placed in square brackets and then the name of the parent hydrocarbon indicating the total number of skeletal atoms, *e.g*. spiro[4.4]nonane. Monospiro hydrocarbons with two monocyclic rings are numbered consecutively starting in the smaller ring at an atom next to the spiro atom, proceeding around the smaller ring back to the spiro atom and then round the second ring. Heteroatoms are indicated by replacement prefixes and unsaturation is indicated in the usual way by the endings ene, diene, etc. Low locants are allocated for radical positions, or, if the ring system is a substituent, its point of attachment. If there is a choice of numbers the name that gives the lower locants for spiro atoms is selected. Any spiro compound illustrated or described herein, is named in accordance and with reference to *"*Extension and revision of the nomenclature for spiro compounds" (IUPAC Recommendations 1999)", IUPAC, Pure Appl. Chem., (1999), Vol.71, pp.531.538.

An asterisk may be used in subgeneric-formulas or groups to indicate the bond which is connected to a parent or core molecule as defined herein.

The term "treatment" and the like as used herein encompasses eliminating or alleviating symptoms of diseases or disorders and keeping them from worsening (stabilization) and more generally bringing about a desired physiological or pharmacological effect. The term "prevention" and the like as used herein encompasses inhibiting or retarding the manifestation of symptoms of such diseases or disorders or reducing (or increasing as the case may be) or eliminating abnormal values in markers thereof.

### Stereochemistry

Unless specifically indicated, throughout the specification and claims, a given chemical formula or name shall encompass tautomers and all stereo, optical and geometrical isomers *(e.g.* enantiomers, diastereomers, +/-, R/S, E/Z isomers *etc.)* racemic mixtures and racemates thereof. This includes mixtures in different proportions of the separate enantiomers, mixtures of diastereomers, or mixtures of any of the foregoing forms where such isomers and enantiomers exist, as well as salts, including pharmaceutically acceptable salts and solvates thereof such as hydrates, solvates of the free compounds or solvates of a salt of the compound.

### Derivatives of Compounds of the Invention

The invention further encompasses salts, solvates, hydrates, N-oxides, produgs and active metabolites of the compounds of formula I.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, and commensurate with a reasonable benefit/risk ratio.

As used herein, "pharmaceutically acceptable salts" refers to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. For example, such salts include salts from ammonia, L-arginine, betaine, benethamine, benzathine, calcium hydroxide, choline, deanol, diethanolamine (2,2'-iminobis(ethanol)), diethylamine, 2-(diethylamino)-ethanol, 2-aminoethanol, ethylenediamine, N-ethyl-glucamine, hydrabamine, 1H-imidazole, lysine, magnesium hydroxide, 4-(2-hydroxyethyl)-morpholine, piperazine, potassium hydroxide, 1-(2-hydroxyethyl)-pyrrolidine, sodium hydroxide, triethanolamine (2,2',2"-nitrilotris(ethanol)), tromethamine, zinc hydroxide, acetic acid, 2,2-dichloro-acetic acid, adipic acid, alginic acid, ascorbic acid, L-aspartic acid, benzenesulfonic acid, benzoic acid, 2,5-dihydroxybenzoic acid, 4-acetamido-benzoic acid, (+)-camphoric acid, (+)-camphor-10-sulfonic acid, carbonic acid, cinnamic acid, citric acid, cyclamic acid, decanoic acid, dodecylsulfuric acid, ethane-1,2-disulfonic acid, ethanesulfonic acid, 2-hydroxy-ethanesulfonic acid, ethylenediaminetetraacetic acid, formic acid, fumaric acid, galactaric acid, gentisic acid, D-glucoheptonic acid, D-gluconic acid, D-glucuronic acid, glutamic acid, glutaric acid, 2-oxo-glutaric acid, glycerophosphoric acid, glycine, glycolic acid, hexanoic acid, hippuric acid, hydrobromic acid, hydrochloric acid, isobutyric acid, DL-lactic acid, lactobionic acid, lauric acid, lysine, maleic acid, (-)-L-malic acid, malonic acid, DL-mandelic acid, methanesulfonic acid, galactaric acid, naphthalene-1,5-disulfonic acid, naphthalene-2-sulfonic acid, 1-hydroxy-2-naphthoic acid, nicotinic acid, nitric acid, octanoic acid, oleic acid, orotic acid, oxalic acid, palmitic acid, pamoic acid (embonic acid), phosphoric acid, propionic acid, (-)-L-pyroglutamic acid, salicylic acid, 4-amino-salicylic acid, sebacic acid, stearic acid, succinic acid, sulfuric acid, tannic acid, (+)-L-tartaric acid, thiocyanic acid, p-toluenesulfonic acid and undecylenic acid. Further pharmaceutically acceptable salts can be formed with cations from metals such as aluminium, calcium, lithium, magnesium, potassium, sodium, zinc and the like (see Pharmaceutical salts, Berge, S. M. etal., J. Pharm. Sci., (1977), Vol.66, pp. 1-19).

Pharmaceutically acceptable salts of the present invention can be synthesized from the parent compound which contains a basic or acidic moiety by conventional chemical methods. Generally, such salts can be prepared by reacting the free acid or base forms of these compounds with a sufficient amount of the appropriate base or acid in water or in an organic diluent like ether, ethyl acetate, ethanol, isopropanol, or acetonitrile, or a mixture thereof.

Salts of other acids than those mentioned above which for example are useful for purifying or isolating the compounds of the present invention (*e.g*. trifluoro acetate salts), also comprise a part of the invention.

Typically, a pharmaceutically acceptable salt of a compound of formula I may be readily prepared by using a desired acid or base as appropriate. The salt may precipitate from solution and be collected by filtration or may be recovered by evaporation of the solvent. For example, an aqueous solution of an acid such as hydrochloric acid may be added to an aqueous suspension of a compound of formula I and the resulting mixture evaporated to dryness (lyophilized) to obtain the acid addition salt as a solid. Alternatively, a compound of formula I may be dissolved in a suitable solvent, for example an alcohol such as isopropanol, and the acid may be added in the same solvent or another suitable solvent. The resulting acid addition salt may then be precipitated directly, or by addition of a less polar solvent such as diisopropyl ether or hexane, and isolated by filtration.

The acid addition salts of the compounds of formula I may be prepared by contacting the free base form with a sufficient amount of the desired acid to produce the salt in the conventional manner. The free base form may be regenerated by contacting the salt form with a base and isolating the free base in the conventional manner. The free base forms differ from their respective salt forms somewhat in certain physical properties such as solubility in polar solvents, but otherwise the salts are equivalent to their respective free base for purposes of the invention.

Also included are both total and partial salts, that is to say salts with 1, 2 or 3, preferably 2, equivalents of base per mole of acid of formula I or salts with 1, 2 or 3 equivalents, preferably 1 equivalent, of acid per mole of base of formula I.

Pharmaceutically acceptable base addition salts are formed with metals or amines, such as alkali and alkaline earth metals or organic amines. Examples of metals used as cations are sodium, potassium, magnesium, calcium, and the like. Examples of suitable amines are N,N'-dibenzylethylenediamine, chloroprocaine, choline, diethanolamine, dicyclohexylamine, ethylenediamine, N-methylglucamine, and procaine.

The base addition salts of said acidic compounds are prepared by contacting the free acid form with a sufficient amount of the desired base to produce the salt in the conventional manner. The free acid form may be regenerated by contacting the salt form with an acid and isolating the free acid.

Compounds of the invention may have both a basic and an acidic centre and may therefore be in the form of zwitterions or internal salts.

Typically, a pharmaceutically acceptable salt of a compound of formula I may be readily prepared by using a desired acid or base as appropriate. The salt may precipitate from solution and be collected by filtration or may be recovered by evaporation of the solvent. For example, an aqueous solution of an acid such as hydrochloric acid may be added to an aqueous suspension of a compound of formula I and the resulting mixture evaporated to dryness (lyophilized) to obtain the acid addition salt as a solid. Alternatively, a compound of formula I may be dissolved in a suitable solvent, for example an alcohol such as isopropanol, and the acid may be added in the same solvent or another suitable solvent. The resulting acid addition salt may then be precipitated directly, or by addition of a less polar solvent such as diisopropyl ether or hexane, and isolated by filtration.

Those skilled in the art of organic chemistry will appreciate that many organic compounds can form complexes with solvents in which they are reacted or from which they are precipitated or crystallized. These complexes are known as "solvates". For example, a complex with water is known as a "hydrate". Solvates of the compound of the invention are within the scope of the invention. The salts of the compound of formula I may form solvates (e.g., hydrates) and the invention also includes all such solvates. The meaning of the word "solvates" is well known to those skilled in the art as a compound formed by interaction of a solvent and a solute (*i.e.,* solvation). Techniques for the preparation of solvates are well established in the art (see, for example, Brittain. Polymorphism in Pharmaceutical solids. Marcel Decker, New York, 1999.).

The invention also encompasses N-oxides of the compounds of formula I. The term "N-oxide" means that for heterocycles containing an otherwise unsubstituted sp² N atom, the N atom may bear a covalently bound O atom, *i.e.,* -N→O. Examples of such N-oxide substituted heterocycles include pyridyl N-oxides, pyrimidyl N-oxides, pyrazinyl N-oxides and pyrazolyl N-oxides.

The invention also encompasses prodrugs of the compounds of formula I, *i.e.,* compounds which release an active parent drug according to formula I *in vivo* when administered to a mammalian subject. A prodrug is a pharmacologically active or more typically an inactive compound that is converted into a pharmacologically active agent by a metabolic transformation. Prodrugs of a compound of formula I are prepared by modifying functional groups present in the compound of formula I in such a way that the modifications may be cleaved *in vivo* to release the parent compound. *In vivo,* a prodrug readily undergoes chemical changes under physiological conditions (*e.g*., are acted on by naturally occurring enzyme(s)) resulting in liberation of the pharmacologically active agent. Prodrugs include compounds of formula I wherein a hydroxy, amino, or carboxy group of a formula I compound is bonded to any group that may be cleaved in vivo to regenerate the free hydroxyl, amino or carboxy group, respectively. Examples of prodrugs include esters (*e.g*., acetate, formate, and benzoate derivatives) of compounds of formula I or any other derivative which upon being brought to the physiological pH or through enzyme action is converted to the active parent drug. Conventional procedures for the selection and preparation of suitable prodrug derivatives are described in the art (see, for example, Bundgaard. Design of Prodrugs. Elsevier, 1985).

Prodrugs may be administered in the same manner as and in effective amounts analogous to the active ingredient to which they convert or they may be delivered in a reservoir form, *e.g.,* a transdermal patch or other reservoir which is adapted to permit (by provision of an enzyme or other appropriate reagent) conversion of a prodrug to the active ingredient slowly over time, and delivery of the active ingredient to the patient.

The invention also encompasses metabolites. A "metabolite" of a compound disclosed herein is a derivative of a compound which is formed when the compound is metabolised. The term "active metabolite" refers to a biologically active derivative of a compound which is formed when the compound is metabolized. The term "metabolized" refers to the sum of the processes by which a particular substance is changed in the living body. In brief, all compounds present in the body are manipulated by enzymes within the body in order to derive energy and/or to remove them from the body. Specific enzymes produce specific structural alterations to the compound. For example, cytochrome P450 catalyses a variety of oxidative and reductive reactions while uridine diphosphate glucuronyltransferases catalyse the transfer of an activated glucuronic-acid molecule to aromatic alcohols, aliphatic alcohols, carboxylic acids, amines and free sulphydryl groups. Further information on metabolism may be obtained from The Pharmacological Basis of Therapeutics, 9th Edition, McGraw-Hill (1996), pages 11-17.

Metabolites of the compounds disclosed herein can be identified either by administration of compounds to a host and analysis of tissue samples from the host, or by incubation of compounds with hepatic cells in vitro and analysis of the resulting compounds. Both methods are well known in the art.

The term "carrier" refers to a diluent, excipient, and/or vehicle with which an active compound is administered. The pharmaceutical compositions of the invention may contain combinations of more than one carrier. Such pharmaceutical carriers can be sterile liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E.W. Martin, 18th Edition.

A "pharmaceutically acceptable excipient" means an excipient that is useful in preparing a pharmaceutical composition that is generally safe, non-toxic and neither biologically nor otherwise undesirable, and includes an excipient that is acceptable for veterinary use as well as human pharmaceutical use. A "pharmaceutically acceptable excipient" as used in the present application includes both one and more than one such excipient.

The compounds of the invention may be formulated for administration in any convenient way for use in human or veterinary medicine and the invention therefore includes within its scope pharmaceutical compositions comprising a compound of the invention adapted for use in human or veterinary medicine. Such compositions may be presented for use in a conventional manner with the aid of one or more suitable carriers. Acceptable carriers for therapeutic use are well-known in the pharmaceutical art, and are described, for example, in Remington's Pharmaceutical Sciences, Mack Publishing Co. (A. R. Gennaro edit. 1985). The choice of pharmaceutical carrier can be selected with regard to the intended route of administration and standard pharmaceutical practice. The pharmaceutical compositions may comprise as, in addition to, the carrier any suitable binder(s), lubricant(s), suspending agent(s), coating agent(s), and/or solubilizing agent(s).

### Pharmaceutical Compositions Comprising a Compound of Formula I

While it is possible that a compound I may be administered as the bulk substance, it is preferable to present the active ingredient in a pharmaceutical formulation, e.g., wherein the agent is in admixture with a pharmaceutically acceptable carrier selected with regard to the intended route of administration and standard pharmaceutical practice.

Accordingly, the invention further provides a pharmaceutical composition comprising a compound of formula I or a solvate, hydrate, isomer (e.g., enantiomer, diastereomer, etc.), N-oxide or pharmaceutically acceptable salt thereof in admixture with a pharmaceutically acceptable carrier. The term "carrier" refers to a diluent, excipient, and/or vehicle with which an active compound is administered.

A compound of formula I may be used in combination with other therapies and/or active agents. Accordingly, the invention provides, in a further aspect, a pharmaceutical composition comprising a compound of formula I or a solvate, hydrate, isomer (e.g., enantiomer, diastereomer, etc.), N-oxide or pharmaceutically acceptable salt thereof, a second active agent, and a pharmaceutically acceptable carrier.

The pharmaceutical compositions may comprise as, in addition to, the carrier any suitable binder, lubricant, suspending agent, coating agent and/or solubilizing agent.

Preservatives, stabilizers, dyes and flavouring agents also may be provided in the pharmaceutical composition. Antioxidants and suspending agents may be also used.

The compounds of the invention may be reduced to fine particulate form (e.g., milled using known milling procedures such as wet milling) to obtain a particle size appropriate for tablet formation and for other formulation types. Finely divided (nanoparticulate) preparations of the compounds of the invention may be prepared by processes known in the art, for example see WO02/00196.

### Routes of Administration and Unit Dosage Forms

The routes for administration include oral *(e.g.,* as a tablet, capsule, or as an ingestible solution), topical, mucosal (*e.g*., as a nasal spray or aerosol for inhalation), nasal, parenteral (*e.g*., by an injectable form), gastrointestinal, intraspinal, intraperitoneal, intramuscular, intravenous, intrauterine, intraocular, intradermal, intracranial, intrathecal, intratracheal, intravaginal, intracerebroventricular, intracerebral, subcutaneous, ophthalmic (including intravitreal or intracameral), transdermal, rectal, buccal, epidural and sublingual. The compositions of the invention may be especially formulated for any of those administration routes. In preferred embodiments, the pharmaceutical compositions of the invention are formulated in a form that is suitable for oral delivery.

There may be different composition/formulation requirements depending on the different delivery systems. It is to be understood that not all of the compounds need to be administered by the same route. Likewise, if the composition comprises more than one active component, then those components may be administered by different routes. By way of example, the pharmaceutical composition of the invention may be formulated to be delivered using a mini-pump or by a mucosal route, for example, as a nasal spray or aerosol for inhalation or ingestible solution, or parenterally in which the composition is formulated by an injectable form, for delivery, by, for example, an intravenous, intramuscular or subcutaneous route. Alternatively, the formulation may be designed to be delivered by multiple routes.

Where the agent is to be delivered mucosally through the gastrointestinal mucosa, it should be able to remain stable during transit though the gastrointestinal tract; for example, it should be resistant to proteolytic degradation, stable at acid pH and resistant to the detergent effects of bile. For example, the compound of Formula I may be coated with an enteric coating layer. The enteric coating layer material may be dispersed or dissolved in either water or in a suitable organic solvent. As enteric coating layer polymers, one or more, separately or in combination, of the following can be used; e.g., solutions or dispersions of methacrylic acid copolymers, cellulose acetate phthalate, cellulose acetate butyrate, hydroxypropyl methylcellulose phthalate, hydroxypropyl methylcellulose acetate succinate, polyvinyl acetate phthalate, cellulose acetate trimellitate, carboxymethylethylcellulose, shellac or other suitable enteric coating layer polymer(s). For environmental reasons, an aqueous coating process may be preferred. In such aqueous processes methacrylic acid copolymers are most preferred.

When appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intravenously, intramuscularly or subcutaneously. For buccal or sublingual administration, the compositions may be administered in the form of tablets or lozenges, which can be formulated in a conventional manner.

When the composition of the invention is to be administered parenterally, such administration includes one or more of: intravenously, intraarterially, intraperitoneally, intrathecally, intraventricularly, intraurethrally, intrasternally, intracranially, intramuscularly or subcutaneously administering the agent; and/or by using infusion techniques.

Pharmaceutical compositions of the invention can be administered parenterally, *e.g.,* by infusion or injection. Pharmaceutical compositions suitable for injection or infusion may be in the form of a sterile aqueous solution, a dispersion or a sterile powder that contains the active ingredient, adjusted, if necessary, for preparation of such a sterile solution or dispersion suitable for infusion or injection. This preparation may optionally be encapsulated into liposomes. In all cases, the final preparation must be sterile, liquid, and stable under production and storage conditions. To improve storage stability, such preparations may also contain a preservative to prevent the growth of microorganisms. Prevention of the action of micro-organisms can be achieved by the addition of various antibacterial and antifungal agents, *e.g.,* paraben, chlorobutanol, sodium acetate, sodium lactate, sodium citrate or ascorbic acid. In many cases isotonic substances are recommended, *e.g.,* sugars, buffers and sodium chloride to assure osmotic pressure similar to those of body fluids, particularly blood. Prolonged absorption of such injectable mixtures can be achieved by introduction of absorption-delaying agents, such as aluminium monostearate or gelatin.

Dispersions can be prepared in a liquid carrier or intermediate, such as glycerin, liquid polyethylene glycols, triacetin oils, and mixtures thereof. The liquid carrier or intermediate can be a solvent or liquid dispersive medium that contains, for example, water, ethanol, a polyol *(e.g.,* glycerol, propylene glycol or the like), vegetable oils, non-toxic glycerine esters and suitable mixtures thereof. Suitable flowability may be maintained, by generation of liposomes, administration of a suitable particle size in the case of dispersions, or by the addition of surfactants.

For parenteral administration, the compound is best used in the form of a sterile aqueous solution which may contain other substances, for example, enough salts or glucose to make the solution isotonic with blood. The aqueous solutions should be suitably buffered (preferably to a pH of from 3 to 9), if necessary. The preparation of suitable parenteral formulations under sterile conditions is readily accomplished by standard pharmaceutical techniques well-known to those skilled in the art.

Sterile injectable solutions can be prepared by mixing a compound of formula I with an appropriate solvent and one or more of the aforementioned carriers, followed by sterile filtering. In the case of sterile powders suitable for use in the preparation of sterile injectable solutions, preferable preparation methods include drying in vacuum and lyophilization, which provide powdery mixtures of the aldosterone receptor antagonists and desired excipients for subsequent preparation of sterile solutions.

The compounds according to the invention may be formulated for use in human or veterinary medicine by injection *(e.g.,* by intravenous bolus injection or infusion or *via* intramuscular, subcutaneous or intrathecal routes) and may be presented in unit dose form, in ampoules, or other unit-dose containers, or in multi-dose containers, if necessary with an added preservative. The compositions for injection may be in the form of suspensions, solutions, or emulsions, in oily or aqueous vehicles, and may contain formulatory agents such as suspending, stabilizing, solubilizing and/or dispersing agents. Alternatively, the active ingredient may be in sterile powder form for reconstitution with a suitable vehicle, *e.g.,* sterile, pyrogen-free water, before use.

The compounds of the invention can be administered *(e.g.,* orally or topically) in the form of tablets, capsules, ovules, elixirs, solutions or suspensions, which may contain flavouring or colouring agents, for immediate-, delayed-, modified-, sustained-, pulsed-or controlled-release applications.

The compounds of the invention may also be presented for human or veterinary use in a form suitable for oral or buccal administration, for example in the form of solutions, gels, syrups, mouth washes or suspensions, or a dry powder for constitution with water or other suitable vehicle before use, optionally with flavouring and colouring agents. Solid compositions such as tablets, capsules, lozenges, pastilles, pills, boluses, powder, pastes, granules, bullets or premix preparations may also be used. Solid and liquid compositions for oral use may be prepared according to methods well-known in the art. Such compositions may also contain one or more pharmaceutically acceptable carriers and excipients which may be in solid or liquid form.

The tablets may contain excipients such as microcrystalline cellulose, lactose, sodium citrate, calcium carbonate, dibasic calcium phosphate and glycine, disintegrants such as starch (preferably corn, potato or tapioca starch), sodium starch glycolate, croscarmellose sodium and certain complex silicates, and granulation binders such as polyvinylpyrrolidone, hydroxypropylmethylcellulose (HPMC), hydroxypropylcellulose (HPC), sucrose, gelatin and acacia.

Additionally, lubricating agents such as magnesium stearate, stearic acid, glyceryl behenate and talc may be included.

The compositions may be administered orally, in the form of rapid or controlled release tablets, microparticles, mini tablets, capsules, sachets, and oral solutions or suspensions, or powders for the preparation thereof. In addition to the new solid-state forms of pantoprazole of the invention as the active substance, oral preparations may optionally include various standard pharmaceutical carriers and excipients, such as binders, fillers, buffers, lubricants, glidants, dyes, disintegrants, odourants, sweeteners, surfactants, mold release agents, antiadhesive agents and coatings. Some excipients may have multiple roles in the compositions, *e.g.,* act as both binders and disintegrants.

Examples of pharmaceutically acceptable disintegrants for oral compositions include starch, pre-gelatinized starch, sodium starch glycolate, sodium carboxymethylcellulose, croscarmellose sodium, microcrystalline cellulose, alginates, resins, surfactants, effervescent compositions, aqueous aluminum silicates and cross-linked polyvinylpyrrolidone.

Examples of pharmaceutically acceptable binders for oral compositions include acacia; cellulose derivatives, such as methylcellulose, carboxymethylcellulose, hydroxypropylmethylcellulose, hydroxypropylcellulose or hydroxyethylcellulose; gelatin, glucose, dextrose, xylitol, polymethacrylates, polyvinylpyrrolidone, sorbitol, starch, pre-gelatinized starch, tragacanth, xanthane resin, alginates, magnesium-aluminum silicate, polyethylene glycol or bentonite.

Examples of pharmaceutically acceptable fillers for oral compositions include lactose, anhydrolactose, lactose monohydrate, sucrose, dextrose, mannitol, sorbitol, starch, cellulose (particularly microcrystalline cellulose), dihydro- or anhydro-calcium phosphate, calcium carbonate and calcium sulphate.

Examples of pharmaceutically acceptable lubricants useful in the compositions of the invention include magnesium stearate, talc, polyethylene glycol, polymers of ethylene oxide, sodium lauryl sulphate, magnesium lauryl sulphate, sodium oleate, sodium stearyl fumarate, and colloidal silicon dioxide.

Examples of suitable pharmaceutically acceptable odourants for the oral compositions include synthetic aromas and natural aromatic oils such as extracts of oils, flowers, fruits *(e.g.,* banana, apple, sour cherry, peach) and combinations thereof, and similar aromas. Their use depends on many factors, the most important being the organoleptic acceptability for the population that will be taking the pharmaceutical compositions.

Examples of suitable pharmaceutically acceptable dyes for the oral compositions include synthetic and natural dyes such as titanium dioxide, beta-carotene and extracts of grapefruit peel.

Examples of useful pharmaceutically acceptable coatings for the oral compositions, typically used to facilitate swallowing, modify the release properties, improve the appearance, and/or mask the taste of the compositions include hydroxypropylmethylcellulose, hydroxypropylcellulose and acrylate-methacrylate copolymers.

Examples of pharmaceutically acceptable sweeteners for the oral compositions include aspartame, saccharin, saccharin sodium, sodium cyclamate, xylitol, mannitol, sorbitol, lactose and sucrose.

Examples of pharmaceutically acceptable buffers include citric acid, sodium citrate, sodium bicarbonate, dibasic sodium phosphate, magnesium oxide, calcium carbonate and magnesium hydroxide.

Examples of pharmaceutically acceptable surfactants include sodium lauryl sulphate and polysorbates.

Solid compositions of a similar type may also be employed as fillers in gelatin capsules. Preferred excipients in this regard include lactose, starch, a cellulose, milk sugar or high molecular weight polyethylene glycols. For aqueous suspensions and/or elixirs, the agent may be combined with various sweetening or flavouring agents, colouring matter or dyes, with emulsifying and/or suspending agents and with diluents such as water, ethanol, propylene glycol and glycerin, and combinations thereof.

The compounds of the invention may also, for example, be formulated as suppositories *e.g.,* containing conventional suppository bases for use in human or veterinary medicine or as pessaries *e.g.,* containing conventional pessary bases.

The compounds according to the invention may be formulated for topical administration, for use in human and veterinary medicine, in the form of ointments, creams, gels, hydrogels, lotions, solutions, shampoos, powders (including spray or dusting powders), pessaries, tampons, sprays, dips, aerosols, drops *(e.g.,* eye ear or nose drops) or pour-ons.

For application topically to the skin, the agent of the invention can be formulated as a suitable ointment containing the active compound suspended or dissolved in, for example, a mixture with one or more of the following: mineral oil, liquid petrolatum, white petrolatum, propylene glycol, polyoxyethylene polyoxypropylene compound, emulsifying wax, sorbitan monostearate, a polyethylene glycol, liquid paraffin, polysorbate 60, cetyl esters wax, cetearyl alcohol, 2-octyldodecanol, benzyl alcohol, and water. Such compositions may also contain other pharmaceutically acceptable excipients, such as polymers, oils, liquid carriers, surfactants, buffers, preservatives, stabilizers, antioxidants, moisturizers, emollients, colourants, and odourants.

Examples of pharmaceutically acceptable polymers suitable for such topical compositions include acrylic polymers; cellulose derivatives, such as carboxymethylcellulose sodium, methylcellulose or hydroxypropylcellulose; natural polymers, such as alginates, tragacanth, pectin, xanthan and cytosan.

Examples of suitable pharmaceutically acceptable oils which are so useful include mineral oils, silicone oils, fatty acids, alcohols, and glycols.

Examples of suitable pharmaceutically acceptable liquid carriers include water, alcohols or glycols such as ethanol, isopropanol, propylene glycol, hexylene glycol, glycerol and polyethylene glycol, or mixtures thereof in which the pseudopolymorph is dissolved or dispersed, optionally with the addition of non-toxic anionic, cationic or non-ionic surfactants, and inorganic or organic buffers.

Examples of pharmaceutically acceptable preservatives include sodium benzoate, ascorbic acid, esters of p-hydroxybenzoic acid and various antibacterial and antifungal agents such as solvents, for example ethanol, propylene glycol, benzyl alcohol, chlorobutanol, quaternary ammonium salts, and parabens (such as methyl paraben, ethyl paraben and propyl paraben).

Examples of pharmaceutically acceptable stabilizers and antioxidants include ethylenediaminetetraacetic acid (EDTA), thiourea, tocopherol and butyl hydroxyanisole.

Examples of pharmaceutically acceptable moisturizers include glycerine, sorbitol, urea and polyethylene glycol.

Examples of pharmaceutically acceptable emollients include mineral oils, isopropyl myristate, and isopropyl palmitate.

The compounds may also be dermally or transdermally administered, for example, by use of a skin patch.

For ophthalmic use, the compounds can be formulated as micronized suspensions in isotonic, pH adjusted, sterile saline, or, preferably, as solutions in isotonic, pH adjusted, sterile saline, optionally in combination with a preservative such as a benzylalkonium chloride.

As indicated, the compounds of the invention can be administered intranasally or by inhalation and is conveniently delivered in the form of a dry powder inhaler or an aerosol spray presentation from a pressurized container, pump, spray or nebulizer with the use of a suitable propellant, *e.g.*, dichlorodifluoromethane, trichlorofluoromethane, dichlorotetrafluoroethane, a hydrofluoroalkane such as 1,1,1,2-tetrafluoroethane (HFA 134AT) or 1,1,1,2,3,3,3-heptafluoropropane (HFA 227EA), carbon dioxide or other suitable gas. In the case of a pressurized aerosol, the dosage unit may be determined by providing a valve to deliver a metered amount. The pressurized container, pump, spray or nebulizer may contain a solution or suspension of the active compound, e.g., using a mixture of ethanol and the propellant as the solvent, which may additionally contain a lubricant, e.g., sorbitan trioleate.

Capsules and cartridges (made, for example, from gelatin) for use in an inhaler or insufflator may be formulated to contain a powder mix of the compound and a suitable powder base such as lactose or starch.

For topical administration by inhalation the compounds according to the invention may be delivered for use in human or veterinary medicine *via* a nebulizer.

The pharmaceutical compositions of the invention may contain from 0.01 to 99% weight per volume of the active material. For topical administration, for example, the composition will generally contain from 0.01-10%, more preferably 0.01-1% of the active material.

The active agents can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The pharmaceutical composition or unit dosage form of the invention may be administered according to a dosage and administration regimen defined by routine testing in the light of the guidelines given above in order to obtain optimal activity while minimizing toxicity or side effects for a particular patient. However, such fine tuning of the therapeutic regimen is routine in the light of the guidelines given herein.

The dosage of the active agents of the invention may vary according to a variety of factors such as underlying disease conditions, the individual's condition, weight, gender and age, and the mode of administration. An effective amount for treating a disorder can easily be determined by empirical methods known to those of ordinary skill in the art, for example by establishing a matrix of dosages and frequencies of administration and comparing a group of experimental units or subjects at each point in the matrix. The exact amount to be administered to a patient will vary depending on the state and severity of the disorder and the physical condition of the patient. A measurable amelioration of any symptom or parameter can be determined by a person skilled in the art or reported by the patient to the physician.

The amount of the agent to be administered can range between about 0.01 and about 25 mg/kg/day, preferably between about 0.1 and about 10 mg/kg/day and most preferably between 0.2 and about 5 mg/kg/day. It will be understood that the pharmaceutical formulations of the invention need not necessarily contain the entire amount of the agent that is effective in treating the disorder, as such effective amounts can be reached by administration of a plurality of doses of such pharmaceutical formulations. In general, an "effective amount" refers to the amount of a pharmaceutical composition administered to improve, inhibit, or ameliorate a disease or disorder or condition of a subject, or a symptom of a disease or disorder, in a clinically relevant manner. Any clinically relevant improvement in the subject is considered sufficient to achieve treatment. Preferably, an amount sufficient to treat is an amount that prevents the occurrence or one or more symptoms of the infection or is an amount that reduces the severity of, or the length of time during which a subject suffers from, or develops, one or more symptoms of the infection relative to a control subject that is not treated with a composition of the invention).

In a preferred embodiment of the invention, the compounds according to formula I are formulated in capsules or tablets, preferably containing 10 to 200 mg of the compounds of the invention, and are preferably administered to a patient at a total daily dose of 10 to 300 mg, preferably 20 to 150 mg and most preferably about 50 mg.

A pharmaceutical composition for parenteral administration contains from about 0.01% to about 100% by weight of the active agents of the invention, based upon 100% weight of total pharmaceutical composition.

Generally, transdermal dosage forms contain from about 0.01% to about 100% by weight of the active agents versus 100% total weight of the dosage form.

The pharmaceutical composition or unit dosage form may be administered in a single daily dose, or the total daily dosage may be administered in divided doses. In addition, co-administration or sequential administration of another compound for the treatment of the disorder may be desirable. To this purpose, the combined active principles are formulated into a simple dosage unit.

For combination treatment where the compounds are in separate dosage formulations, the compounds can be administered concurrently, or each can be administered at staggered intervals. Additional compounds may be administered at specific intervals too. The order of administration will depend upon a variety of factors including age, weight, gender and medical condition of the patient; the severity and aetiology of the disorders to be treated, the route of administration, the renal and hepatic function of the patient, the treatment history of the patient, and the responsiveness of the patient. Determination of the order of administration may be fine-tuned and such fine-tuning is routine in the light of the guidelines given herein.

### DESCRIPTION OF THE INVENTION

### Synthesis

Compounds of formula I, or indeed those of formulae 1a through 1f, and enantiomers, diastereomers, N-oxides, and pharmaceutically acceptable salts thereof, may be prepared by the general methods outlined hereinafter, said methods constituting a further aspect of the invention.

The compounds of this invention can be prepared by employing reactions as shown in the following schemes, in addition to other standard manipulations that are known in the literature, exemplified in the experimental section or clear to one skilled in the art. The starting materials which are not described herein are either commercially available or may be prepared by employing reactions described in the literature or are clear to one skilled in the art. The following examples are provided so that the invention might be more fully understood, are illustrative only, and should not be construed as limiting.

It will be appreciated by those skilled in the art that it may be desirable to use protected derivatives of intermediates used in the preparation of the compounds according to formula I. Protection and deprotection of functional groups may be performed by methods known in the art (see, for example, Green and Wuts Protective Groups in Organic Synthesis. John Wiley and Sons, New York, 1999).

The abbreviation PG describes a "protecting group" which is introduced to a reactive group before a certain manipulation is carried out, and which is later removed. Examples of PG's for protecting a reactive group include: acetyl-, trifluoracetyl-, benzoyl-, ethoxycarbonyl-, N-tert-butoxycarbonyl- (BOC), N-benzyloxycarbonyl- (Cbz), benzyl-, methoxybenzyl-, 2,4-dimethoxybenzyl- and for amino groups additionally the phthalyl- group for amino-alkylamino or imino groups; N-methoxynethyl- (MOM), N-benzyloxymethyl-(BOM), N-(trimethylsilyl)ethoxymethyl- (SEM), N-tert-butyl-dimethylsiloxymethyl-, N-tert-butyl-dimethylsilyl- (TBDMS), N-triisopropylsilyl- (TIPS), N-benzyl-, N-4-methoxybenzyl (PMB), N-triphenylmethyl- (Tr), N-tert-butoxycarbonyl- (BOC), N-benzyloxycarbonyl- (Cbz) or N-trimethylsilylethylsulfonyl- (SES) for amide groups; methoxy-, benzyloxy-, trimethylsilyl- (TMS), acetyl-, benzoyl-, tert-butyl-, trityl-, benzyl-, or tetrahydropyranyl (THP) groups for hydroxy groups; or trimethylsilyl- (TMS), methyl-ethyl-, tert-butyl-, benzyl-, or tetrahydropyranyl (THP) groups for carboxyl groups.

The compounds of the invention are generally prepared according to the following different schemes. In some cases, the final product may be further modified, for example by manipulation of substituents. These manipulations may include, but are not limited to, reduction, oxidation, alkylation, acylation and hydrolysis reactions, which are commonly known to those skilled in the art. In some cases, the order of carrying out the following reactions may be varied in order to facilitate the reaction or to avoid unwanted reaction products or side reactions. The following examples are provided so that the invention might be more fully understood. These examples are illustrative only and should not be constructed as limiting the invention in any way.

As shown in Scheme 1 below, Compounds **6,** where the group nR1 is an optionally substituted amino group, can be synthesised starting from commercially available compound 1, which can be selectively converted by nucleophilic aromatic substitution into 3-(R₂-NH)-5-halopyridine-2-carbonitriles (2) by using consolidated methodologies well known to people skilled in the art. In turn, 2 is condensed with the proper diamines (3) to obtain 2-(4,5-dihydro-1H-imidazol-2-yl)- or 2-(1,4,5,6-tetrahydropyrimidin-2-yl) derivatives **4** e.g. by condensing the starting nitrile with the proper diamine or its ammonium salts heating in a suitable solvent like N,N-dimethylacetamide (DMA). Compounds **4** undergo cyclization by selected reagents (e.g. 1,1'-carbonyldiimidazole (CDI), 2,2,2-trichloroethylisocyanate, triphosgene or alternative carbonylating reagent) to afford the corresponding intermediates **5**, which are finally reacted to give the nR₁-substituted compounds **6**, falling under the scope of formula **1a.** This last derivatization procedure can be carried out using standard methods such as *e.g.* Palladium catalyzed cross coupling reactions like Buchwald reactions, acylation reactions, alkylation or any kind of N-derivatization reaction useful to the aim of forming compounds according to formula **1a**, and is very well known to people skilled in the art.

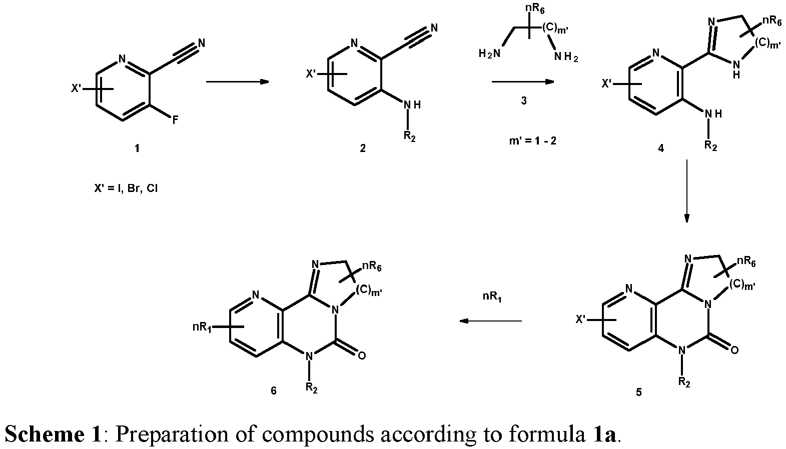

Alternatively, compounds of formula **1a** can be obtained using the procedure illustrated below.

As shown in Scheme 2 , commercially available compound **1**, that is 3,5-difluoro-2-pyridinecarbonitrile can be selectively converted by classical nucleophilic aromatic substitution with amino compounds nR₁ into 5-(R₁-optionally substituted amino)-3-fluoropyridine-2-carbonitrile (**2**), which in turn is reacted under heating with the substituted benzylamino reagent R₂N to obtain the 5-(R₁- optionally substituted amino)-3-(R₂-amino)-pyridine-2-carbonitrile (**3**), by using consolidated methodologies well known to the people skilled in the art such as nucleophilic aromatic substitution. Afterwards, Intermediates **3** are reacted with the proper diamines **4** to obtain 2-(4,5-dihydro-1H-imidazol-2-yl)- or 2-(1,4,5,6-tetrahydropyrimidin-2-yl) derivatives **5**. In the next step, compounds 5 are cyclized by the proper reactant to afford the corresponding compounds 6, falling under the scope of formula **1a**. This last cyclization procedure can be carried out using standard carbonylating reagents such as 1,1'-carbonyldiimidazole (CDI) or bis(trichloromethyl) carbonate (triphosgene) or alternative methods useful to the scope of forming compounds according to formula **1a**, and very well known to people skilled in the art.

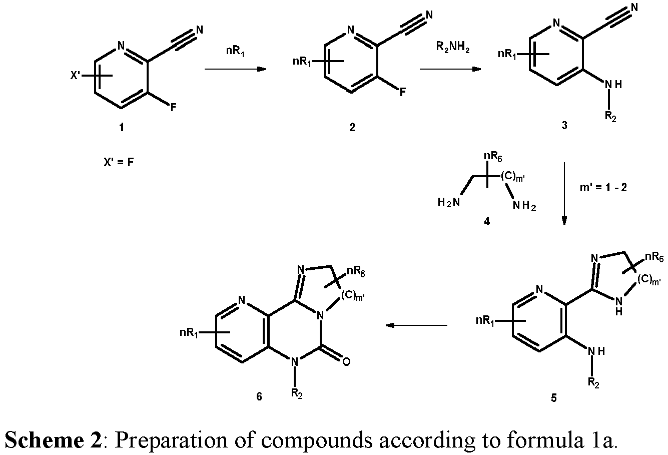

Compounds of formula **1a** where A is a carbon atom may be synthesized from different starting materials following a different reaction procedure shown below (Scheme 3). The same reaction sketch can also be useful to prepare Compounds of formula **1b** where A is a nitrogen atom.

As depicted in Scheme 3 (below), commercially available compound **1** can be selectively converted by nucleophilic aromatic substitution with amines R₂N into 3-(R₂-amino)-5-halopyridine-2-carboxylic acid alkyl ester or 4-(R₂-amino)-6-halo-pyridazine-3-carboxylic acid alkyl esters (**2**) by using consolidated methodologies well known to people skilled in the art. In turn, esters **2** are reacted with ammonium hydroxide to obtain the primary amides (3), which then undergo to nucleophilic aromatic substitution with nR₁ to obtain the 3-(R₂-amino)-5-(R₁-amino)-pyridine-2-carboxamide (**4**) or 4-(R₂-amino)-6-(R₁-amino)-pyridazine-3-carboxamide. In the following step, compounds **4** are then cyclized by the proper reactant as described above (scheme 1 and 2) to afford the corresponding compounds **5**, which are in turn reacted with the thionating Lawesson's reagent or an alternative thionating reagent to obtain the thioxoderivatives **6**. These thioxo compounds are then S-methylated by e.g. MeI to obtain compounds 7. Methylthio derivatives 7 are reacted with 1,2 or 1,3 optionally alkyl substituted aminoalcohol to give the corresponding amino derivatives via nucleophilic substitution of the methylthio group by the amino group of the reactant. These last intermediates are not isolated and directly cyclized by conversion of the hydroxy group into its mesylate (or alternative suitable leaving group e.g., a halogen atom) using methanesulfonyl chloride or other proper reagent like e.g. thionyl chloride. These procedures afford compounds falling under the scope of formula **1a** or **1b**. The group of procedures referring to Scheme **3** can be carried out using standard methods of alkylation, amination and activation of a hydroxy group as a leaving group, well described in the literature and known to the people skilled in the art.

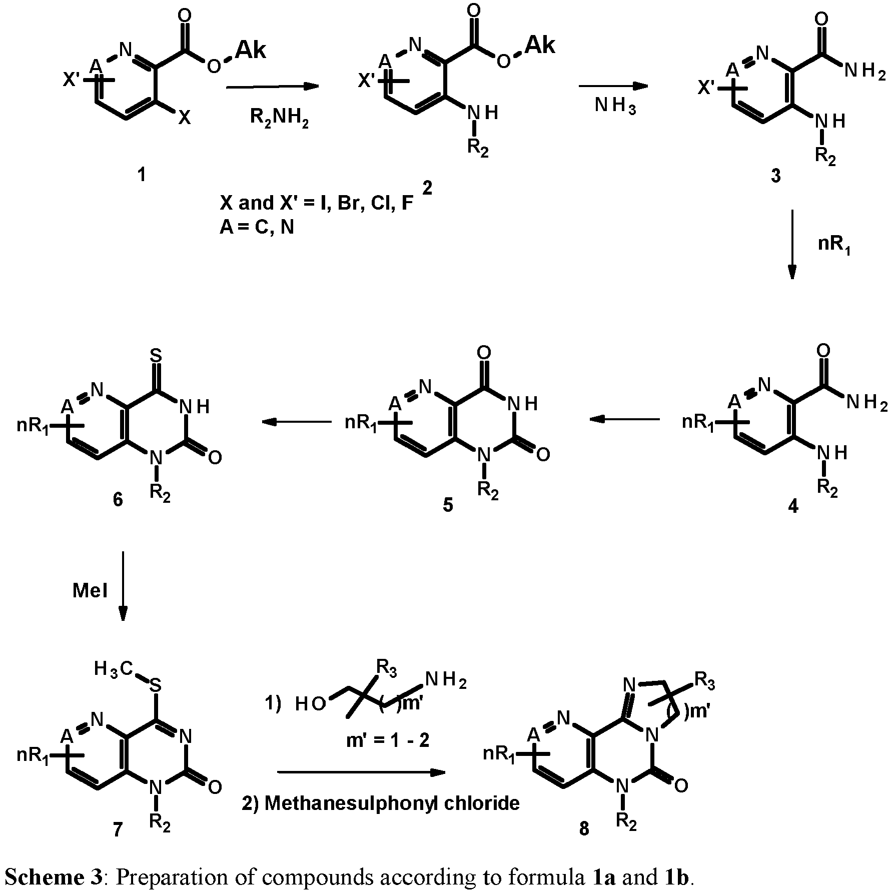

As shown in Scheme 4 below, commercially available compound **1** can be selectively converted by aromatic nucleophilic substitution into 3-halo-5-(R₁-amino)-pyridine-2-carbonitrile or 4-(R₁-amino)-6-halopyridazine-3-carbonitrile (**2**) by using consolidated methodologies well known to people skilled in the art. In turn, derivatives **2** are reacted with benzylamines to obtain the 3-(R₂-amino)-5-(R₁-amino)-pyridine-2-carbonitrile or 4-(R₂-amino)-6-(R₁-amino)-pyridazine-3-carbonitrile (**3**). Compounds **3** are reacted with e.g. triphosgene in pyridine, to obtain the carbamoyl chlorides **4,** which are in turn reacted with commercially available aminoacid carboxylic esters **5** to afford ureas **6.** Afterwards, the latter are cyclized under basic condition (e.g. DBU or sodium carbonate or the like) to obtain compounds 7 falling under the scope of formula **1c** or **1d**. The reactions described in Scheme 4 are well documented inside the Experimental part.

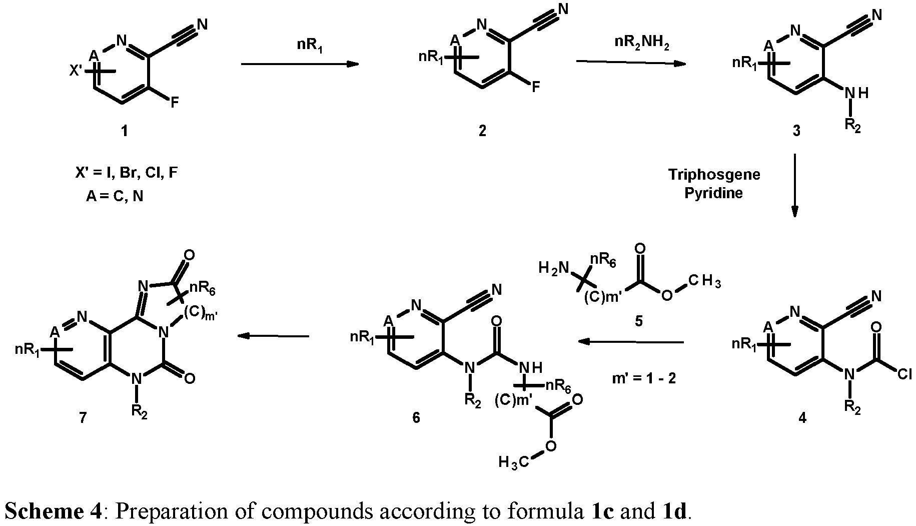

Compounds of Formula **1d** can be prepared as shown in Scheme 5 below. Commercially available compound **1** can be selectively converted by nucleophilic aromatic substitution with the proper benzylamino derivatives into the 4-(R₂-amino)-6-halopyridazine-3-carboxylic methyl ester (**2**) by using consolidated methodologies well known to people skilled in the art. In turn, esters **2** can be reacted with ammonium hydroxide to obtain the primary amides (**3**), which undergo nucleophilic aromatic substitution to give the 4-(R₂-amino)-6-(R₁-amino)-pyridazine-3-carboxamide (**4**). By reacting **4** with POCl₃ (or other well-known dehydrating reagent) carbonitrile derivatives **5** are prepared. Compounds **5** are reacted with e.g. triphosgene in pyridine, to obtain the carbamoyl chlorides **6**, which are in turn reacted with commercially available aminoacid carboxylic esters 7 to afford ureas **8**. Afterwards, the latter are cyclized under basic condition (e.g. DBU or sodium carbonate or the like) to obtain compounds **9** falling under the scope of formula **1d**. The reactions described in Scheme 5 are well documented inside the Experimental part.

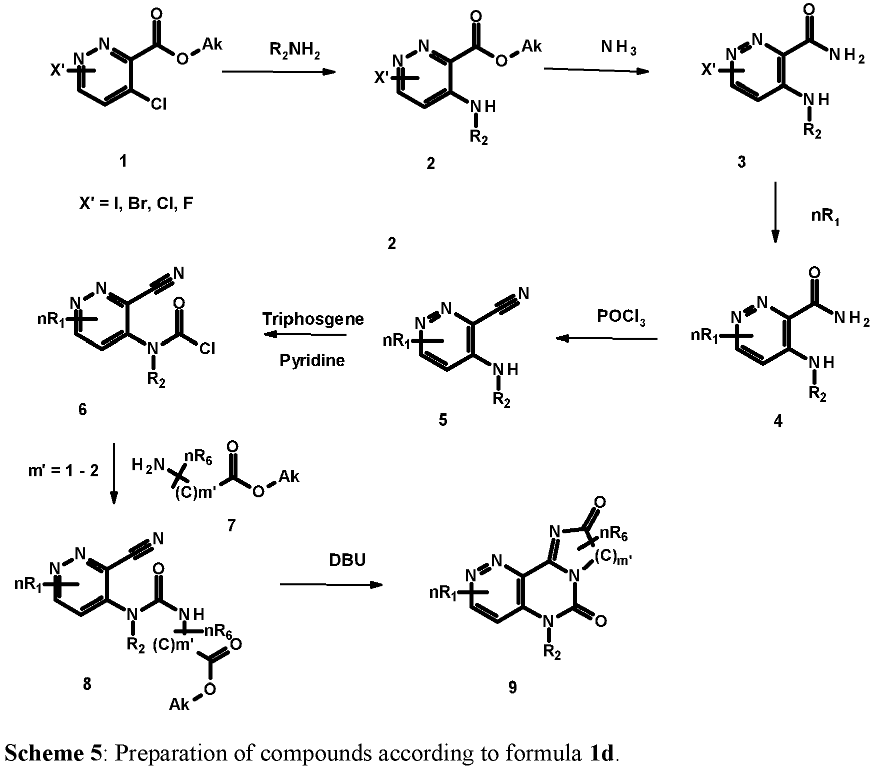

As shown in Scheme 6 below, commercially available compound **1** can be converted into alkyl 3-(R₂-amino)-5-halopyridine-2-carboxylate (**2**) either by reductive amination with the proper aldehyde where Y' is NH₂ or by aromatic substitution where Y' is halogen. Both these conversions can be performed by using consolidated methodologies well known to people skilled in the art. Afterwards, compounds **2** are cyclized by methods cited above to afford the corresponding compounds **3,** which are in turn reacted with POCl₃ to obtain haloderivatives **4.** Compounds **4** are then cyclized with the proper hydrazide to obtain tricyclic derivatives **5**. Afterwards, compounds **5,** where X' is a halogen atom, are reacted to give nR₁-substituted compounds falling under the scope of formula 1e. This derivatization procedure can be carried out using standard methods such as e.g., Buchwald or alternative metal catalysed amination reactions, aromatic nucleophilic reactions. If further functionalization is needed, deprotection or hydrolysis reactions followed by acylation, alkylation reductive-amination or any kind of N-derivatization reaction, very well known to people skilled in the art and useful to the aim of forming compounds according to formula **1e**, can be conducted. Prolonged heating of a solution of compounds **6** in the presence or not of an acid or basic catalyst may cause a partial conversion into the thermodynamically more stable compounds **7.** This reaction is very well known to the people skilled in the art as Dimroth rearrangement. Partial conversion by Dimroth rearrangement can also occur during the cyclization to Compounds **5** or the following conversion of **5** to **6,** generating mixture of **5** and **6** with the corresponding rearranged product, which are not isolated.

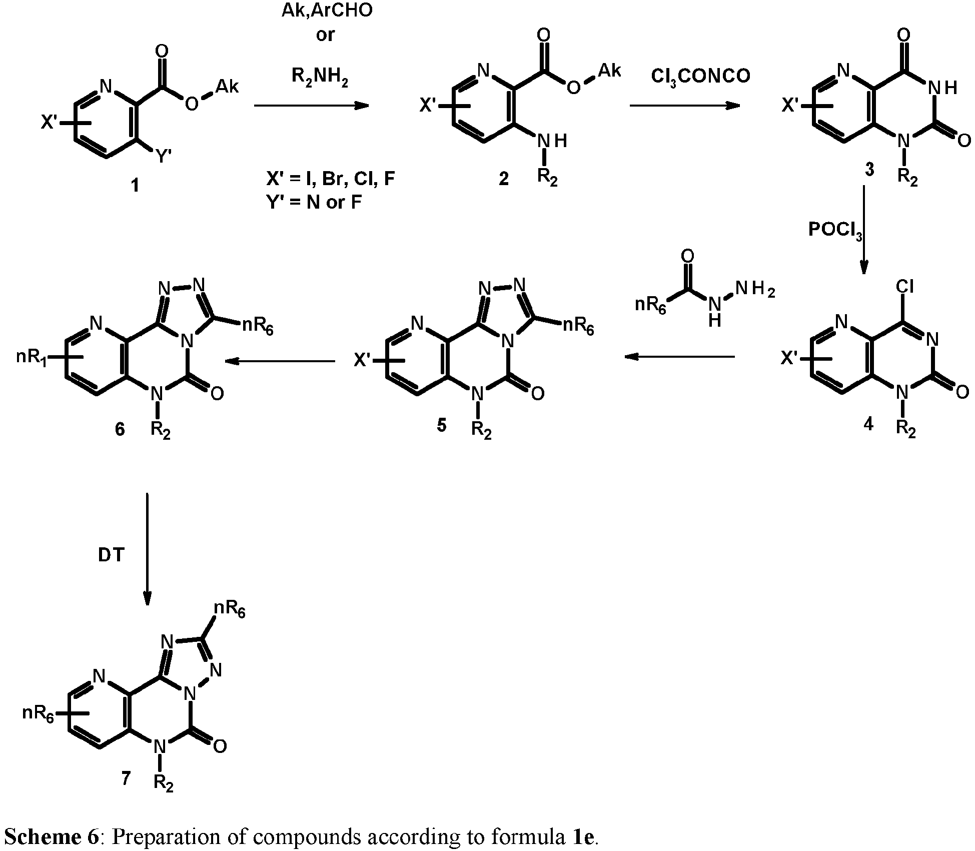

Alternatively, as shown in Scheme 7 below, commercially available compound **1** can be converted by aromatic nucleophilic substitution or metal catalysed amination reactions, using consolidated methodologies well known to people skilled in the art, into the 3-amino- or 3-halo-5-nR₁-pyridine-2-carbonitrile **2**. Compounds **2** are then converted either by reductive amination with the proper aldehyde or by aromatic substitution with the proper aminoderivatives, using consolidated methodologies well known to people skilled in the art, to a the 3-(R₂-amino)-5-nR₁-pyridine-2-carbonitrile intermediates **3.** Compounds **3** are then converted to carboxamides **4** by partial hydrolysis (e.g. with hydrogen peroxide and sodium hydroxide). In turn, compounds **4** are cyclized by the already described carbonylating reagents (see above) to afford the corresponding compounds **5,** which undergo thionation with Lawesson's reagent to obtain the thioxo derivatives **6.** Afterwards, **6** is reacted with the proper hydrazides **7** to obtain the corresponding hydrazones (not isolated), which are cyclized as crude with a cyclodehydrating reagent (such as Burgess reagent) to afford **8**. Hydrazides **7** are either commercially available or can be prepared following known procedures to person skilled in the art.

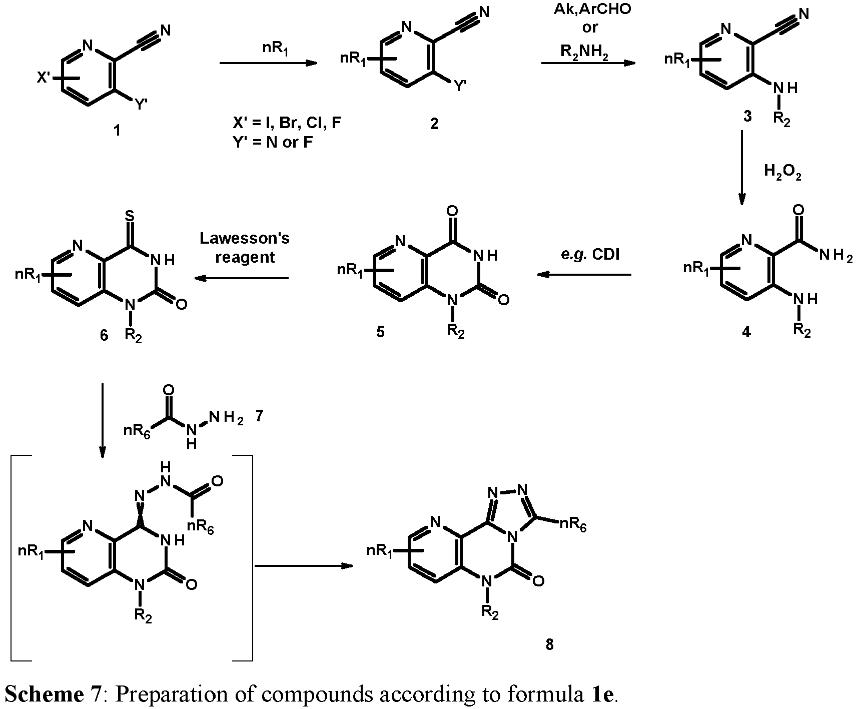

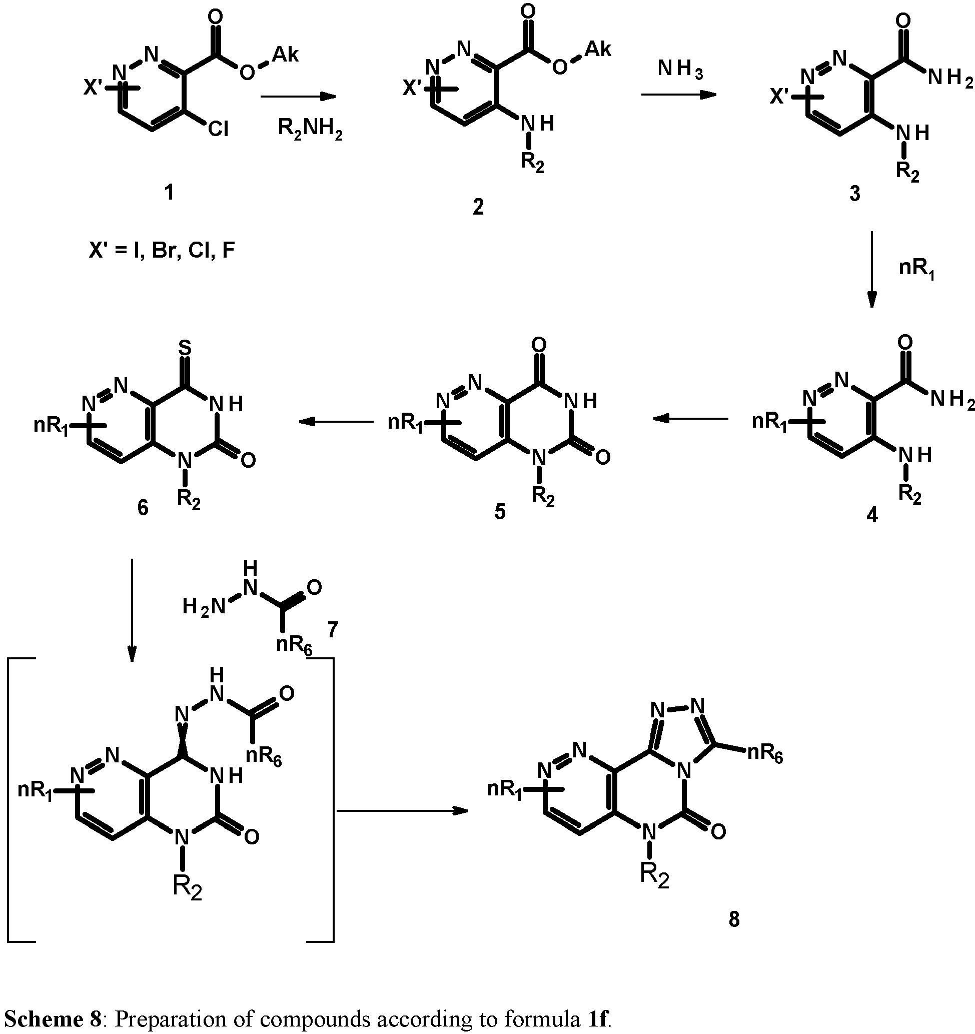

Compounds according to formula **1f** can be prepared as drawn in Scheme 8. Intermediates **6** can be prepared following the methodology described for Intermediate **6** belonging to Scheme 3. Afterwards, compounds **6** are reacted with the proper hydrazides **7** to obtain the corresponding hydrazones (not isolated), which are cyclized as crude with a cyclodehydrating reagent (such as Burgess reagent) to afford **8.** Hydrazides **7** are either commercially available or can be prepared following known procedures to person skilled in the art. An alternative preparation method of compounds **8** consists in synthesizing not N-substituted hydrazones by reacting intermediates **6** and hydrazine. Following acylation afford the hydrazones which can be cyclized to compounds **8** without isolation. This cyclization is conducted in THF with 1,1,1,2,2,2-hexachloroethane and triphenylphosphine where nR₆ is N(Me)₂.

The syntheses of other compounds not currently described or included in this general description are well documented inside the experimental part of this invention which follows.

The free bases of compounds according to formula I, or indeed those of formulae 1a through 1f, their diastereomers or enantiomers can be converted to the corresponding pharmaceutically acceptable salts under standard conditions well known in the art. For example, the free base is dissolved in a suitable organic solvent, such as methanol, treated with, for example one equivalent of maleic or oxalic acid, one or two equivalents of hydrochloric acid or methanesulphonic acid, and then concentrated under vacuum to provide the corresponding pharmaceutically acceptable salt. The residue can then be purified by recrystallization from a suitable organic solvent or organic solvent mixture, such as methanol/diethyl ether.

The N-oxides of compounds according to formula I, or indeed those of formulae 1a through 1f, can be synthesized by simple oxidation procedures well known to those skilled in the art.

### Preparation of Compounds of the General Formula I

Unless otherwise stated, one or more tautomeric forms of compounds of the examples described hereinafter may be prepared in situ and/or isolated. All tautomeric forms of compounds of the examples described hereinafter should be considered to be disclosed.

The invention is illustrated by way of the following examples, in which the following abbreviations may be employed:
- AcOH: acetic acid
- MeCN: acetonitrile
- Aq.: aqueous
- BOC: tert-butyloxycarbonyl
- conc.: concentrated
- DCM: dichloromethane
- DCE: 1,2-dichloroethane
- DIPEA: N,N-diisopropylethylamine
- DMA: N,N-dimethylacetamide
- DMF: N,N-dimethylformamide
- DMSO: dimethyl sulfoxide
- EI: electron ionisation
- ESI: electrospray ionisation
- EtOAc: ethyl acetate
- EtOH: ethanol
- HCl: hydrochloric acid
- HCOOH: formic acid
- MeOH: methanol
- MS: mass spectrometry
- MW: molecular weight
- NaOH: sodium hydroxide
- NH₄OH: ammonium hydroxide (30% ammonia in water)
- NMP: N-methyl-2-pyrrolidone
- PE: petroleum ether
- R_{f}: retention value (from thin layer chromatography)
- RT or r.t.: room temperature
- Rₜ: retention time (from HPLC)
- THF: tetrahydrofuran
- TEA: triethylamine
- TFA: trifluoracetic acid
- UPLC: ultra high-performance liquid chromatography
- UPLC-MS: UPLC coupled with mass spectrometry

The following examples illustrate some of the compounds of general formula I as described above. These examples are illustrative only and are not intended to limit the scope of the invention. The reagents and starting materials are readily available to those skilled in the art.

### Example 1

### 6-(4-Chlorobenzyl)-8-(morpholin-4-yl)-3-(propan-2-yl)-2,6-dihydroimidazo[1,2-c]pyrido[2,3-e]pyrimidin-5(3H)-one

### 3-Fluoro-5-(morpholin-4-yl)pyridine-2-carbonitrile (Intermediate 1)

To a solution of 3,5-difluoropyridine-2-carbonitrile (10 g, 71.38 mmol) in DMAC (60 mL) in a round bottomed flask, was added morpholine (6.23 mL, 6.22 g, 71.38 mmol) followed by TEA (19.9 mL, 14.45 g, 142.77 mmol) under stirring at r.t.; then, the mixture was heated at 100°C for 2 hours. UPLC/MS showed a complete conversion of the starting material. After cooling to room temperature, water was added to the mixture and a white solid precipitated. It was filtered and the filtrate was washed with water and dried *in vacuo.* 3-Fluoro-5-(morpholin-4-yl)pyridine-2-carbonitrile (12.50 g, 84.5 % yield) was isolated as a white solid.

MS (ESI) m/z 208.09 [M + H]⁺

¹H NMR (400 MHz, DMSO-*d6*) δ ppm 8.28 - 8.35 (m, 1H) 7.40 (dd, 1H) 3.66 - 3.77 (m, 4H) 3.40 - 3.51 (m, 4H).

### Alternative procedure for the synthesis of Intermediate 1:

To a solution of 3,5-difluoropyridine-2-carbonitrile (1.04 g, 7.46 mmol) in DMAC (5 mL) in a microwave vial were added morpholine (0.65 g, 7.46 mmol, 0.65 mL) and TEA (1.51 g, 14.92 mmol, 2.08 mL). The mixture was stirred in a microwave oven Biotage Initiator Robot Eight^{®} at 100°C for 30 min. After cooling down to room temperature, water was added to the reaction mixture and a white solid precipitated. It was filtered, washed with water on the funnel and dried *in vacuo,* affording 1.3 g (84% yield) of the title compound as a white solid.

### 3-[(4-Chlorobenzyl)amino]-5-(morpholin-4-yl)pyridine-2-carbonitrile (Intermediate 2)

To a solution of 3-fluoro-5-(morpholin-4-yl)pyridine-2-carbonitrile (Intermediate 1, 12.5 g, 60.3 mmol) in NMP (150 mL) was added (4-chlorophenyl)methanamine (14.7 mL, 17.1 g, 121 mmol) followed by DIPEA (20.7 mL, 15.6 g, 121 mmol) and the mixture was stirred at 130°C for 3 hours. UPLC/MS showed the complete conversion of the starting material. After cooling down to room temperature, water was added to the mixture. The precipitated solid was filtered, washed with water and dried *in vacuo* to obtain 14.4 g of the title compound (72.6 % yield) as a pale brown solid. This compound was used in the following step without any further purification.

MS (ESI) m/z 328.87 [M + H]⁺

¹H NMR (400 MHz, DMSO-*d6*) δ: 7.70 (d, 1H), 7.32-7.45 (m, 4H), 6.83 (m, 1H), 6.30 (d, 1H), 4.43 (d, 2H), 3.59-3.71 (m, 4H), 3.11-3.25 (m, 4H)

### Alternative procedure for the synthesis of Intermediate 2:

A solution of 3-fluoro-5-(morpholin-4-yl)pyridine-2-carbonitrile (Intermediate 1, 2.30 g, 11.1 mmol), (4-chlorophenyl)methanamine (1.49 mL, 1.73 g, 12.2 mmol, 1.1 equiv.) and DIPEA (3.80 mL, 2.87 g, 22.2 mmol, 2 equiv.) in NMP (15 mL) was stirred at 150°C in a microwave oven (Biotage Initiator Robot Eight^{®} ) for 2 hours. Additional (4-chlorophenyl)methanamine (0.5 mL) was added and the mixture stirred for further 40 min at 150°C. After cooling, the reaction mixture was poured into water under vigorous stirring. The precipitate formed was filtered, washed with water, and dried to give the title compound (3.07 g, 84 % yield), used for the next step without further purification.

### N-[(4-Chlorophenyl)methyl]-5-(morpholin-4-yl)-2-[4-(propan-2-yl)-4,5-dihydro-1H-imidazol-2-yl]pyridin-3-amine (Intermediate 3)

To a solution of 3-[(4-chlorobenzyl)amino]-5-(morpholin-4-yl)pyridine-2-carbonitrile (Intermediate 2, 1 g, 3.041 mmol) in DMAC (8 mL) in a microwave vial, 3-methylbutane-1,2-diamine HCl (1.331 g, 7.603 mmol) was added followed by TEA (1.69 mL, 1.231 g, 12.17 mmol). The tube was sealed, and the reaction was heated at 160°C for 8 h. After overnight stirring at room temperature, UPLC/MS showed the completion of the reaction. Water and EtOAc were added to the reaction mixture, the organic phase was separated and washed with water and brine, dried over anhydrous Na₂SO₄, filtered and evaporated to dryness *in vacuo.* The crude was purified via automated flash chromatography (Biotage Isolera Four coupled with Dalton 2000^{®}), SNAP50 cartridge, eluting with isocratic CHCl₃ : MeOH 95:5. The title product (0.75 g, 59.6 % yield) was isolated as a yellow oil.

MS (ESI) m/z 414.23 [M + H]⁺

### 6-[(4-Chlorophenyl)methyl]-8-(morpholin-4-yl)-3-(propan-2-yl)-2,6-dihydroimidazo[1,2-c]pyrido[2,3-e]pyrimidin-5(3H)-one (Example 1)

To a solution of N-[(4-chlorophenyl)methyl]-5-(morpholin-4-yl)-2-[4-(propan-2-yl)-4,5-dihydro-1H-imidazol-2-yl]pyridin-3-amine (Intermediate 3, 500 mg, 1.208 mmol) in acetonitrile (8 mL) in a microwave vial was added 1,1'-carbonyldiimidazole (CDI, 235 mg, 1.45 mmol) followed by DMAP (29.5 mg, 0.242 mmol). The vial was sealed, and the mixture was heated under MW irradiation at 150°C for 90 min (Biotage Initiator Robot Eight^{®}). Following, 1.2 equivalents of CDI and 0.2 equivalents of DMAP, were added to the reaction and the mixture was heated under MW irradiation for further 90 min. UPLC/MS showed a complete conversion of the starting material. The reaction mixture was poured into water and extracted with EtOAc. The organic layer was dried over anhydrous Na₂SO₄, filtered and evaporated to dryness *in vacuo.* The crude residue was purified via automated flash chromatography (Biotage Isolera Four coupled with Dalton 2000^{®}) with a SNAP25 Ultra cartridge, eluting with isocratic CHCl₃ - MeOH 95:5. Two groups of fractions were collected, the second one enriched in the title compound and containing in addition the corresponding regioisomer 6-(4-chlorobenzyl)-8-(morpholin-4-yl)-2-(propan-2-yl)-2,6-dihydroimidazo[1,2-c]pyrido[2,3-e]pyrimidin-5(3H)-one as minor component and the first one vice-versa. The second group of fractions was re-purified on a SNAP10 Ultra cartridge eluting with a EtOAc: MeOH gradient from 5% to 10% of MeOH affording 20 mg of the title compound (3.8 % yield), isolated as a pale-yellow solid.

HPLC purity = 96 %

MS (ESI) m/z 440.28 [M + H]⁺

¹H NMR (400 MHz, DMSO-*d6*) δ ppm 8.17 (d, 1H) 7.38 - 7.45 (m, 2H) 7.30 - 7.35 (m, 2H) 6.76 (d, 1H) 5.17 - 5.33 (m, 2H) 4.35 - 4.46 (m, 1H) 3.87 - 3.96 (m, 1H) 3.77 (dd, 1H) 3.70 (t, 4H) 3.20 - 3.28 (m, 4H) 2.53 (br d, 1H) 0.89 (d, 3H) 0.74 (d, 3H).

### Example 2

### 6-(4-Chlorobenzyl)-2,2-dimethyl-8-(morpholin-4-yl)-2,6-dihydroimidazo[1,2-c]pyrido[2,3-e]pyrimidin-5(3H)-one

### N-[(4-Chlorophenyl)methyl]-2-(4,4-dimethyl-4,5-dihydro-1H-imidazol-2-yl)-5-(morpholin-4-yl)pyridin-3-amine (Intermediate 4)

To a solution of 3-[(4-chlorobenzyl)amino]-5-(morpholin-4-yl)pyridine-2-carbonitrile (Intermediate 2, 300 mg, 0.912 mmol) in DMAC (5 mL) in a microwave vial, 1,1-dimethyl-1,2-ethanediamine.2HCl (293.9 mg, 1.83 mmol) was added followed by TEA (0.51 mL, 369 mg, 3.65 mmol). The vial was sealed, and the reaction was heated at 160°C for 8 h under stirring. After overnight resting at room temperature, UPLC/MS showed the completion of the reaction. Water and EtOAc were added to the mixture. The organic phase was separated, washed with water and brine, dried over anhydrous Na₂SO₄, filtered and evaporated to dryness. The crude was purified by column chromatography using a Biotage Isolera^{®} instrument, SNAP25 cartridge, eluting using a gradient of EtOAc - PE from 15% to 70% of EtOAc. The title compound (120 mg, 33 % yield) was isolated as a yellow oil.

MS (ESI) m/z 400.19 [M + H]⁺

### 6-(4-Chlorobenzyl)-2,2-dimethyl-8-(morpholin-4-yl)-2,6-dihydroimidazo[1,2-c]pyrido[2,3-e]pyrimidin-5(3H)-one (Example 2)

To a solution of N-[(4-chlorophenyl)methyl]-2-(4,4-dimethyl-1,5-dihydroimidazol-2-yl)-5-morpholino-pyridin-3-amine (Intermediate 4, 120 mg, 0.300 mmol) in acetonitrile (4 mL) in a microwave vial, 1,1'-carbonildiimidazole (CDI, 58.4 mg, 0.36 mmol) was added followed by DMAP (7.33 mg, 0.06 mmol). The vial was sealed and mixture was heated under microwave irradiation at 150°C for 90 min (Biotage Initiator Robot Eight^{®}). After addition of further 1.2 equivalents of CDI and 0.2 equivalents of DMAP, the reaction mixture was heated in the microwave oven for 90 min. UPLC/MS showed the complete conversion of the starting material. The reaction mixture was poured into water, extracted with EtOAc, dried over anhydrous Na₂SO₄ and the solvent was evaporated to dryness. The crude was purified via automated flash chromatography (Biotage Isolera Four coupled with Dalton 2000^{®}), SNAP25 Ultra cartridge, eluting with an isocratic mixture of CHCl₃ : MeOH 95 : 5. The collected fractions gave 50 mg (39.1% yield) of the desired product as a pale-yellow solid.

HPLC Purity = 95.5 %

MS (ESI) m/z 426.21 [M + H]⁺

¹H NMR (400 MHz, DMSO-d6) δ ppm 8.15 (d, 1H) 7.31 - 7.45 (m, 4H) 6.72 (d, 1H) 5.23 (s, 2H) 3.68 - 3.75 (m, 4H) 3.67 (s, 2H) 3.18 - 3.27 (m, 4H) 1.30 (s, 6H).

### Example 3

### 2-tert-Butyl-6-(3,5-dimethoxybenzyl)-8-(morpholin-4-yl)-2,6-dihydroimidazo[1,2-c]pyrido[2,3-e]pyrimidin-5(3H)-one

### 3-[(3,5-Dimethoxybenzyl)amino]-5-(morpholin-4-yl)pyridine-2-carbonitrile (Intermediate 5)

To a solution of 3-fluoro-5-(morpholin-4-yl)pyridine-2-carbonitrile (Intermediate 1, 450 mg, 2.172 mmol) in NMP (5 mL) in a microwave vial, was added 3,5-dimethoxyphenylmethanamine (726.25 mg, 4.34 mmol) followed by DIPEA (0.74 mL, 561.37 mg, 4.34 mmol). The mixture was stirred at 150°C under microwave irradiation for 1 h (Biotage Initiator Robot Eight^{®}). UPLC/MS showed a complete conversion of the starting materials. After cooling at room temperature, water and EtOAc were added. The organic phase was separated, washed with water and brine, dried over anhydrous Na₂SO₄, filtered and evaporated to dryness *in vacuo.* The crude was purified by automated flash chromatography on Biotage Isolera^{®}, SNAP25 cartridge, eluting with a gradient of EtOAc - PE from 20% to 80% of EtOAc. The desired product 3-[(3,5-dimethoxybenzyl)amino]-5-(morpholin-4-yl)pyridine-2-carbonitrile (350 mg, 45.5 % yield) was isolated as a white solid.

MS (ESI) m/z 355.29 [M + H]⁺

¹H NMR (400 MHz, DMSO-*d6*) δ ppm 7.70 (d, 1H) 6.72 (t, 1H) 6.58 (d, 2H) 6.31 - 6.39 (m, 2H) 4.35 (d, 2H) 3.71 (s, 6H) 3.67 (dd, 4H) 3.15 - 3.25 (m, 4H).

### 2-(4-tert-Butyl-4,5-dihydro-1H-imidazol-2-yl)-N-(3,5-dimethoxybenzyl)-5-(morpholin-4-yl)pyridin-3-amine (Intermediate 6)

To a solution of 3-[(3,5-dimethoxybenzyl)amino]-5-(morpholin-4-yl)pyridine-2-carbonitrile (Intermediate 5, 300 mg, 0.847 mmol) in DMAC (5 mL) in a microwave vial, was added 3,3-dimethylbutane-1,2-diamine dihydrochloride (320.2 mg, 1.69 mmol) followed by TEA (0.47 mL, 342.6 mg, 3.39 mmol). The vial was sealed and the reaction was heated at 160°C (conventional heating) for 8h. After overnight resting, UPLC/MS showed the correct title product peak and the complete starting materials conversion. Water and EtOAc were added. The organic phase was washed with water and brine, dried over anhydrous Na₂SO₄, filtered and evaporated to dryness *in vacuo.* The crude residue was purified by automated flash chromatography on Biotage Isolera^{®}, SNAP25 cartridge using a mixture of EtOAc - PE in gradient from 15% to 70% of EtOAc. The title product (260 mg, 67.7% yield) was isolated as a yellow oil.

MS (ESI) m/z 454.51 [M + H]⁺

### 2-tert-Butyl-6-(3,5-dimethoxybenzyl)-8-(morpholin-4-yl)-2,6-dihydroimidazo[1,2-c]pyrido[2,3-e]pyrimidin-5(3H)-one (Example 3)

The title product was prepared using the same method described for the preparation of the product of Example 2 but replacing Intermediate 6 (2-(4-tert-butyl-4,5-dihydro-1H-imidazol-2-yl)-N-(3,5-dimethoxybenzyl)-5-(morpholin-4-yl)pyridin-3-amine) for Intermediate 4. The crude was purified via automated flash chromatography (Biotage Isolera Four coupled with - Dalton 2000^{®}), SNAP25 Ultra cartridge, eluting with an isocratic mixture of CHCl₃-MeOH 95:5. The collected fractions afforded 70 mg (33% yield) of the title product as a pale-yellow solid.

HPLC purity = 96.9 %

MS (ESI) m/z 480.27 [M + H]⁺

¹H NMR (400 MHz, DMSO-*d6*) δ ppm 8.16 (d, 1H) 6.80 (d, 1H) 6.49 (d, 2H) 6.39 (t, 1H) 5.06 - 5.25 (m, 2H) 3.85 - 4.01 (m, 2H) 3.66 - 3.74 (m, 11H) 3.22 - 3.28 (m, 4H) 0.93 (s, 9H).

### Example 4

### 2-tert-Butyl-6-(4-chlorobenzyl)-8-(morpholin-4-yl)-2,6-dihydroimidazo[1,2-c]pyrido[2,3-e]pyrimidin-5(3H)-one

### 2-(4-tert-Butyl-4,5-dihydro-1H-imidazol-2-yl)-N-(4-chlorobenzyl)-5-(morpholin-4-yl)pyridin-3-amine (Intermediate 7)

The title compound was prepared starting from 3-[(4-chlorobenzyl)amino]-5-(morpholin-4-yl)pyridine-2-carbonitrile (Intermediate 2) instead of 3-[(3,5-dimethoxybenzyl)amino]-5-(morpholin-4-yl)pyridine-2-carbonitrile, using the procedure described above for Intermediate 6. Purification yielded 160 mg (41%) of the title product 2-(4-tert-butyl-4,5-dihydro-1H-imidazol-2-yl)-N-[(4-chlorophenyl)methyl]-5-morpholino-pyridin-3-amine as a yellow oil.

MS (ESI) m/z 428.31 [M + H]⁺

### 2-tert-Butyl-6-(4-chlorobenzyl)-8-(morpholin-4-yl)-2,6-dihydroimidazo[1,2-c]pyrido[2,3-e]pyrimidin-5(3H)-one (Example 4)

The title product was prepared using the same method described for the preparation of the product of Example 2 but replacing the Intermediate 7 (2-(4-tert-butyl-4,5-dihydro-1H-imidazol-2-yl)-N-(4-chlorobenzyl)-5-(morpholin-4-yl)pyridin-3-amine) for Intermediate 4. The crude was purified via automated flash chromatography (Biotage Isolera Four coupled with Dalton^{®}), SNAP25 Ultra cartridge, eluting with isocratic CHCl₃ : MeOH 95 : 5. The desired product was isolated as a pale-yellow solid (50 mg, 31.4% yield).

MS (ESI) m/z 454.21 [M + H]⁺

¹H NMR (400 MHz, DMSO-*d6*) δ ppm 8.17 (d, 1H) 7.38 - 7.43 (m, 2H) 7.33 - 7.38 (m, 2H) 6.74 (d, 1H) 5.11 - 5.33 (m, 2H) 3.85 - 4.03 (m, 2H) 3.65 - 3.74 (m, 5H) 3.18 - 3.29 (m, 4H) 0.93 (s, 9H).

### Example 5

### 6-(4-Chlorobenzyl)-2-(2-methoxypropan-2-yl)-8-(morpholin-4-yl)-2,6-dihydroimidazo[1,2-c]pyrido[2,3-e]pyrimidin-5(3H)-one

### 3-{[(4-Chlorophenyl)methyl]amino}-5-(morpholin-4-yl)pyridine-2-carboxamide (Intermediate 8)

To an ice bath cooled solution of 3-[(4-chlorobenzyl)amino]-5-(morpholin-4-yl)pyridine-2-carbonitrile (Intermediate 2, 4 g, 14.60 mmol) in DMSO (60 mL), 1N sodium hydroxide solution (7.30 mL, 7.30 mmol) was added followed by hydrogen peroxide (30-35%) (1.49 mL, 1.655 g, 14.60 mmol) and the mixture was stirred at room temperature for 30 min. Afterwards, water was added and the precipitated solid was filtered off and dried *in vacuo.* 3.5 g (83% yield) of the title compound as a white powder were collected and used in the next step without any further purification.

MS (ESI) m/z 346.89 [M + H]⁺

### 1-[(4-Chlorophenyl)methyl]-7-(morpholin-4-yl)pyrido[3,2-d]pyrimidine-2,4(1H,3H)-dione (Intermediate 9)

To a solution of 3-{[(4-chlorophenyl)methyl]amino}-5-(morpholin-4-yl)pyridine-2-carboxamide (Intermediate 8, 2.77 g, 7.99 mmol) in dry THF (100 mL) under nitrogen atmosphere, 60 % sodium hydride, free-flowing powder, moistened with oil (319.5 mg, 7.99 mmol) was added and the reaction solution was stirred at room temperature for 15 minutes. The mixture was cooled to 0°C, ethyl chloroformate (3.82 mL, 4334 mg 39.94 mmol) was added dropwise, and the reaction mixture stirred at room temperature for 1 h and at reflux for 5 h. After overnight at r.t., the mixture was cooled to 0°C and sodium hydride (1 g; 5 equiv.) was added, stirring at reflux for 12 h. Because the conversion of the intermediate carbamate (M+H⁺= 419) was not complete, the mixture was cooled again to 0°C, further sodium hydride (1 g; 5 equiv.) was added. The mixture was stirred at reflux for 12 h, then cooled to 0°C; water (200 mL) was slowly added, and the pH was adjusted at around 4-5 with acetic acid and extracted with EtOAc. The organic phase was separated, washed with water, dried over anhydrous Na₂SO₄ and filtered. The solvent was evaporated under vacuum to give 2.47 g (83 % yield) of the title compound.

### Alternative procedure for the synthesis of Intermediate 9:

To a solution of 3-{[(4-chlorophenyl)methyl]amino}-5-(morpholin-4-yl)pyridine-2-carboxamide (Intermediate 8, 750 mg, 2.16 mmol) in N,N-dimethylformamide (15 mL) under nitrogen atmosphere stirred at 0-5°C was added 60 % sodium hydride oil dispersion (4 equiv., 8.65 mmol). The reaction mixture was stirred at room temperature for 30 minutes. At the same temperature 1,1'carbonyldiimidazole (1.052 g, 6.49 mmol) was added portionwise to the solution. Then the reaction mixture was stirred for 2 hours at r.t. Afterwards, 2 ml of acetic were added to the reaction stirred at 0-5°C until pH 5, followed by water (45 ml). 1.6 g of a yellow precipitate were filtered, collected and dried in a oven at 50°C for 4 hours to obtain 696 mg (86 % yield) of the title compound.

MS (ESI) m/z 373.07 [M + H]⁺

¹H NMR (400 MHz, DMSO-*d6*) δ ppm 11.52 (br d, 1H) 8.23 (d, 1H) 7.37 - 7.45 (m, 2H) 7.30 - 7.36 (m, 2H) 6.79 (d, 1H) 5.30 (s, 2H) 3.65 - 3.78 (m, 5H) 3.29 (br s, 3H).

### 1-(4-Chlorobenzyl)-7-(morpholin-4-yl)-4-sulfanylidene-3,4-dihydropyrido[3,2-d]pyrimidin-2(1H)-one (Intermediate 10)

To a mixture of 1-[(4-chlorophenyl)methyl]-7-morpholino-pyrido[3,2-d]pyrimidine-2,4-dione (Intermediate 9, 650 mg, 1.74 mmol) in 10 mL of anhydrous pyridine, Lawesson's reagent (2.821 g, 6.97 mmol) was added and the resulting mixture was stirred at 110°C for 2 hours. The solvent was evaporated. Water was added to the residue and the mixture extracted with EtOAc (3 times). The combined organic layers were washed with water and dried over anhydrous Na₂SO₄, filtered and concentrated *in vacuo.* The crude residue was used in the next step without any further purification. The title product 1-(4-chlorobenzyl)-7-(morpholin-4-yl)-4-sulfanylidene-3,4-dihydropyrido[3,2-d]pyrimidin-2(1H)-one (600 mg, 88.5 % yield) was isolated as a brown solid.

MS (ESI) m/z 389.19 [M + H]⁺

### 1-[(4-Chlorophenyl)methyl]-4-methylsulfanyl-7-morpholino-pyrido[3,2-d]pyrimidin-2-one (Intermediate 11)

To a solution of 1-(4-chlorobenzyl)-7-(morpholin-4-yl)-4-sulfanylidene-3,4-dihydropyrido[3,2-d]pyrimidin-2(1H)-one (Intermediate 10) in a mixture of 20 mL of 0.5 M sodium hydroxide solution and 6 mL of ethanol stirred at 0-5°C, were added 0.145 mL of iodomethane (331.2 mg, 2.33 mmol) and the mixture was stirred at room temperature for 2 hours. Then, a solution of 2N HCl was added. A precipitate was formed, which was filtered and dried *in vacuo.* 600 mg (95.7% yield) of the title compound as a yellow powder were collected and used in the next step without any further purification.

MS (ESI) m/z 403.12 [M + H]⁺

### 6-(4-Chlorobenzyl)-2-(2-methoxypropan-2-yl)-8-(morpholin-4-yl)-2,6-dihydroimidazo[1,2-c]pyrido[2,3-e]pyrimidin-5(3H)-one (Example 5)

To an ice bath cooled solution of crude of 2-amino-3-methoxy-3-methylbutan-1-ol hydrochloride (Intermediate 44, 202 mg, 1.191 mmol) in 0.5 mL of dry DMSO and 0.249 mL of TEA (0.181 g, 1.79 mmol) was added 1-[(4-chlorophenyl)methyl]-4-methylsulfanyl-7-morpholino-pyrido[3,2-d]pyrimidin-2-one (Intermediate 11, 80 mg, 0.1986 mmol). The mixture was heated at 150°C for 24 h. After cooling at room temperature, water and EtOAc were added, the two phases were separated. The aqueous layer was extracted with EtOAc, and the combined organic layers were washed with brine, dried over anhydrous Na₂SO₄, filtered and evaporated to dryness *in vacuo.* The crude dark brown oil was purified by automated flash chromatography on a Biotage Isolera^{®}, SNAP10 cartridge using a gradient of EtOAc - MeOH from 0% to 15% of MeOH. Evaporation of the collected fractions gave 8 mg (8.6 % yield) of the title compound as a yellow powder.

HPLC Purity = 92.2 %

MS (ESI) m/z 470.27 [M + H]⁺

¹H NMR (400 MHz, CHLOROFORM-d) δ ppm 8.19 (d, 1H) 7.32 - 7.39 (m, 2H) 7.23 (d, 2H) 6.47 (d, 1H) 5.00 - 5.41 (m, 2H) 4.41 (dd, 1H) 3.98 - 4.24 (m, 2H) 3.84 (t, 4H) 3.29 (s, 3H) 3.18 (q, 4H) 1.45 (s, 3H) 1.24 (s, 3H).

Alternative procedure for the synthesis of Example 5:

### 1-[(4-Chlorophenyl)methyl]-4-[(1-hydroxy-3-methoxy-3-methylbutan-2-yl)amino]-7-(morpholin-4-yl)pyrido[3,2-d]pyrimidin-2(1H)-one (Intermediate 12)

To an ice bath cooled solution of crude Intermediate 11 (0.17 g, 0.42 mmol) in 1 mL of dry DMSO was added 2-amino-3-methoxy-3-methylbutan-1-ol hydrochloride (Intermediate 44, 0.358 g, 2.11 mmol), followed by TEA (0.53 mL, 0.38 g, 3.80 mmol) and the mixture was heated at 150°C for 16 h. After cooling at room temperature, water and EtOAc were added; the two phases were separated and the aqueous layer was extracted with EtOAc. The combined organic phases were washed with brine, dried over anhydrous Na₂SO₄, filtered and evaporated to dryness *in vacuo.* 205 mg of a mixture as a dark brown oil containing the title compound were collected and used in the next step without further purification.

MS (ESI) m/z 488.27 [M + H]⁺

### 6-(4-Chlorobenzyl)-2-(2-methoxypropan-2-yl)-8-(morpholin-4-yl)-2,6-dihydroimidazo[1,2-c]pyrido[2,3-e]pyrimidin-5(3H)-one (Example 5)

To an ice bath cooled solution of the crude of 1-[(4-chlorophenyl)methyl]-4-[(1-hydroxy-3-methoxy-3-methylbutan-2-yl)amino]-7-(morpholin-4-yl)pyrido[3,2-d]pyrimidin-2(1H)-one (Intermediate 12, 205 mg) in 2 mL of DMF were added 1.17 mL TEA (0.850 g, 8.40 mmol) followed by 0.488 mL of methanesulfonyl chloride (0.722 g, 6.30 mmol). The mixture was stirred at room temperature for 1 h. Afterwards, an aq. sodium bicarbonate saturated solution and EtOAc were added; the two phases were separated, the aqueous layer was extracted with EtOAc (2x), the combined organic phases were dried over anhydrous Na₂SO₄, filtered and evaporated to dryness. The crude residue was purified by means of a Biotage Selekt^{®} instrument for flash chromatography, cartridge type SNAP25, using a gradient from EtOAc : MeOH 95:5 to EtOAc : MeOH 7:3. The collected fractions were re-purified by a reversed phase chromatography using a Biotage Selekt^{®} instrument, cartridge type SNAP30 KP-C18-HS, eluting with NH₄HCO₃ buffer : MeCN in gradient from 8:2 to 4:6. The collected fractions gave 73 mg (37 % yield) of the title compound as a yellow powder.

HPLC Purity = 91.5 %

MS (ESI) m/z 470.28 [M + H]⁺

¹H NMR (400 MHz, DMSO-d6) δ ppm 8.16 (d, 1H) 7.28 - 7.49 (m, 4H) 6.74 (d, 1H) 5.11 - 5.34 (m, 2H) 4.23 (dd, 1H) 3.85 - 3.96 (m, 1H) 3.78 - 3.85 (m, 1H) 3.70 (t, 4H) 3.24 (q, 4H) 3.17 (s, 3H) 1.26 (s, 3H) 1.07 (s, 3H).

### Example 6

### N-(5-Fluoropyridin-2-yl)-2-[8-(morpholin-4-yl)-5-oxo-2-(propan-2-yl)-2,3-dihydroimidazo[1,2-c]pyrido[2,3-e]pyrimidin-6(5H)-yl]acetamide

### 5-Bromo-2-(5-isopropyl-4,5-dihydro-1H-imidazol-2-yl)pyridin-3-amine (Intermediate 13)

A solution of 3-amino-5-bromopyridine-2-carbonitrile (500 mg, 2.53 mmol), 3-methylbutane-1,2-diamine dihydrochloride (884.25 mg, 5.05 mmol) and DIPEA (1.76 mL, 1.305 g, 10.100 mmol) in 4.5 mL of DMA was placed in a microwave tube. The tube was sealed, and the reaction mixture was heated at 160°C overnight. The reaction mixture was poured into water, extracted with EtOAc, dried over anhydrous Na₂SO₄, filtered and the solvent was evaporated to dryness. The crude product of the title compound was isolated as a pale-yellow oil (650 mg, 90.9 % yield) that was used in the next step without further purification.

MS (ESI) m/z 284.02 [M + H]⁺

### 8-Bromo-2-(propan-2-yl)-2,6-dihydroimidazo[1,2-c]pyrido[2,3-e]pyrimidin-5(3H)-one (Intermediate 14)

To a solution of 5-bromo-2-(5-isopropyl-4,5-dihydro-1H-imidazol-2-yl)pyridin-3-amine (Intermediate 13, 650 mg, 2.30 mmol) in 10 mL of acetonitrile was added 1,1'-carbonyldiimidazole (2.76 mmol, 446.7 mg) and the mixture stirred at 80°C for 6 hours. UPLC/MS showed a complete conversion. After overnight resting, the formed precipitate was filtered to give 650 mg (91.6 % yield) of the title product 8-bromo-2-(propan-2-yl)-2,6-dihydroimidazo[1,2-c]pyrido[2,3-e]pyrimidin-5(3H)-one as white solid, which was used in the next step without further purification.

MS (ESI) m/z 309.24 [M + H]⁺

### 2-Chloro-N-(5-fluoropyridin-2-yl)acetamide (Intermediate 15)

To a solution of of 5-fluoropyridin-2-amine (1 g, 8.92 mmol) in 30 mL of DCM stirred at 0-5°C, were added TEA (3.74 mL, 2.708 g, 26.76 mmol) and 2-chloroacetyl chloride (0.782 mL, 1.108 g, 9.813 mmol). The mixture was stirred at r.t. for 3 h. Then water was added, the two phases were separated. The organic layer was washed with brine, dried over anhydrous Na₂SO₄, filtered and evaporated *in vacuo.* The crude was purified by automated flash chromatography (Biotage Isolera^{®}, SNAP50 cartridge) eluting with a PE : EtOAc gradient from 10 % to 60 % of EtOAc. The title compound 2-chloro-N-(5-fluoro-2-pyridyl)acetamide was isolated as a white solid (0.85 g, 50.5 % yield).

MS (ESI) m/z 188.83 [M + H]⁺

### 2-(8-Bromo-5-oxo-2-(propan-2-yl)-2,3-dihydroimidazo[1,2-c]pyrido[2,3-e]pyrimidin-6(5H)-yl]-N-(5-fluoropyridin-2-yl)acetamide (Intermediate 16)

To a solution of 8-bromo-2-(propan-2-yl)-2,6-dihydroimidazo[1,2-c]pyrido[2,3-e]pyrimidin-5(3H)-one (Intermediate 14, 300 mg, 0.971 mmol) in 20 mL of anhydrous DMF, under N₂ atm., was added NaH 60% oil dispersion (42.69 mg, 1.067 mmol) and the mixture stirred at r.t. for 30 min. Afterwards, 2-chloro-N-(5-fluoro-2-pyridyl)acetamide (Intermediate 15, 219.6 mg, 1.164 mmol) was added and the mixture was stirred at r.t. overnight. UPLC/MS showed the reaction completion. The solid formed was filtered and washed with a small portion of DMF. The crude product was taken up with DCM and the suspension filtered affording the title product 2-[8-bromo-5-oxo-2-(propan-2-yl)-2,3-dihydroimidazo[1,2-c]pyrido[2,3-e]pyrimidin-6(5H)-yl]-N-(5-fluoropyridin-2-yl)acetamide (280 mg, yield: 62.55 %) as a pale yellow solid that was used for the next step without further purification.

MS (ESI) m/z 461.37 [M + H]⁺

### N-(5-Fluoropyridin-2-yl)-2-[8-(morpholin-4-yl)-5-oxo-2-(propan-2-yl)-2,3-dihydroimidazo[1,2-c]pyrido[2,3-e]pyrimidin-6(5H)-yl]acetamide (Example 6)

In a microwave vial a solution of 2-[8-bromo-5-oxo-2-(propan-2-yl)-2,3-dihydroimidazo[1,2-c]pyrido[2,3-e]pyrimidin-6(5H)-yl]-N-(5-fluoropyridin-2-yl)acetamide (Intermediate 16, 200 mg, 0.434 mmol) and morpholine (0.057 mL, 56.66 mg, 0.650 mmol) in 5 mL of 1,4-dioxane was purged with N₂. After 5 minutes, palladium (II) acetate (19.47 mg, 0.087 mmol,), XantPhos (100.3 mg, 0.173 mmol,) and caesium carbonate (282.5 mg, 0.867 mmol) were added and the mixture was stirred in a MW oven at 150°C for 2 hours, poured into water and extracted with EtOAc. The organic layer was washed with water, dried over anhydrous Na₂SO₄ and the solvent was evaporated to give a crude that was purified via automated flash chromatography (Biotage Isolera^{®}, SNAP10 cartridge) eluting with EtOAc : 1.4 N solution of NH₃ in MeOH in gradient from 5% to 20% of methanolic ammonia. The collected fractions gave 35 mg (17.27% yield) of the title compound, N-(5-Fluoropyridin-2-yl)-2-[8-(morpholin-4-yl)-5-oxo-2-(propan-2-yl)-2,3-dihydroimidazo[1,2-c]pyrido[2,3-e]pyrimidin-6(5H)-yl]acetamide, as a pale yellow solid.

MS (ESI) m/z 468.24 [M + H]⁺

### Example 7

### 8-(4-Aminopiperidin-1-yl)-6-[(4-chlorophenyl)methyl]-2-(propan-2-yl)-2,6-dihydroimidazo[1,2-c]pyrido[2,3-e]pyrimidin-5(3H)-one

### 8-Bromo-6-[(4-chlorophenyl)methyl]-2-(propan-2-yl)-2,6-dihydroimidazo[1,2-c]pyrido[2,3-e]pyrimidin-5(3H)-one (Intermediate 17)

To a solution of 8-bromo-2-isopropyl-2,6-dihydroimidazo[1,2-c]pyrido[2,3-e]pyrimidin-5(3H)-one (Intermediate 14, 325 mg, 1.05 mmol) in 10 mL of DMF, sodium hydride 60% oil dispersion (46.25 mg, 1.16 mmol) were added followed by 1-(bromomethyl)-4-chlorobenzene (237.6 mg, 1.16 mmol). The reaction mixture was then stirred at room temperature for 4h, diluted with EtOAc and washed with water. The organic layer was separated and dried over anhydrous Na₂SO₄, filtered and concentrated under reduced pressure. The crude was purified by flash chromatography (Biotage Isolera^{®}, Silicycle SiliaSep 12 g cartridge), eluting with PE : EtOAc in gradient from 0 to 100 % of EtOAc to afford 260 mg (57% yield) of 8-bromo-6-(4-chlorobenzyl)-2-isopropyl-2,6-dihydroimidazo[1,2-c]pyrido[2,3-e]pyrimidin-5(3H)-one.

MS (ESI) m/z 435 [M + H]⁺

### tert-Butyl (1-(6(4-chlorobenzyl)-2-isopropyl-5-oxo-2,3,5,6-tetrahydroimidazo[1,2-c]pyrido[2,3-e]pyrimidin-8-yl)piperidin4-yl)carbamate (Intermediate18)

In a vial equipped with a magnetic stirring bar 5.17 mg of 4,4'-Di-t-butyl-2,2'-bipyridine)bis[3,5-difluoro-2-[5-trifluoromethyl-2-pyridinylkN)phenyl-kC]iridium(III) hexafluorophosphate, tert-butyl N-(4-piperidyl)carbamate (115.4 mg, 0.58 mmol), DABCO (46.55 mg, 0.420 mmol) and 8-bromo-6-(4-chlorobenzyl)-2-isopropyl-2,6-dihydroimidazo[1,2-c]pyrido[2,3-e]pyrimidin-5(3H)-one (Intermediate 17, 100 mg, 0.230 mmol) were dissolved in 1 mL DMA. Then Nickel(2+) chloride - 1,2-dimethoxyethane (1:2:1) (10.13 mg, 0.05 mmol ) was added as a solution in 0.30 mL of DMA. The vial was then stirred under N₂, cooled at -78°C and degassed, backfilled with N₂ and heated up slowly to r.t. The cycle was repeated 3 times, then the reaction mixture was irradiated (blue light), while stirring for 3h. UPLC-MS showed the formation of desired product. The reaction mixture was diluted with water (10 mL); the mixture was then extracted with EtOAc (2x20 mL). The combined organic layers were washed with brine (15 mL), dried over anhydrous Na₂SO₄, filtered and evaporated under reduced pressure. The obtained crude was purified by flash chromatography, Biotage Isolera^{®} instrument, flow rate: 36 mL/min, eluting with CHCl₃: MeOH in gradient from 0 to 10% of MeOH. The collected fractions gave 70 mg (54.89 % yield) of the title compound.

### 8-(4-Aminopiperidin-1-yl)-6-[(4-chlorophenyl)methyl]-2-(propan-2-yl)-2,6-dihydroimidazo[1,2-c]pyrido(2,3-e]pyrimidin-5(3H)-one (Example 7)

To a solution of tert-butyl (1-(6-(4-chlorobenzyl)-2-isopropyl-5-oxo-2,3,5,6-tetrahydroimidazo[1,2-c]pyrido[2,3-e]pyrimidin-8-yl)piperidin-4-yl)carbamate (Intermediate 18, 70 mg, 0.13 mmol) in 5 mL of DCM was added dropwise TFA (0.50 mL, 0.007 mol).

Then, the reaction mixture was stirred for 2 hours. UPLC-MS showed the formation of the desired title product. The reaction mixture was then diluted with 20 mL of toluene and evaporated to dryness. The residue was then purified by preparative HPLC (Fraction Lynx) to get 24 mg (41.4 % yield) of 8-(4-aminopiperidin-1-yl)-6-[(4-chlorophenyl)methyl]-2-(propan-2-yl)-2,6-dihydroimidazo[1,2-c]pyrido[2,3-e]pyrimidin-5(3H)-one.

HPLC purity = 99 %

MS (ESI) m/z 453.31 [M + H]⁺

¹H NMR (400 MHz, ACETONITRILE-d3) δ ppm 9.00 (br s, 1H), 8.36 (s, 1H), 7.53 - 7.21 (m, 4H), 6.64 (s, 1H), 6.38 (br s, 2H), 5.34 (q, J = 16.6 Hz, 2H), 4.58 - 4.28 (m, 2H), 4.23 - 3.93 (m, 3H), 3.52 (br s, 1H), 3.12 (br t, J = 12.8 Hz, 2H), 2.20 - 2.04 (m, 2H), 1.60 (br d, J = 12.1 Hz, 2H), 1.45 - 1.25 (m, 1H), 1.30 (br s, 1H), 1.12 - 0.97 (m, 6H).

### Example 8

### 6-[(4-Chlorophenyl)methyl]-8-(piperazin-1-yl)-2-(propan-2-yl)-2,6-dihydroimidazo[1,2-c]pyrido[2,3-e]pyrimidin-5(3H)-one

### tert-Butyl 4-(6-(4-chlorobenzyl)-2-isopropyl-5-oxo-2,3,5,6-tetrahydroimidazo[1,2-c]pyrido[2,3-e]pyrimidin-8-yl)piperazine-1-carboxylate (Intermediate 19)

The title compound was prepared following the procedure described for the compound of Intermediate 18 replacing tert-butyl N-(4-piperidyl)carbamate with tert-butyl piperazine-1-carboxylate. The purification carried out as described above afforded 29 mg (25% yield) of tert-butyl 4-(6-(4-chlorobenzyl)-2-isopropyl-5-oxo-2,3,5,6-tetrahydroimidazo[1,2-c]pyrido[2,3-e]pyrimidin-8-yl)piperazine-1-carboxylate.

### 6-[(4-Chlorophenyl)methyl]-8-(piperazin-1-yl)-2-(propan-2-yl)-2,6-dihydroimidazo[1,2-c]pyrido[2,3-e]pyrimidin-5(3H)-one (Example 8)

The title compound was prepared following the procedure described for the compound of Example 7 starting from Intermediate 19 instead of Intermediate 18. The same purification methodology afforded 7.6 mg (32% yield) of the title compound as a solid.

HPLC Purity > 99%

MS (ESI) m/z 439.40 [M + H]⁺

¹H NMR (400 MHz, ACETONITRILE-d3) δ =9.10 (br s, 1H) 8.32 (s, 1H), 7.41 - 7.31 (m, 4H), 6.66 (s, 1H), 5.38 - 5.23 (m, 2H), 4.46 - 4.34 (m, 2H), 4.19 - 3.94 (m, 1H), 3.77 (m, 4H), 3.29 (m, 4H), 2.10 (m, 1H), 1.03 (br dd, J = 7.1, 10.6 Hz, 6H).

### Example 9

### 6-[(4-Chlorophenyl)methyl]-8-(morpholin-4-yl)-2-(propan-2-yl)-2,6-dihydroimidazo[1,2-c]pyrido[2,3-e]pyrimidin-5(3H)-one

The first group of fractions collected from the column chromatography described in Example 1 was re-purified on a SNAP10 Ultra cartridge eluting with a EtOAc-MeOH gradient from 5% to 10% of MeOH, affording 270 mg of the title compound (50.8 % yield), as a pale-yellow solid.

HPLC purity = 97.7 %

MS (ESI) m/z 440.24 [M + H]⁺

¹H NMR (400 MHz, DMSO-*d6*) δ ppm 8.16 (d, 1H) 7.32 - 7.45 (m, 4H) 6.73 (d, 1H) 5.12 - 5.33 (m, 2H) 3.91 - 4.05 (m, 2H) 3.71 (t, 4H) 3.52 - 3.65 (m, 1H) 3.24 (dd, 4H) 1.65 - 1.86 (m, 1H) 0.99 (d, 3H) 0.92 (d, 3H).

### Example 10

### (2S)-6-(4-Chlorobenzyl)-8-(morpholin-4-yl)-2-(propan-2-yl)-2,6-dihydroimidazo[1,2-c]pyrido[2,3-e]pyrimidin-5(3H)-one

The title compound was obtained from the racemic mixture of compound of Example 9, by chiral HPLC separation using a column Astec Chirobiotic V2, 150×4.6 mm 5 µm, eluting in isocratic mode with MeOH, at 2 mL/min flow for 20 min, at the temperature of 20°C. The compound corresponds to the first eluted of the HPLC chiral separation.

Alternatively, the following procedure was carried out to obtain the Example 10:

### 1-[(4-Chlorophenyl)methyl]-4-{[(2S)-1-hydroxy-3-methylbutan-2-yl]amino}-7-(morpholin-4-yl)pyrido[3,2-d]pyrimidin-2(1H)-one (Intermediate 20)

To an ice bath cooled solution of Intermediate 11 (80 mg, 0.1986 mmol) in 0.5 mL of dry DMSO, was added (S)-(+)-valinole (102 mg, 0.993 mmol) and the mixture was heated at 150°C for 5 h. After cooling to room temperature, water and EtOAc were added, the two phases were separated and the aqueous layer was extracted with EtOAc. The combined organic phases were washed with brine, dried over anhydrous Na₂SO₄, filtered and evaporated to dryness. 190 mg of the title compound as dark oil were collected as a crude and used in the next step without any further purification.

MS (ESI) m/z 458.27 [M + H]⁺

### (2S)-6-(4-Chlorobenzyl)-8-(morpholin-4-yl)-2-(propan-2-yl)-2,6-dihydroimidazo(1,2-c]pyrido[2,3-e]pyrimidin-5(3H)-one (Example 10)

To an ice bath cooled solution of 1-[(4-chlorophenyl)methyl]-4-{[(2S)-1-hydroxy-3-methylbutan-2-yl]amino}-7-(morpholin-4-yl)pyrido[3,2-d]pyrimidin-2(1H)-one (Intermediate 20, 90 mg, 0.1965 mmol) in dry DCM (2 mL), thionyl chloride (0.285 mL, 468 mg, 3.93 mmol,) was added and the mixture was stirred at room temperature overnight. Afterwards, dichloromethane (DCM) and excess thionyl chloride were removed under vacuum, sodium bicarbonate saturated solution and DCM were added, the two layers were separated; the aqueous layer was extracted with DCM (3x) and the combined organic phases were washed with brine, dried over Na₂SO₄, filtered, and evaporated to dryness *in vacuo.* The crude residue was purified by means of a Biotage Selekt^{®} instrument, cartridge type SNAP10, using a gradient of EtOAc : MeOH from 100 to 50:50. The collected fractions gave 22 mg (25 % yield) of the title compound as a brown powder.

MS (ESI) m/z 440.44 [M + H]⁺

¹H NMR (DMSO-d6) δ ppm 8.17 (d, 1H), 7.29-7.47 (m, 4H), 6.73 (d, 1H), 5.23 (m, 2H), 3.93-4.01 (m, 2H), 3.66-3.76 (m, 4H), 3.56-3.66 (m, 1H), 3.20-3.27 (m, 4H), 1.72-1.86 (m, 1H), 1.00 (d, 3H), 0.92 (d, 3H)

### Example 11

### (2R)-6-(4-Chlorobenzyl)-8-(morpholin-4-yl)-2-(propan-2-yl)-2,6-dihydroimidazo[1,2-c]pyrido[2,3-e]pyrimidin-5(3H)-one

The title compound was obtained from the racemic mixture of the compound of Example 9, by chiral HPLC separation using a column Astec Chirobiotic V2, 150×4.6 mm 5 µm, eluting in isocratic mode with MeOH, at 2 mL/min flow for 20 min, at the temperature of 20°C. The compound corresponds to the second eluted of the HPLC chiral separation.

Alternatively, the following procedure was carried out to obtain the Example 11:

### 1-(4-Chlorobenzyl)-4-{[(2R)-1-hydroxy-3-methylbutan-2-yl]amino}-7-(morpholin-4-yl)pyrido[3,2-d]pyrimidin-2(1H)-one (Intermediate 21)

The title compound was prepared as described above for the Intermediate 20, but using (R)-(-)-valinol instead of the (S)-enantiomer. The title compound was isolated from column chromatography as a thick dark oil (300 mg, 68.5 % yield).

MS (ESI) m/z 458.49 [M + H]⁺

¹H NMR (400 MHz, DMSO-*d6*) δ ppm 8.20 (d, 1H) 7.58 (d, 1H) 7.35 - 7.42 (m, 2H) 7.26 - 7.34 (m, 2H) 6.82 (d, 1H) 5.15 - 5.44 (m, 2H) 4.79 (t, 1H) 3.97 - 4.14 (m, 1H) 3.69 - 3.83 (m, 4H) 3.48 - 3.69 (m, 2H) 3.30 - 3.33 (m, 4H) 2.05 (spt, 1H) 0.88 - 1.02 (m, 6H).

### (2R)-6-(4-Chlorobenzyl)-8-(morpholin-4-yl)-2-(propan-2-yl)-2,6-dihydroimidazo[1,2-c]pyrido[2,3-e]pyrimidin-5(3H)-one (Example 11)

To an ice bath cooled solution of 1-(4-chlorobenzyl)-4-{[(2R)-1-hydroxy-3-methylbutan-2-yl]amino}-7-(morpholin-4-yl)pyrido[3,2-d]pyrimidin-2(1H)-one (Intermediate 21, 100 mg, 0.2183 mmol) in 2 mL of dry DMF, TEA (0.61 mL, 0.442 g, 4.37 mmol) was added followed by methanesulfonyl chloride (0.253 mL, 0.375 g, 3.28 mmol). The mixture was stirred at room temperature for 24 h. Then water and DCM were added, the two phases were separated, the aqueous layer was extracted with multiple portions of DCM. The combined organic phases were washed with brine, dried over anhydrous Na₂SO₄, filtered and evaporated *in vacuo.* The crude residue was purified by means of a Biotage Selekt^{®} instrument, cartridge type SNAP10, using a gradient of DCM : 1.4 N solution of NH₃ in MeOH from 99:1 to 70:30. The isolated fractions were further purified by reversed phase column chromatography by means of a Biotage Selekt^{®} instrument, cartridge type SNAP12 KP-C18-HS, using a gradient from NH₄HCO₃ buffer - MeCN 8:2 to NH₄HCO₃ buffer - MeCN 4:6. The collected fractions gave 22 mg (22.9% yield) of the title compound as a yellow powder.

HPLC Purity = 89.92 %

MS (ESI) m/z 440.34 [M + H]⁺

¹H NMR (400 MHz, DMSO-*d6*) δ ppm 8.17 (d, 1H), 7.29-7.47 (m, 4H), 6.73 (d, 1H), 5.23 (m, 2H), 3.93-4.01 (m, 2H), 3.66-3.76 (m, 4H), 3.56-3.66 (m, 1H), 3.20-3.27 (m, 4H), 1.72-1.86 (m, 1H), 1.00 (d, 3H), 0.92 (d, 3H)

### Example 12

### 6-(4-Chlorobenzyl)-2-(1-hydroxy-2-methylpropan-2-yl)-8-(morpholin-4-yl)-2,6-dihydroimidazo[1,2-c]pyrido[2,3-e]pyrimidin-5(3H)-one

### 1-[(4-Chlorophenyl)methyl]-4-[(1,4-dihydroxy-3,3-dimethylbutan-2-yl)amino]-7-(morpholin-4-yl)pyrido[3,2-d]pyrimidin-2(1H)-one (Intermediate 22)

To an ice bath cooled solution of Intermediate 11 (600 mg, 1.489 mmol) in dry DMSO (1 mL) crude 3-amino-2,2-dimethylbutane-1,4-diol hydrochloride (Intermediate 33, 1.77 g, 10.42 mmol) was added, followed by TEA (2.49 mL, 1.808 g, 17.87 mmol) and the mixture was heated at 130°C for 4 hours, then stirred at room temperature overnight. Additional crude Intermediate 33 (1.2 g, 7.1 mmol) was added, followed by 1.45 ml of TEA and the mixture was heated at 130°C for further 8 hours. This operational cycle was repeated two times. Then the reaction was stirred 140°C for 3 days. After cooling at room temperature, water and EtOAc were added. The two phases were separated, and the aqueous layer was extracted with multiple portions of EtOAc. The combined organic layers were washed with brine, dried over anhydrous Na₂SO₄, filtered and evaporated to dryness *in vacuo.* The crude residue was purified by means of a Biotage Selekt^{®} instrument, cartridge type SNAP50, using a gradient from EtOAc : MeOH from 99: 1 to 80:20. The fractions collected gave 1.3 g of crude Intermediate 22. The mixture was further purified by means of a Biotage Selekt^{®} instrument, cartridge type SNAP50, using a gradient with EtOAc : MeOH from 100:0 to 80:20, affording 450 mg (61.9 % yield ) of the title compound as a dark yellow oil.

MS (ESI) m/z 488.31 [M + H]⁺

### 6-(4-Chlorobenzyl)-2-(1-hydroxy-2-methylpropan-2-yl)-8-(morpholin-4-yl)-2,6-dihydroimidazo[1,2-c]pyrido[2,3-e]pyrimidin-5(3H)-one (Example 12)

To an icebath cooled solution of 1-[(4-chlorophenyl)methyl]-4-[[3-hydroxy-1-(hydroxymethyl)-2,2-dimethyl-propyl]amino]-7-morpholino-pyrido[3,2-d]pyrimidin-2-one (Intermediate 22, 450 mg, 0.9222 mmol) in 3 mL of DMF, was added TEA (0.257 mL, 0.187 g, 1.844 mmol), followed by methanesulfonyl chloride (71 µL, 106 mg, 0.922 mmol) and the mixture was stirred at room temperature overnight. Another equivalent of methanesulfonyl chloride and TEA were added and the mixture stirred again at room temperature for overnight. This operational cycle was performed 2 times. The title compound mass spectra peak was detected ([M + H]⁺= 470) along with the mass spectra of the mesylate derivative of title compound ([M + H]⁺ = 548). 1 mL of 2N NaOH was added and the mixture was stirred at room temperature for 4 h, then gradually heated to 80°C for 6 h. After cooling at room temperature, water and EtOAc were added; the two phases were separated, the organic layer was washed with citric acid saturated aq. solution and water, dried over anhydrous Na₂SO₄ affording 210 mg of crude residue. The latter was purified by means of a Biotage Selekt^{®} instrument, cartridge type SNAP25, eluting with EtOAc: MeOH in gradient from 95:5 to 80:20 affording 24 mg (5.5% yield) of the title compound as a light-yellow gummy powder.

HPLC purity = 80.97 %

MS (ESI) m/z 470.40 [M + H]⁺

¹H NMR (400 MHz, DMSO-*d6*) δ ppm 8.17 (d, 1H) 7.25 - 7.51 (m, 4H) 6.74 (d, 1H) 5.11 - 5.34 (m, 2H) 4.65 (br s, 1H) 4.12 - 4.23 (m, 1H) 3.91 (t, 1 H) 3.78 (dd, 1H) 3.71 (t, 5H) 3.33 - 3.36 (m, 2H) 3.17 - 3.28 (m, 5H) 0.75 - 0.92 (m, 7H).

### Example 13

### 6-[(5-Chloropyridin-2-yl)methyl]-8-(morpholin-4-yl)-2-(propan-2-yl)-2,6-dihydroimidazo[1,2-c]pyrido[2,3-e]pyrimidin-5(3H)-one

### 3-[(5-Chloro-2-pyridyl)methylamino]-5-morpholino-pyridine-2-carbonitrile (Intermediate 23)

The title product was prepared following the alternative procedure reported for the compound of Intermediate 2, replacing (4-chlorophenyl)methanamine with (5-chloro-2-pyridyl)methanamine. In this case the crude was purified via automated flash chromatography using Biotage Isolera Four coupled with Dalton 2000^{®} instrument, SNAP 10 Cartridge, eluting with an EtOAc - PE in gradient from 1:1 to 9:1 to give the title compound (18 % yield).

MS (ESI) m/z 330.12 [M + H]⁺

### N-[(5-Chloropyridin-2-yl)methyl]-5-(morpholin-4-yl)-2-[4-(propan-2-yl)-4,5-dihydro-1H-imidazol-2-yl]pyridin-3-amine (Intermediate 24)

The title product was prepared starting from Intermediate 23, following the procedure reported for Intermediate 3. After the usual work-up procedure the crude was purified via automated flash chromatography (Biotage Isolera Four coupled with Dalton 2000^{®}), SNAP25 cartridge, eluting with EtOAc - PE in gradient from 6:4 to 10:0 to give the wished compound (48% yield).

MS (ESI) m/z 415.21 [M + H]⁺

### 6-[(5-Chloropyridin-2-yl)methyl]-8-(morpholin-4-yl)-2-(propan-2-yl)-2,6-dihydroimidazo[1,2-c]pyrido[2,3-e]pyrimidin-5(3H)-one (Example 13)

The title compound was synthesized as reported for the compound of Example 1 but starting from Intermediate 24. After the usual work-up procedure the crude was purified via automated flash chromatography (Biotage Isolera Four coupled with Dalton 2000^{®}), SNAP30 KP-C18-HS cartridge, eluting with MeCN - H₂O in gradient from 1:9 to 8:2 to give the title product (42% yield).

HPLC purity = 95.3 %

MS (ESI) m/z 441.41 [M + H]⁺

¹H NMR (400 MHz, DMSO-J6) δ ppm 8.51 - 8.58 (m, 1H) 8.16 (d, 1H) 7.90 (dd, 1H) 7.44 (d, 1H) 6.82 (d, 1H) 5.21 - 5.40 (m, 2H) 3.86 - 4.07 (m, 2H) 3.70 (t, 4H) 3.52 - 3.61 (m, 1H) 3.19 - 3.27 (m, 4H) 1.71 - 1.83 (m, 1H) 0.86 - 1.07 (m, 6H).

### Example 14

### 6-[(5-Methoxypyridin-2-yl)methyl]-8-(morpholin-4-yl)-2-(propan-2-yl)-2,6-dihydroimidazo[1,2-c]pyrido[2,3-e]pyrimidin-5(3H)-one

### 3-{[(5-Methoxypyridin-2-yl)methyl]amino}-5-(morpholin-4-yl)pyridine-2-carbonitrile (Intermediate 25)

The title product was prepared following the alternative procedure reported for Intermediate 2, replacing (4-chlorophenyl)methanamine with (5-methoxy-2-pyridyl)methanamine. In this case, after cooling at room temperature water was added to the mixture and a white solid precipitated and was filtered. This solid was washed with water and dried. 3-[(5-methoxy-2-pyridyl)methylamino]-5-morpholino-pyridine-2-carbonitrile (95.5% yield) was used for the next step without further purification.

MS (ESI) m/z 326.29 [M + H]⁺

### N-[(5-Methoxypyridin-2-yl)methyl]-5-(morpholin-4-yl)-2-[4-(propan-2-yl)-4,5-dihydro-1H-imidazol-2-yl]pyridin-3-amine (Intermediate 26)

The title product was prepared starting from Intermediate 25 following the procedure reported in Example 1 for Intermediate 3. After the usual work-up procedure, the crude product was purified by Isolera Biotage^{®} (SNAP25 cartridge) eluting with isocratic mixture of EtOAc - MeOH 95:5. The desired product 2-(4-isopropyl-4,5-dihydro-1H-imidazol-2-yl)-N-[(5-methoxy-2-pyridyl)methyl]-5-morpholinopyridin-3-amine (31.71 % yield) was isolated as a yellow oil.

MS (ESI) m/z 411.37 [M + H]⁺

### 6-[(5-Methoxypyridin-2-yl)methyl]-8-(morpholin-4-yl)-2-(propan-2-yl)-2,6-dihydroimidazo(1,2-c]pyrido(2,3-e]pyrimidin-5(3H)-one (Example 14)

The title compound was synthesized as reported for the compound of Example 1 but starting from Intermediate 26. After the usual work-up procedure the crude was purified via automated flash chromatography (Biotage Isolera Four coupled with Dalton 2000^{®}), SNAP25 Ultra cartridge eluting with isocratic CHCl₃ - MeOH 95:5. The desired product (31.4 % yield) was isolated as pale-yellow solid.

HPLC purity = 98.3 %

MS (ESI) m/z 437.34 [M + H]⁺

¹H NMR (400 MHz, DMSO-*d6*) δ ppm 8.22 (dd, 1H) 8.15 (d, 1H) 7.35 - 7.41 (m, 1H) 7.28 - 7.35 (m, 1H) 6.94 (d, 1H) 5.09 - 5.35 (m, 2H) 3.88 - 4.11 (m, 2H) 3.80 (s, 3H) 3.72 (t, 4H) 3.54 - 3.63 (m, 1H) 3.24 (br dd, 5H) 1.78 (dq, 1H) 0.85 - 1.04 (m, 7H).

### Example 15

### 6-[(4-Chlorophenyl)methyl]-8-[(1,3-dihydroxypropan-2-yl)amino]-2-(propan-2-yl)-2,6-dihydroimidazo[1,2-c]pyrido[2,3-e]pyrimidin-5(3H)-one

### 5-[(2,2-Dimethyl-1,3-dioxan-5-yl)amino]-3-fluoropyridine-2-carbonitrile (Intermediate 27)

The title compound was synthesized as reported for Intermediate 1 using the described alternative procedure and 2,2-dimethyl-1,3-dioxan-5-amine instead of morpholine. After the usual work-up procedure, the crude residue was purified with automated flash chromatography by means of a Biotage Selekt^{®} instrument, cartridge type SNAP50, eluting with PE : EtOAc in gradient from 9:1 to 4:6. Yield: 40 %. White solid.

MS (ESI) m/z 252.07 [M + H]⁺

### 3-{[(4-Chlorophenyl)methyl]amino}-5-[(2,2-dimethyl-1,3-dioxan-5-yl)amino]pyridine-2-carbonitrile (Intermediate 28)

The title compound was synthesized as reported for Intermediate 2 using the described alternative procedure and starting from Intermediate 27 instead of Intermediate 1. The crude residue obtained from the usual work-up was purified by means of a Biotage Isolera One^{®} instrument, cartridge type SNAP50, eluting with PE: EtOAc in gradient from 75:25 to 30:70. The title compound was obtained as off-white powder (59% yield).

MS (ESI) m/z 373.12 [M + H]⁺

### N3-[(4-Chlorophenyl)methyl]-N5-(2,2-dimethyl-1,3-dioxan-5-yl)-2-[4-(propan-2-yl)-4,5-dihydro-1H-imidazol-2-yl]pyridine-3,5-diamine (Intermediate 29)

Prepared following the procedure reported for Intermediate 3 but starting from 3-{[(4-chlorophenyl)methyl]amino}-5-[(2,2-dimethyl-1,3-dioxan-5-yl)amino]pyridine-2-carbonitrile (Intermediate 28). The crude residue was purified by means of a Biotage Selekt^{®} instrument, cartridge type SNAP50, using a mixture of EtOAc - MeOH in gradient from 10:0 to 8:2. The title compound was isolated as a yellow oil (58 %).

MS (ESI) m/z 458.27 [M + H]⁺

### 6-[(4-Chlorophenyl)methyl]-8-[(2,2-dimethyl-1,3-dioxan-5-yl)amino]-2-(propan-2-yl)-2,6-dihydroimidazo[1,2-c]pyrido(2,3-e]pyrimidin-5(3H)-one (Intermediate 30)

Prepared following the procedure reported for Intermediate 4, but using N3-[(4-chlorophenyl)methyl]-N5-(2,2-dimethyl-1,3-dioxan-5-yl)-2-[4-(propan-2-yl)-4,5-dihydro-1H-imidazol-2-yl]pyridine-3,5-diamine (Intermediate 29)instead of Intermediate 3. The crude residue was purified by means of a Biotage Selekt^{®} instrument, cartridge type SNAP25, using a gradient of DCM : 1.4 N methanolic ammonia from 98:2 to 85:15. The tile compound was isolated as a brown oil (28% yield).

MS (ESI) m/z 484.29 [M + H]⁺

### 6-[(4-Chlorophenyl)methyl]-8-[(1,3-dihydroxypropan-2-yl)amino]-2-(propan-2-yl)-2,6-dihydroimidazo[1,2-c]pyrido[2,3-e]pyrimidin-5(3H)-one (Example 15)

To a solution of 6-[(4-chlorophenyl)methyl]-8-[(2,2-dimethyl-1,3-dioxan-5-yl)amino]-2-(propan-2-yl)-2,6-dihydroimidazo[1,2-c]pyrido[2,3-e]pyrimidin-5(3H)-one (Intermediate 30, 32 mg, 0.066 mmol) in 1 mL THF, 0.5 mL of 2N aqueous HCl solution were added and the mixture was stirred at room temperature overnight. Then, the mixture was alkalinized with potassium carbonate, the aqueous layer was extracted with EtOAc (2x), the combined organic phases were dried over anhydrous Na₂SO₄, filtered and evaporated. The crude residue was purified by means of a Biotage Selekt^{®} instrument, cartridge type SNAP10, using a gradient from DCM : 1.4 N methanolic ammonia from 98:2 to 8:2. Collected fractions gave 2 mg (of the title compound as a white powder.

HPLC purity = 95.9 %

MS (ESI) m/z 444.31 [M + H]⁺

¹H NMR (400 MHz, DMSO-*d6*) δ ppm 7.92 (br s, 1H) 7.19 - 7.28 (m, 4H) 6.42 (br s, 1H) 6.14 - 6.38 (m, 1H) 5.03 - 5.27 (m, 2H) 4.08 - 4.33 (m, 1H) 3.60 - 3.99 (m, 6H) 3.47 (br s, 1H) 1.98 (spt, 1H) 0.90 - 1.16 (m, 6H).

### Example 16

### 6-[(4-Chlorophenyl)methyl]-8-[(oxan-4-yl)amino]-2-(propan-2-yl)-2,6-dihydroimidazo[1,2-c]pyrido[2,3-e]pyrimidin-5(3H)-one

In a vial equipped with a magnetic stirring bar 4,4'-di-t-butyl-2,2'-bipyridine)bis[3,5-difluoro-2-[5-trifluoromethyl-2-pyridinylkN)phenyl-kC]iridium(III) hexafluorophosphate (3.1 mg), tetrahydropyran-4-amine (35.8 µL, 0.35 mmol ), DABCO (27.93 mg, 0.250 mmol) and 8-bromo-6-(4-chlorobenzyl)-2-isopropyl-2,6-dihydroimidazo[1,2-c]pyrido[2,3-e]pyrimidin-5(3H)-one (Intermediate 17, 60 mg, 0.140 mmol ) were dissolved in DMA (1 mL) . Then Nickel(2+) chloride - 1,2-dimethoxyethane (1:2:1) (6.08 mg, 0.030 mmol ) was added as a solution in DMA (0.30 mL). The vial was then placed under N₂, cooled at -78°C, degassed, backfilled with N₂ and heated up slowly to room temperature. The cycle was repeated 3 times, then the reaction mixture was irradiated (blue light) while stirring for 3h. UPLC-MS showed the formation of desired product. The reaction mixture was filtered, evaporated and purified by means of FractionLynx preparative HPLC to get 14 mg (22.3% yield) of 6-(4-chlorobenzyl)-2-isopropyl-8-((tetrahydro-2H-pyran-4-yl)amino)-2,6-dihydroimidazo[1,2-c]pyrido[2,3-e]pyrimidin-5(3H)-one as powder.

HPLC purity > 99 %

MS (ESI) m/z 454.21 [M + H]⁺

¹H NMR (400 MHz, DMSO-d6) δ ppm 11.51 (br s, 1H), 8.09 (br s, 1H), 7.81 (br d, J = 6.8 Hz, 1H), 7.43 (s, 4H), 6.41 (br s, 1H), 5.45 - 5.26 (m, 2H), 4.40 - 4.24 (m, 2H), 4.12 - 3.96 (m, 1H), 3.82 (br s, 2H), 3.70 - 3.47 (m, 2H), 2.00 (br d, J = 5.9 Hz, 1H), 1.57 (br s, 2H), 1.38 - 1.14 (m, 3H), 0.98 (br t, J = 8.1 Hz, 6H)

### Example 17

### 5-[(4-Chlorophenyl)methyl]-9-(1-hydroxy-2-methylpropan-2-yl)-3-(morpholin-4-yl)-8,9-dihydroimidazo[1',2':1,6]pyrimido[5,4-c]pyridazin-6(5H)-one

### tert-Butyl (4,4-dimethyl-2-oxooxolan-3-yl)carbamate (Intermediate 31)

To a suspension of (4,4-dimethyl-2-oxooxolan-3-yl)amine hydrochloride (4.4 g, 26.57 mmol) in 50 mL of DCM, 11.1 mL of TEA (8.06 g, 79.70 mmol) were added. The resulting solution was cooled at 0-5°C and a solution of di-tert-butyl dicarbonate (8.697 g, 39.850 mmol) in DCM was dripped maintaining the reaction temperature at 0-5°C. The reaction was then stirred at room temperature overnight. Afterwards, it was concentrated under vacuum, diluted with EtOAc, washed with a saturated aq. solution of citric acid, followed by NaHCOs aq. solution, water, dried over anhydrous Na₂SO₄, filtered and concentrated to afford 6.58 g of a white solid used in the next step without any further purification.

MS (ESI) m/z 230.21 [M + H]⁺

¹H NMR (400 MHz, DMSO-*d6*) δ ppm 4.82 (br s, 1H) 4.38 (br d, 1H) 4.00 - 4.08 (m, 2H) 1.49 (s, 9H) 1.27 (s, 3H) 1.03 (s, 3H)

### tert-Butyl (1,4-dihydroxy-3,3-dimethylbutan-2-yl)carbamate (Intermediate 32)

To a suspension of tert-Butyl (4,4-dimethyl-2-oxooxolan-3-yl)carbamate (Intermediate 31, 6.58 g, 28.7 mmol) in 50 mL of THF cooled at 0-5°C was added a 2M THF solution of LiAlH₄ (21.5 mL, 43.05 mmol). The reaction was then stirred at room temperature overnight, then quenched with water and extracted with EtOAc. The organic phase was dried on anhydrous Na₂SO₄, filtered, and evaporated *in vacuo* to obtain 5 g (74.6 %yield) of the title compound as white solid.

MS (ESI) m/z 234.26 [M + H]⁺

### 3-Amino-2,2-dimethyl-butane-1,4-diol (Intermediate 33)

To a suspension of tert-butyl N-[3-hydroxy-1-(hydroxymethyl)-2,2-dimethylpropyl]carbamate (Intermediate 32, 5 g, 21.432 mmol) in 5 mL of DCM, was added a 2M hydrochloric acid solution in diethyl ether (32.1 mL, 64.295 mmol). The reaction was stirred at r.t. overnight. Afterwards, the solvent was concentrated *in vacuo* to obtain 3.6 g (99% yield) of 3-amino-2,2-dimethyl-butane-1,4-diol hydrochloride as raw material, which was used in the next step without further purification.

MS (ESI) m/z 134.16 [M + H]⁺

### Methyl 6-chloro-4-[(4-chlorophenyl)methylamino]pyridazine-3-carboxylate (Intermediate 34)

To a solution of methyl 4,6-dichloropyridazine-3-carboxylate (10 g, 48.307 mmol) in 80 mL of acetonitrile was added DIPEA (9.26 mL, 6.867 g, 53.14 mmol), followed by (4-chlorophenyl)methanamine (6.49 mL, 7.524 g, 53.138 mmol). The mixture was stirred at room temperature for 3h. A white precipitate was formed. The suspension was cooled at 0-5°C and filtered. The solid was washed with cold acetonitrile and dried under vacuum at room temperature to obtain 15 g (yield: 99%) of methyl 6-chloro-4-[(4-chlorophenyl)methylamino]pyridazine-3-carboxylate as light brown powder.

MS (ESI) m/z 311.88 [M + H]⁺

### 6-Chloro-4-[(4-chlorophenyl)methylamino]pyridazine-3-carboxamide (Intermediate 35)

Methyl 6-chloro-4-[(4-chlorophenyl)methylamino]pyridazine-3-carboxylate (Intermediate 34, 8.635 g, 27.66 mmol) were suspended in 158 mL of a 7 N solution of ammonia in methanol and stirred at 50 °C for 1 h. The suspension was cooled at room temperature and the solvent was removed *in vacuo* to the final volume of about 30 mL. The mixture was filtered off to obtain a white solid, which was dried *in vacuo* at room temperature to obtain 7834 mg (yield: 95.35 %) of 6-chloro-4-[(4-chlorophenyl)methylamino]pyridazine-3-carboxamide.

MS (ESI) m/z 297.01 [M + H]⁺

### 4-{[(4-Chlorophenyl)methyl]amino}-6-(morpholin-4-yl)pyridazine-3-carboxamide (Intermediate 36)

To a solution of 6-chloro-4-[(4-chlorophenyl)methylamino]pyridazine-3-carboxamide (Intermediate 35, 5 g, 16.83 mmol) in 20 mL of DMAC, morpholine was added (14.7 mL, 14.66 g, 168.3 mmol) and the solution was stirred at 120°C for 2.5 h. The mixture was cooled to r.t.. and 20 mL of water were added. The obtained precipitated solid was filtered and washed with 24 mL of water in 3 portions. The solid was dried under vacuum to obtain 5.88 g (100% yield) of the title product, which was used in the next step without any further purification.

MS (ESI) m/z 348.05 [M + H]⁺

### 5-[(4-Chlorophenyl)methyl]-3-(morpholin-4-yl)pyrimido[5,4-c]pyridazine-6,8(5H,7H)-dione (Intermediate 37)

To a solution, of 4-[(4-chlorophenyl)methylamino]-6-morpholino-pyridazine-3-carboxamide (Intermediate 36, 6.1 g, 17.54 mmol) in 10 mL of dry DMF cooled at 0-5°C, was added 60% sodium hydride oil dispersion (3.507 g, 87.69 mmol) followed, after stirring 15 minutes, by CDI (8.532 g, 52.62 mmol) portionwise in 15 minutes. The mixture was stirred at 0-5°C for 30 min, then the temperature was warmed up to room temperature. The reaction was cooled at 0-5°C and 5 ml of acetic were added until pH 5. Water was added dropwise (50 mL) to obtain a white off precipitate, that was filtered and dried under vacuum in a oven at 50°C for 4 hours to obtain 6.47 g (88% yield) of the desired title compound.

MS (ESI) m/z 374.03 [M + H]⁺

### 5-[(4-Chlorophenyl)methyl]-3-(morpholin-4-yl)-8-sulfanylidene-7,8-dihydropyrimido[5,4-c]pyridazin-6(5H)-one (Intermediate 38)

To a solution of 5-[(4-chlorophenyl)methyl]-3-(morpholin-4-yl)pyrimido[5,4-c]pyridazine-6,8(5H,7H)-dione (Intermediate 37, 6.740 g, 18.03 mmol) in 150 mL of pyridine, Lawesson's reagent (21.88 g, 54.09 mmol) was added and the mixture was stirred at 120°C for 8h. After cooling at room temperature, 150 mL of water were added. The yellow precipitate formed, was filtered and dried under vacuum to obtain 4.7 g (67 % yield) of the title compound, as a yellow powder, used in the next step without any further purification.

MS (ESI) m/z 390.21 [M + H]⁺

### 5-[(4-Chlorophenyl)methyl]-8-(methylsulfanyl)-3-(morpholin-4-yl)pyrimido[5,4-c]pyridazin-6(5H)-one (Intermediate 39)

To a suspension of 5-[(4-chlorophenyl)methyl]-3-(morpholin-4-yl)-8-sulfanylidene-7,8-dihydropyrimido[5,4-c]pyridazin-6(5H)-one (Intermediate 38, 175 mg, 0.449 mmol) in 3 mL of EtOH were added 3 mL of potassium carbonate aq. saturated solution. The mixture was cooled at 0-5°C, iodomethane 0.0419 mL, 95.56 mg, 0.6732 mmol) was added and the reaction was stirred at room temperature for 90 minutes. The suspension was filtered off to obtain a solid which was dried under vacuum to yield 180 mg (99 %) of the title compound as a yellow powder, which was used in the next step without any further purification.

MS (ESI) m/z 404.05 [M + H]⁺

### 5-[(4-Chlorophenyl)methyl]-8-[(1,3-dihydroxy-3-methylbutan-2-yl)amino]-3-(morpholin-4-yl)pyrimido[5,4-c]pyridazin-6(5H)-one (Intermediate 40)

To a solution cooled at 0 - 5°C of 5-[(4-chlorophenyl)methyl]-8-methylsulfanyl-3-morpholino-pyrimido[5,4-c]pyridazin-6-one (Intermediate 39, 42 mg, 0.1040 mmol) in 1 mL of dry DMSO was added 3-amino-2,2-dimethylbutane-1,4-diol hydrochloride (Intermediate 33, 0.1764 g 1.040 mmol) dissolved in 1 mL of dry DMSO and 0.289 mL of TEA (0.2104 g, 2.080 mmol). The mixture was heated at 130°C for 3 h. After cooling at r.t., water and EtOAc were added, the two phases separated and the aqueous layer was extracted with EtOAc. The combined organic phases were washed with brine, dried over anhydrous Na₂SO₄, filtered and evaporated under vacuum. The crude residue was purified by flash chromatography using a Biotage Selekt^{®} instrument, cartridge type SNAP10, eluting with a gradient from EtOAc 100% to EtOAc - MeOH 8:2. The fractions collected and evaporated gave 24 mg (47 % yield) of the title compound as a yellow oil.

MS (ESI) m/z 489.27 [M + H]⁺

### 5-[(4-Chlorophenyl)methyl]-9-(1-hydroxy-2-methylpropan-2-yl)-3-(morpholin-4-yl)-8,9-dihydroimidazo[1',2':1,6]pyrimido[5,4-c]pyridazin-6(5H)-one (Example 17)

To a solution of 5-[(4-chlorophenyl)methyl]-8-[(1,3-dihydroxy-3-methylbutan-2-yl)amino]-3-(morpholin-4-yl)pyrimido[5,4-c]pyridazin-6(5H)-one (Intermediate 40, 24 mg, 0.0491 mmol) in 1 mL of anhydrous DMF cooled at 0-5°C were added TEA (013.7 µL, 0.098 mmol) and methanesulfonyl chloride (3.8 µL, 5.62 mg, 0.049 mmol) and the mixture was stirred at r.t. for 1 h. Afterwards, an additional, equivalent (3.8 µL) of methanesulfonyl chloride was added and the mixture stirred at room temperature overnight. UPLC-MS showed two main peaks, one corresponding to the desired product [M+H]⁺ = 471 and the other one corresponding to its methanesulfonyl derivative [M+H]⁺ = 549. The reaction was quenched with a 3N aqueous solution of NaOH and the mixture was stirred for 30 min until complete cleavage of the methanesulfonyl ester cited above. The aqueous basic solution was extracted with EtOAc. The organic layer was washed with water, brine, dried over anhydrous Na₂SO₄, filtered, and evaporated *in vacuo.* The crude residue was purified by flash chromatography using a Biotage Selekt^{®} instrument, cartridge type SNAP10, eluting with a gradient from EtOAc 100 to EtOAc - MeOH 85:15. The combined collected fractions gave 5 mg (21.6 % yield) of the title compound as a light-yellow powder.

HPLC purity = 97.47 %

MS (ESI) m/z 471.25 [M + H]⁺

¹H NMR (400 MHz, CHLOROFORM-d) δ ppm 7.34 - 7.43 (m, 2H) 7.21 (d, 2H) 5.99 (s, 1H) 4.98 - 5.30 (m, 2H) 4.42 (dd, 1H) 4.07 (t, 1H) 3.93 (dd, 1H) 3.76 - 3.86 (m, 5H) 3.51 - 3.67 (m, 5H) 0.98 (d, 6H).

### Example 18

### 5-[(4-Chlorophenyl)methyl]-9-(2-methoxypropan-2-yl)-3-(morpholin-4-yl)-8,9-dihydroimidazo[1',2':1,6]pyrimido[5,4-c]pyridazin-6(5H)-one

### 3-tert-Butyl 4-methyl 2,2-dimethyl-1,3-oxazolidine-3,4-dicarboxylate (Intermediate 41)

To a solution of Boc-D,L-serine methyl ester (5.5 g, 25.087 mmol) in 50 mL of toluene, 2,2-dimethoxypropane (6.17 mL, 5.225 g, 50.173 mmol) and pyridinium 4-methylbenzenesulfonate (94.56 mg, 0.376 mmol) were added and the reaction mixture was heated at 100°C for 16 h. After cooling at room temperature, sodium bicarbonate saturated solution and EtOAc were added, the two phases were separated, the organic layer was dried over Na₂SO₄, filtered and evaporated. The crude residue was purified by flash chromatography using a Biotage Selekt^{®} instrument, cartridge type SNAP50, eluting with PE : EtOAc using a gradient from 85:15 to 50:50. The evaporation of the combined collected fractions gave 4 g (61.49% yield) of the title compound as a yellow oil.

MS (ESI) m/z 260.21 [M + H]⁺

### tert-Butyl 4-(2-hydroxypropan-2-yl)-2,2-dimethyl-1,3-oxazolidine-3-carboxylate (Intermediate 42)

To a solution of Intermediate 41 (7 g, 26.996 mmol) in 150 mL of anhydrous THF cooled at - 15°C, under nitrogen atmosphere methylmagnesium bromide (1.4 M solution in THF - toluene 3: 1, 64 mL, 89.086 mmol) was added. The mixture was stirred at 0°C for 1 h, and then at room temperature overnight. The mixture was cooled at 0-5°C and then carefully quenched with water. The aqueous layer was repeatedly extracted with EtOAc. Afterwards, the combined organic phases were washed with brine, dried over anhydrous Na₂SO₄, filtered and evaporated *in vacuo.* The obtained residue 6.96 g (99.4% yield) as a yellow oil was used in the next step without any further purification.

MS (ESI) m/z 260.21 [M + H]⁺

### tert-Butyl 4-(2-methoxypropan-2-yl)-2,2-dimethyl-1,3-oxazolidine-3-carboxylate (Intermediate 43)

To an ice bath cooled suspension of sodium hydride (60% dispersion in mineral oil,1.234 g, 30.85 mmol) in 50 mL of dry DMF stirred under nitrogen atmosphere, a solution of tert-butyl 4-(2-hydroxypropan-2-yl)-2,2-dimethyl-1,3-oxazolidine-3-carboxylate (Intermediate 42, 4 g, 15.43 mmol) in 10 mL of dry DMF was added. After 15 min, iodomethane (1.92 mL, 4.379 g, 30.85 mmol) was added and the mixture was stirred at room temperature for 36 h. The reaction was quenched with water. EtOAc was added, and the two phases were separated. The organic layer was washed with water and brine, dried over anhydrous Na₂SO₄, filtered and evaporated *in vacuo.* The crude residue was purified by means of a Biotage Selekt^{®} instrument, cartridge type SNAP50, eluting with PE - EtOAc in gradient from 100:0 to 80:20. The collected fractions gave 2.74 g (65% yield) of Intermediate 43 as a clear oil.

MS (ESI) m/z 274.07 [M + H]⁺

### 2-Amino-3-methoxy-3-methylbutan-1-ol hydrochloride (Intermediate 44)

To a stirred solution of tert-butyl 4-(2-methoxypropan-2-yl)-2,2-dimethyl-1,3-oxazolidine-3-carboxylate (Intermediate 43, 2.74 g, 10.02 mmol) in 15 mL of methanol, cooled with an ice bath at 0-5°C, 15 mL of a 4N HCl solution in 1,4 dioxane were added and the mixture stirred at room temperature for 15 min. The solvent was removed *in vacuo* to afford 1.6 g (yield 94 %) of the title compound as an off-white powder.

MS (ESI) m/z 134.03 [M + H]⁺

### 5-[(4-Chlorophenyl)methyl]-8-[(1-hydroxy-3-methoxy-3-methylbutan-2-yl)amino]-3-(morpholin-4-yl)pyrimido[5,4-c]pyridazin-6(5H)-one (Intermediate 45)

To an ice bath cooled solution of crude 5-[(4-chlorophenyl)methyl]-8-methylsulfanyl-3-morpholino-pyrimido[5,4-c]pyridazin-6-one (Intermediate 39, 70 mg, 0.1733 mmol) in 1 mL of dry DMSO, 2-amino-3-methoxy-3-methylbutan-1-ol hydrochloride (Intermediate 44, 140 mg, 0.867 mmol) was added, followed by 0.217 mL of TEA (158 mg, 1.56 mmol). The mixture was stirred at room temperature for 2 h, then heated at 130°C for 12 h. After cooling at room temperature, water and EtOAc were added, the two phases were separated and the aqueous layer was extracted with EtOAc. The combined organic phases were washed with brine, dried over anhydrous Na₂SO₄, filtered and evaporated to dryness *in vacuo.* 110 mg (99% yield) of the crude title compound as a dark brown oil were obtained.

MS (ESI) m/z 489.27 [M + H]⁺

### 5-[(4-Chlorophenyl)methyl]-9-(2-methoxypropan-2-yl)-3-(morpholin-4-yl)-8,9-dihydroimidazo[1',2':1,6]pyrimido[5,4-c]pyridazin-6(5H)-one (Example 18)

To a solution of 5-[(4-chlorophenyl)methyl]-8-[(1-hydroxy-3-methoxy-3-methylbutan-2-yl)amino]-3-(morpholin-4-yl)pyrimido[5,4-c]pyridazin-6(5H)-one (Intermediate 45, 84 mg, 0.1718 mmol) in 2 mL of dry DMF cooled at 0-5°C, 0.478 mL of TEA (348.8 mg, 3.445 mmol) were added followed by methanesulfonyl chloride (0.199 mL, 295 mg 2.59 mmol). The mixture was stirred at room temperature overnight. Then, sodium bicarbonate aq. saturated solution and EtOAc were added. The two phases were separated, the aqueous layer was extracted with EtOAc. The combined organic phases were dried over anhydrous Na₂SO₄, filtered and evaporated. The crude residue was purified by flash chromatography with a Biotage Selekt^{®} instrument, cartridge type SNAP10, eluting with a gradient from EtOAc - MeOH 95:5 to EtOAc - MeOH 80:20. The collected fractions afforded 23 mg (yield: 28.4%) of the title compound as light-yellow powder.

HPLC purity = 97.1 %

MS (ESI) m/z 471.17 [M + H]⁺

¹H NMR (400 MHz, DMSO-*d6*) δ ppm 7.24 - 7.52 (m, 4H) 6.51 (s, 1H) 5.03 - 5.31 (m, 2H) 4.30 (dd, 1H) 3.78 - 3.96 (m, 2H) 3.65 - 3.75 (m, 4H) 3.55 - 3.65 (m, 4H) 3.18 (s, 3H) 1.29 (s, 3H) 1.11 (s, 3H)

### Example 19

### 5-[(4-Chlorophenyl)methyl]-9-(2-hydroxypropan-2-yl)-3-(morpholin-4-yl)-8,9-dihydroimidazo[1',2':1,6]pyrimido[5,4-c]pyridazin-6(5H)-one

### 2-Amino-3-methylbutane-1,3-diol hydrochloride (Intermediate 46)

To a solution of tert-butyl 4-(2-hydroxypropan-2-yl)-2,2-dimethyl-1,3-oxazolidine-3-carboxylate (Intermediate 42, 0.75 g, 2.892 mmol) in 1 mL of ethanol, 2 mL of a 4N HCl solution in 1,4-dioxane were added and the mixture was heated at 85°C for 16 hours. After cooling at room temperature, additional 2 mL of a 4N HCl solution in 1,4-dioxane were added and the mixture stirred at reflux for 16 h. Afterwards, the solvent was evaporated under reduced pressure affording 344 mg (99.8% yield) of crude title compound used in the next step without any further purification.

MS (ESI) m/z 120.03 [M + H]⁺

### 5-[(4-Chlorophenyl)methyl]-8-[(1,3-dihydroxy-3-methylbutan-2-yl)amino]-3-(morpholin-4-yl)pyrimido[5,4-c]pyridazin-6(5H)-one (Intermediate 47)

To an ice bath cooled solution of crude 5-[(4-chlorophenyl)methyl]-8-methylsulfanyl-3-morpholino-pyrimido[5,4-c]pyridazin-6-one (Intermediate 39, 70 mg, 0.1733 mmol) in 1 mL of dry DMSO, 2-amino-3-methylbutane-1,3-diol hydrochloride (Intermediate 46, 0.351 g, 2.253 mmol), and 0.482 mL of TEA (0.3507 g, 3.466 mmol) were added. The mixture was heated at 130°C for 4 hours. After cooling at room temperature, water and EtOAc were added, and the two phases were separated. The aqueous layer was extracted with EtOAc, and the combined organic phases were washed with brine, dried over anhydrous Na₂SO₄, filtered and evaporated *in vacuo.* The crude residue was purified by means of a Biotage Selekt^{®} instrument, cartridge type SNAP10, using a gradient from EtOAc 100% to EtOAc - MeOH 80:20. The collected fractions afforded 50 mg (60 % yield) of the title compound as a light-yellow powder.

MS (ESI) m/z 475.12 [M + H]⁺

### 5-[(4-Chlorophenyl)methyl]-9-(2-hydroxypropan-2-yl)-3-(morpholin-4-yl)-8,9-dihydroimidazo[1',2':1,6]pyrimido[5,4-c]pyridazin-6(5H)-one (Example 19)

To an ice bath cooled solution of 5-[(4-chlorophenyl)methyl]-8-[(1,3-dihydroxy-3-methylbutan-2-yl)amino]-3-(morpholin-4-yl)pyrimido[5,4-c]pyridazine-6(5H)-one (Intermediate 47, 50 mg, 0.1053 mmol) in 2 mL ofDMF, were added 0.0293 µL of TEA (21.31 mg, 0.2106 mmol) followed by methanesulfonyl chloride (8.15 µL, 12.06 mg, 0.105 mmol). The mixture was stirred at room temperature overnight. 5 additions each of 0.2 equivalents of methanesulfonyl chloride were done every 4 h. Afterwards, water and EtOAc were added, and the two phases separated. The aqueous layer was extracted with two portions of EtOAc, and the combined organic phases were dried over anhydrous Na₂SO₄, filtered and evaporated. The crude residue was purified by means of a Biotage Selekt^{®} instrument, cartridge type SNAP10, eluting with a gradient from EtOAc 100 to EtOAc - MeOH 80:20. The collected fractions afforded 18 mg (yield: 37.4 %) as light-yellow powder.

HPLC purity = 83.1 %

MS (ESI) m/z 458.07 [M + H]⁺

¹H NMR (400 MHz, DMSO-*d6*) δ ppm 7.32 - 7.46 (m, 4H) 6.50 (s, 1H) 5.07 - 5.31 (m, 2H) 4.45 (s, 1H) 4.09 - 4.22 (m, 1H) 3.80 - 3.95 (m, 2H) 3.65 - 3.73 (m, 4H) 3.53 - 3.64 (m, 4H) 1.23 (s, 3H) 1.13 (s, 3H).

### Example 20

### 3-tert-Butyl-6-[(4-methoxyphenyl)methyl]-8-(morpholin-4-yl)imidazo[1,2-c]pyrido[2,3-e]pyrimidine-2,5(3H,6H)-dione

Prepared following the procedure described in Patent US 2021/0246139 for Example 64 and characterized as Example 164.

### Example 21

### 3-tert-Butyl-6-[(4-chlorophenylmethyl]-8-(morpholin-4-yl)imidazo[1,2-c]pyrido[2,3-e]pyrimidine-2,5(3H,6H)-dione

Prepared following the procedure described in Patent US 2021/0246139 for Example 64 and characterized as Example 69.

### Example 22

### 6-[(4-Chlorophenyl)methyl]-8-(morpholin-4-yl)-3-(propan-2-yl)imidazo[1,2-c]pyrido[2,3-e]pyrimidine-2,5(3H,6H)-dione

Prepared following the procedure described in Patent US 2021/0246139 for Example 64 and characterized as Example 68.

Alternatively, the compound of Example 22 was prepared using the following procedure:

### N-[(4-Chlorophenyl)methyl]-N-(2-cyano-5-morpholino-3-pyridyl)carbamoyl chloride (Intermediate 107)

To an ice-bath cooled solution of Intermediate 2 (5g, 15.21 mmol) in 150 mL of DCM, 3.07 mL of pyridine (3.01 g, 38.02 mmol) were added, followed by bis(trichloromethyl) carbonate (2.257 g, 7.603 mmol). The reaction was stirred at 0°C for 30 min, warmed up to room temperature and stirred for 2 hours. The mixture was cooled at 0-5°C and 0.25 equivalents of bis(trichloromethyl) carbonate were added, followed by 1 equivalent of pyridine. The mixture was stirred at room temperature overnight, then the solvent was removed under reduced pressure affording the title compound (5.9 g, 99% yield), which was used in the next step without any further purification.

UPLC-MS [M + H]⁺ = 391.01

### Ethyl (2S)-2-[[(4-chlorophenyl)methyl-(2-cyano-5-morpholino-3-pyridyl)carbamoyl]amino]-3-methyl-butanoate (Intermediate 108)

To a solution of N-[(4-chlorophenyl)methyl]-N-(2-cyano-5-morpholino-3-pyridyl)carbamoyl chloride (Intermediate 107, 5.9 g, 15.08 mmol) in 100 mL of DCM, 7.88 mL of DIPEA (5.848 g, 45.25 mmol) were added, followed by ethyl (2S)-2-amino-3-methyl-butanoate hydrochloride (3.288 g, 18.10 mmol). The pH was adjusted to 8-9 by addition of DIPEA and the reaction mixture was stirred at room temperature for 3h. Then, it was washed with water and brine. The organic layer was dried over anhydrous Na₂SO₄, filtered and evaporated *in vacuo.* The crude residue was purified by means of Biotage Isolera Four instrument, coupled with a Dalton 2000^{®}, using a cartridge type SNAP100, and eluting in gradient with a mixture of PE : EtOAc from 7:3 to 3:7. The collected fractions were combined and concentrated under vacuum to obtain 3.7 g of the title compound as yellow oil, which were used in the next step without further purification.

UPLC-MS [M + H]⁺ = 500.01

### 6-[(4-Chlorophenyl)methyl]-8-(morpholin-4-yl)-3-(propan-2-yl)imidazo[1,2-c]pyrido[2,3-e]pyrimidine-2,5(3H,6H)-dione (Example 22)

To a solution of ethyl (2S)-2-[[(4-chlorophenyl)methyl-(2-cyano-5-morpholino-3-pyridyl)carbamoyl]amino]-3-methyl-butanoate (Intermediate 108, 3.7 g, 7.40 mmol) in 50 mL of anhydrous THF, 1,8-diazabiciclo[5.4.0]undec-7-ene (DBU, 1.107 mL 1.127 g, 7.4 mmol) was added dropwise and the reaction mixture was stirred at room temperature. After 1 hour, a light-yellow precipitate was formed. After 5h of stirring at r.t., the conversion was almost complete. The formed solid was filtered and washed with a small amount of THF, dried overnight under vacuum at 60°C to afford 1.65 g of the title compound. The mother liquors were concentrated under vacuum forming a precipitate, which was filtered, washed with THF, dried affording a second crop of 500 mg of the title compound were obtained. The two precipitates were mixed to afford 2.15 g (yield: 64 %) of the title compound.

HPLC purity = 99.54 %

UPLC-MS [M + H]⁺ = 454.50

¹H NMR (400 MHz, CHLOROFORM-d) δ ppm 8.38 (d, 1H) 7.35 - 7.44 (m, 2H) 7.21 (d, 2H) 6.52 (d, 1H) 5.25 - 5.41 (m, 2H) 4.50 (d, 1H) 3.81 - 3.94 (m, 4H) 3.24 - 3.41 (m, 4H) 2.86 - 3.04 (m, 1H) 1.31 (d, 3H) 0.94 (d, 3H).

### Example 23 and Example 24

### (R)-6-[4-Chlorophenyl)methyl]-8-(morpholin-4-yl)-3-(propan-2-yl)imidazo[1,2-c]pyrido[2,3-e]pyrimidine-2,5(3H,6H)-dione and (S)-6-[(4-chlorophenyl)methyl]-8-(morpholin-4-yl)-3-(propan-2-yl)imidazo[1,2-c]pyrido[2,3-e]pyrimidine-2,5(3H,6H)-dione

Prepared by enantiomer separation by means of preparative HPLC chiral column chromatography starting from the racemate compound of Example 22. The chiral HPLC separation was performed using a column Phenomenex Lux Amylose-2, 250x4.6 mm 5 µm, eluting in isocratic mode with 90% of MeOH and 10% of ACN, at 0.5 mL/min flow for 20 min, at the temperature of 25°C.

UPLC-MS [M + H]⁺ = 454.38

¹H NMR (400 MHz, DMSO-J6) δ ppm 8.46 (d, 1H), 7.34-7.47 (m, 4H), 6.83 (d, 1H), 5.46 (d, 1H), 5.33 (d, 1H), 4.41 (d, 1H), 3.66-3.76 (m, 4H), 3.39-3.56 (m, 4H), 2.69-2.86 (m, 1H), 1.16 (d, 3H), 0.78 (d, 3H)

UPLC-MS [M + H]⁺ = 454.36

¹H NMR (400 MHz, DMSO-*d6*) δ ppm 8.46 (d, 1H), 7.34-7.47 (m, 4H), 6.83 (d, 1H), 5.46 (d, 1H), 5.33 (d, 1H), 4.41 (d, 1H), 3.66-3.76 (m, 4H), 3.39-3.56 (m, 4H), 2.69-2.86 (m, 1H), 1.16 (d, 3H), 0.78 (d, 3H)

### Example 25

### 6-[(4-Chlorophenyl)methyl]-8-(4-oxopiperidin-1-yl)-3-(propan-2-yl)imidazo[1,2-c]pyrido[2,3-e]pyrimidine-2,5(3H,6H)-dione

### 5-(1,4-Dioxa-8-azaspiro[4.5]decan-8-yl)-3-fluoropyridine-2-carbonitrile (Intermediate 48)

In a round bottomed flask, to a solution of 3,5-difluoropyridine-2-carbonitrile (1.5 g, 10.71 mmol) in 20 mL of DMAC were added under stirring 1,4-dioxa-8-azaspiro[4.5]decane (1.373 mL, 1.533 g, 10.707 mmol) and TEA (2.98 mL, 2.17 g, 21.42 mmol). The mixture was heated at 100°C for 3 hours. After cooling at room temperature, water and EtOAc were added. The two phases were separated, the organic layer was washed with water (2x), dried over anhydrous Na₂SO₄, filtered and evaporated *in vacuo.* The crude residue was purified by means of a Biotage Selekt^{®} instrument, cartridge type SNAP50, eluting with a gradient from PE : EtOAc 8:2 to PE : EtOAc 3:7. Evaporation to dryness of the combined fractions afforded 2.19 g (yield: 78%) of the title compound as white powder.

MS (ESI) m/z 264.12 [M + H]⁺

### 3-{[(4-Chlorophenyl)methyl]amino}-5-(1,4-dioxa-8-azaspiro[4.5]decan-8-yl)pyridine-2-carbonitrile (Intermediate 49)

To a solution of 5-(1,4-dioxa-8-azaspiro[4.5]decan-8-yl)-3-fluoro-pyridine-2-carbonitrile (Intermediate 48, 1.3 g, 48, 4.9 mmol) in 40 mL of NMP, (4-chlorophenyl)methanamine (0.66 mL, 0.77 g, 5.4 mmol) and DIPEA (1.7 mL, 1.3 g, 9.9 mmol) were added. The mixture was then stirred at 130°C for 3 hours. After cooling at room temperature, water and EtOAc were added, and the two phases were separated. The organic layer was washed with water, dried over anhydrous Na₂SO₄, filtered and evaporated *in vacuo.* The crude residue was purified by means of a Biotage Isolera^{®} instrument, cartridge type SNAP50, eluting with a gradient from PE : EtOAc 8:2 to PE : EtOAc 3:7. The desired product, 3-[(4-chlorophenyl)methylamino]-5-(1,4-dioxa-8-azaspiro[4.5]decan-8-yl)pyridine-2-carbonitrile (1.20 g, 63 % yield) was isolated as a pale brown solid.

MS (ESI) m/z 385.07 [M + H]⁺

### 4-Amino-1-[(4-chlorophenyl)methyl]-7-(1,4-dioxa-8-azaspiro[4.5]decan-8-yl)pyrido[3,2-d]pyrimidin-2(1H)-one (Intermediate 50)

To a solution of 3-[(4-chlorophenyl)methylamino]-5-(1,4-dioxa-8-azaspiro[4.5]decan-8-yl)pyridine-2-carbonitrile (Intermediate 49, 1 g, 49, 2.60 mmol) in 30 mL of anhydrous THF cooled at 0-5°C stirred under N₂ atm. trichloroacetyl isocyanate (0.31 mL, 489.5 mg, 2.606 mmol) was added dropwise. The reaction mixture was stirred at r.t. until the disappearance on UPLC/MS of the starting material and the formation of the intermediate with MW 573. The reaction was quenched at 0°C with excess MeOH and the solvents were evaporated under reduced pressure. The residue was taken up with 4 mL of 7M ammonia solution in MeOH (28 mmol), stirred overnight to reaction completion. A white precipitate was formed, filtered and washed with cold methanol to afford 300 mg (27 % yield) of the title product, as white solid, which was used in the next step without further purification.

MS (ESI) m/z 428.12 [M + H]⁺

### 6[(4-Chlorophenyl)methyl]-8-(1,4-dioxa-8-azaspiro[4.5]decan-8yl)-3-(propan-2-yl)imidazo[1,2-c]pyrido[2,3-e]pyrimidine-2,5(3H,6H)-dione (Intermediate 51)

To a solution of 2-bromo-3-methyl-butanoic acid (291.9 mg, 1.622 mmol) in 5 mL of toluene were added, a solution of oxalyl chloride (0.14 mL, 204.7 mg, 1.613 mmol) in 5 mL toluene and then two drops of DMF. The solution was stirred at room temperature for 3 hours. Then, the solvent was evaporated and the residue was dissolved in 5 mL of NMP and added to a solution of 4-amino-1-[(4-chlorophenyl)methyl]-7-(1,4-dioxa-8-azaspiro[4.5]decan-8-yl)pyrido[3,2-d]pyrimidin-2-one (Intermediate 50, 230 mg, 0.538 mmol) and DIPEA (0.99 mL, 743.2 mg, 6.45 mmol). Afterwards, the reaction mixture was stirred overnight at room temperature then heated at 120°C for 3 hours. Afterwards, the reaction mixture was poured into water, extracted with EtOAc, and dried over anhydrous Na₂SO₄. The solvent was evaporated to dryness, and the residual crude was purified via automated flash chromatography (Biotage Isolera Four coupled with Dalton 2000^{®} instrument), SNAP10 cartridge, eluting with an isocratic mixture of CHCl₃ - MeOH 95:5. The title product 6, 8-[(4-chlorophenyl)methyl]-8-(1,4-dioxa-8-azaspiro[4.5]decan-8-yl)-3-(propan-2-yl)imidazo[1,2-c]pyrido[2,3-e]pyrimidine-2,5(3H,6H)-dione (170 mg, 62 % yield) was isolated as a yellow solid.

MS (ESI) m/z 510.37 [M + H]⁺

### 6-[(4-Chlorophenyl)methyl]-8-(4-oxopiperidin-1-yl)-3-(propan-2-yl)imidazo[1,2-c]pyrido[2,3-e]pyrimidine-2,5(3H,6H)-dione (Example 25)

To a solution of 6-[(4-chlorophenyl)methyl]-8-(1,4-dioxa-8-azaspiro[4.5]decan-8-yl)-3-(propan-2-yl)imidazo[1,2-c]pyrido[2,3-e]pyrimidine-2,5(3H,6H)-dione (Intermediate 51, 100 mg, 0.196 mmol) in 1 mL of 1,4-dioxane. 1 mL of 6N aqueous HCl solution was added, and the mixture was stirred at room temperature for 30 min. The reaction mixture was then basified with aq. potassium carbonate and the aqueous layer extracted with DCM. The combined organic phases were dried over anhydrous Na₂SO₄, filtered and evaporated. The crude residue was purified by Biotage Isolera^{®} instrument (SNAP10 cartridge), eluting with EtOAc - MeOH in gradient from 95:5 to 90:10. The title product, 6-[(4-chlorophenyl)methyl]-8-(4-oxopiperidin-1-yl)-3-(propan-2-yl)imidazo[1,2-c]pyrido[2,3-e]pyrimidine-2,5(3H,6H)-dione was isolated as a pale yellow solid (30 mg, 32.8 % yield).

HPLC purity = 93.6%

MS (ESI) m/z 466.40 [M + H]⁺

¹H NMR (400 MHz, DMSO-*d6*) δ ppm 8.48 (d, 1H) 7.41 (s, 4H) 6.82 (d, 1H) 5.44 - 5.54 (m, 1H) 5.31 - 5.39 (m, 1H) 4.41 (d, 1H) 3.85 (t, 4H) 2.70 - 2.83 (m, 1H) 2.43 - 2.49 (m, 4H) 1.12 - 1.20 (m, 3H) 0.79 (d, 3H)

### Example 26

### 6-[(4-Chlorophenyl)methyl]-8-(4-hydroxypiperidin-1-yl)-3-(propan-2-yl)imidazo[1,2-c]pyrido[2,3-e]pyrimidine-2,5(3H,6H)-dione

To a solution of the compound of Example 25 (70 mg, 0.1502 mmol) in 5 mL of MeOH stirred at 0-5°C sodium borohydride (8.53 mg, 0.2254 mmol) was added and the reaction mixture was stirred at room temperature overnight. Then it was cooled to 0°C and quenched with water; DCM was added and the two phases were separated. The aqueous layer was extracted with DCM, and the combined extracts were dried over anhydrous Na₂SO₄, filtered and evaporated *in vacuo.* The crude residue was purified by means of a Biotage Isolera One^{®} instrument, SNAP 10 cartridge, eluting with EtOAc - MeOH in gradient from 100:0 to 90:10. The collected fractions gave 40 mg (56.9 % yield) of desired product, 6-[(4-chlorophenyl)methyl]-8-(4-hydroxypiperidin-1-yl)-3-(propan-2-yl)imidazo[1,2-c]pyrido[2,3-e]pyrimidine-2,5(3H,6H)-dione,as a pale yellow solid.

HPLC purity = 88.9%

MS (ESI) m/z 468.39 [M + H]⁺

¹H NMR (400 MHz, DMSO-*d6*) δ ppm 8.45 (d, 1H) 7.41 (s, 4H) 6.77 (d, 1H) 5.41 - 5.54 (m, 1H) 5.32 (d, 1H) 4.76 (d, 1H) 4.39 (d, 1H) 3.81 (dt, 2H) 3.75 (td, 1H) 3.18 - 3.28 (m, 2H) 2.69 - 2.84 (m, 1H) 1.66 - 1.82 (m, 2H) 1.27 - 1.42 (m, 2H) 1.16 (d, 3H) 0.79 (d, 3H)

### Example 27

### 5-[(4-Chlorophenyl)methyl]-8-(2-methoxypropan-2-yl)-3-(morpholin-4-yl)imidazo[1',2':1,6]pyrimido[5,4-c]pyridazine-6,9(5H,8H)-dione

### 8-Amino-5-[(4-chlorophenyl)methyl]-3-morpholino-pyrimido[5,4-c]pyridazin-6-one (Intermediate 97)

A suspension of 5-[(4-chlorophenyl)methyl]-3-(morpholin-4-yl)-8-sulphanilidene-dihydropyrimido[5,4-c]pyridazin-6(5H)-one (Intermediate 38, 1 g, 2.565 mmol) in 10 mL of 7M ammonia solution in MeOH (70 mmol) was stirred at 50-60°C for 2 hours. The mixture was evaporated under vacuum affording a brown solid that was suspended in MeOH and filtered. The solid formed was dried in a oven affording 720 mg (yield: 75.3 %) of the desired product 8-amino-5-[(4-chlorophenyl)methyl]-3-morpholino-pyrimido[5,4-c]pyridazin-6-one, as a pale yellow solid that was used in the next step without further purification.

MS (ESI) m/z 373.79 [M + H]⁺

### 5-[(4-Chlorophenyl)methyl]-8-(2-methoxypropan-2-yl)-3-(morpholin-4-yl)imidazo[1';2':1,6]pyrimido[5,4-c]pyridazine-6,9(5H,8H)-dione (Example 27)

Prepared following the procedure reported for the compound of Example 25 but starting from Intermediate 97. The crude residue was purified by means of a Biotage Selekt^{®} instrument, cartridge type SNAP50, using a mixture of EtOAc - MeOH in gradient from 9:1 to 7:3. The title compound was isolated as a yellow solid (3 % yield).

MS (ESI) m/z 484.23 [M + H]⁺

¹H NMR (400 MHz, DMSO-*d6*) δ ppm 7.36 (d, 1H) 7.36 (d, 1H) 7.25 (s, 1H) 7.24 (d, 1H) 7.24 (d, 1H) 5.09 (spt, 1H) 5.04 (m, 1H) 5.03 (m, 1H) 3.83 (m, 1H) 3.83 (m, 1H) 3.83 (m, 1H) 3.83 (m, 1H) 3.55 (m, 1H) 3.55 (m, 1H) 3.55 (m, 1H) 3.55 (m, 1H) 3.11 (s, 3H) 1.31 (d, 3H) 1.10 (d, 3H)

### Example 28

### 5-[(4-Chlorophenyl)methyl]-3-(morpholin-4-yl)-8-(propan-2-yl)imidazo[1',2':1,6]pyrimido[5,4-c]pyridazine-6,9(5H,8H)-dione

### 4-[(4-Chlorophenyl)methylamino]-6-morpholino-pyridazine-3-carbonitrile (Intermediate 52)

To a solution of 4-[(4-chlorophenyl)methylamino]-6-morpholino-pyridazine-3-carboxamide (Intermediate 36, 5.7 g, 16.39 mmol) and TEA (18.3 mL, 13.27 g, 131.1 mmol) in 200 mL of DCM cooled at 0-5°C, were added portionwise phosphoryl trichloride (5.35 mL, 8.8 g, 57.36 mmol). The mixture was stirred at 0-5°C for 30 min. After overnight stirring at r. 1., the reaction mixture was cooled at 0-5°C, diluted with DCM and washed with aq. NaHCOs saturated solution and water, dried over anhydrous Na₂SO₄ and concentrated under vacuum to obtain 5.3 g of a reddish solid (98 % yield) which was used in the next step without further purifications.

MS (ESI) m/z 330.03 [M + H]⁺

### N-[(4-Chlorophenyl)methyl]-N-(3-cyano-6-morpholino-pyridazin-4-yl)carbamoyl chloride (Intermediate 53)

To a solution of 4-[(4-chlorophenyl)methylamino]-6-morpholino-pyridazine-3-carbonitrile (Intermediate 52, 1.1 g, 3.34 mmol) in 25 mL of DCM cooled at 0-5°C were added 0.5 mL of pyridine (0.495 g, 6.25 mmol), followed by dropwise addition of a solution of *bis*(trichloromethyl) carbonate (0.62 g, 2.085 mmol) in 8 mL of DCM. The reaction was stirred at 0-5°C for 30 min, then warmed up to r.t. and stirred for two days. The solvent was removed under vacuum affording the crude title compound (1.3 g, 99 % yield), used in the next step without any further purification.

MS (ESI) m/z 392.05 [M + H]⁺

### Ethyl (2S)-2-[[(4-chlorophenyl)methyl-(3-cyano-6-morpholino-pyridazin-4-yl)carbamoyl]amino]3-methyl-butanoate (Intermediate 54)

To a solution of N-[(4-chlorophenyl)methyl]-N-(3-cyano-6-morpholino-pyridazin-4-yl)carbamoyl chloride (Intermediate 53, 1.3 g, 3.323 mmol) in 40 mL of DCM were added 1.73 mL of DIPEA (1.285 g, 9.944 mmol) and ethyl (2S)-2-amino-3-methyl-butanoate hydrochloride (0.602 g, 3.323 mmol). The reaction mixture was stirred at r.t. for 2 days. The solvent was removed under vacuum. The residue was dissolved in EtOAc and washed with water, brine and dried over anhydrous Na₂SO₄, filtered and evaporated. The crude residue was purified by chromatography using a Biotage Selekt^{®} instrument, cartridge type SNAP10, eluting with a mixture of PE: EtOAc in gradient from 7:3 to 3:7. The combined collected fractions gave by evaporation to dryness 590 mg of the title compound (35 % yield).

MS (ESI) m/z 501.5 [M + H]⁺

### 5-[(4-Chlorophenyl)methyl]-3-(morpholin-4-yl)-8-(propan-2yl)imidazo[1',2':1,6]pyrimido[5,4-c]pyridazine-6,9(5H,8H)-dione (Example 28)

To a solution of ethyl (2S)-2-[[(4-chlorophenyl)methyl-(3-cyano-6-morpholino-pyridazin-4-yl)carbamoyl]amino]-3-methyl-butanoate (Intermediate 54, 0.59 g, 1.18 mmol) in 10 mL of anhydrous THF was added dropwise DBU (0.176 mL, 0.1793 g, 1.178 mmol) and the reaction mixture was stirred at r.t. overnight. The formed precipitate was filtered and washed with a small amount of THF, dried overnight under vacuum at 60°C, to yield 190 mg (35% yield) of the title compound as a pale-yellow powder.

HPLC purity 93.48%

MS (ESI) m/z 455.34 [M + H]⁺

¹H NMR (400 MHz, DMSO-d6) δ ppm 7.40 (d, 4H) 6.64 (s, 1H) 5.16 - 5.47 (m, 2H) 4.42 (d, 1H) 3.64 - 3.88 (m, 8H) 2.71 (dtt, 1H) 1.17 (d, 3H) 0.82 (d, 3H).

### Example 29

### 3-tert-Butyl-6-(4-methoxybenzyl)-8-(morpholin-4-yl)pyrido[2,3-e][1,2,4]triazolo[4,3-c]pyrimidin-5(6H)-one

The title compound was prepared by the procedures described for the compound of Example 96 belonging to Patent US 2021/0246139.

An alternative preparation pathway is described below:

### 3-[(4-Methoxyphenyl)methylamino]-5-morpholino-pyridine-2-carbonitrile (Intermediate 55)

To a solution of 3-fluoro-5-morpholino-pyridine-2-carbonitrile (Intermediate 1, 450 mg, 2.17 mmol) in 5 mL of NMP in a microwave vial were added (4-methoxyphenyl)methanamine (0.311 mL , 327.72 mg, 2.399 mmol) and 0.744 mL of DIPEA (561.4 mg, 4.34 mmol). The mixture was stirred at 150°C under microwave irradiation for 1 h. After cooling at room temperature water was added to the mixture. The white solid which precipitated was filtered, washed with water and dried under vacuum to obtain 450 mg of 3-[(4-methoxyphenyl)methylamino]-5-morpholino-pyridine-2-carbonitrile (yield: 63.87%) as a pale yellow solid, which was used in the next step without further purification.

MS (ESI) m/z 325.52 [M + H]⁺

### 3-[(4-Methoxyphenyl)methylamino]-5-morpholino-pyridine-2-carboxamide (Intermediate 56)

To a solution of 3-[(4-methoxyphenyl)methylamino]-5-morpholino-pyridine-2-carbonitrile (Intermediate 55, 15 g, 46.24 mmol) in 400 mL of DMSO cooled at 0-5°C were added 28 mL of 1N aq. sodium hydroxide solution (28 mmol) followed by 30-35% hydrogen peroxide solution (5.677 mL, 6.29 g, 55.49 mmol). The mixture was stirred at room temperature for 3 hours. Water and EtOAc were added to the reaction mixture, the two phases were separated and the aqueous layer was extracted with EtOAc. The combined organic layers were dried over anhydrous Na₂SO₄, filtered and evaporated *in vacuo.* The crude residue was crystallized from acetonitrile affording 9 g of the desired product. The mother liquors were evaporated under vacuum and purified on a Biotage Isolera^{®}, instrument, using a SNAP50 cartridge, eluting with a gradient PE - EtOAc from 80:20 to 20:80. A total amount of 12 g of the title product were obtained as brown solid (yield: 75.8%).

MS (ESI) m/z 343.17 [M + H]⁺

¹H NMR (400 MHz, DMSO-d6) δ ppm 8.73 (t, 1H) 7.68 (br d, 1H) 7.58 (d, 1H) 7.24 - 7.35 (m, 2H) 7.05 (br d, 1H) 6.85 - 6.96 (m, 2H) 6.43 (d, 1H) 4.33 (d, 2H) 3.74 (s, 3H) 3.68 - 3.73 (m, 4H) 3.15 - 3.26 (m, 4H).

### 1-[(4-Methoxyphenyl)methyl]-7-morpholino-pyrido[3,2-d]pyrimidine-2,4-dione (Intermediate 57)

To a solution cooled at 0-5°C of 3-[(4-methoxyphenyl)methylamino]-5-morpholino-pyridine-2-carboxamide (Intermediate 56, 2 g, 5.84 mmol) cooled at 0-5°C in 30 mL of dry DMF under nitrogen atmosphere sodium hydride 60 % oil dispersion (1.402 g, 35.05 mmol) was added. The suspension was stirred for 15 min at 0-5°C, then di(imidazol-1-yl)methanone (CDI, 5.68 g , 35.05 mmol) was added and the mixture was stirred at r.t. for 30 min. After overnight stirring at room temperature, the reaction was cooled at 0-5°C and acetic acid was added carefully until pH 5, followed by 400 mL of water.. The obtained yellow precipitate was filtered and dried in a oven at 50°C affording 730 mg of the title compound (yield: 30.5%) as a yellow solid.

MS (ESI) m/z 369.03 [M + H]⁺

### 4-Chloro-1-[(4-methoxyphenyl)methyl]-7-morpholino-pyrido[3,2-d]pyrimidin-2-one (Intermediate 58)

To a mixture of 1-[(4-methoxyphenyl)methyl]-7-morpholino-pyrido[3,2-d]pyrimidine-2,4-dione (Intermediate 57, 4.7 g, 12.76 mmol) in 6.55 mL of DIPEA (4946 mg, 38.27 mmol) under nitrogen atmosphere, 164 mL of POCl₃ (273.9 g, 1786 mmol, 140 eq.) were cautiously added dropwise and the mixture was stirred at 50°C for 1h. To check the completion of the reaction, the crude was quenched with MeOH, checking by UPLC/MS the methoxy derivative formation. The mixture was cooled down to room temperature, POCl₃ was evaporated under reduced pressure and the residue was diluted with 1,4-dioxane and evaporated (3 times) to remove the residual POCl₃. The obtained crude title compound was used in the next step without any further purification.

### 3-tert-Butyl-6-(4-methoxybenzyl)-8-(morpholin-4-yl)pyrido[2,3-e][1,2,4]triazolo[4,3-cjpyrimidin-5(6H)-one (Example 29)

A solution of 4-chloro-1-[(4-methoxyphenyl)methyl]-7-morpholino-pyrido[3,2-d]pyrimidin-2-one (Intermediate 58, 4.9 g, 12.67 mmol) and 2,2-dimethylpropionhydrazide (2.21 g, 19.00 mmol) in 200 mL of 1,4-dioxane was stirred at room temperature for 30 min. Then, the mixture was heated at reflux for 10 hours. After cooling at room temperature, water and EtOAc were added, and the two phases were separated. The aqueous layer was extracted again with EtOAc and the combined organic phases were dried over anhydrous Na₂SO₄, filtered and evaporated under reduced pressure. The crude residue was purified by the chromatographic system Biotage Isolera One^{®}, cartridge type SNAP100, using a gradient of EtOAc - MeOH from 100:0 to 85:15. The collected fractions (1.3 g) were further purified by reverse phase column chromatography using the Isolera One system, cartridge type SNAP60 KP-C18-HS, eluting with a gradient from NH₄HCO₃ buffer - MeCN 8:2 to NH₄HCO₃ buffer - ACN 1:1. The collected fractions gave 775 mg of the title compound as a light yellow powder (yield: 13.6 %).

HPLC purity > 97%

MS (ESI) m/z 449.26 [M + H]^{+ 1}H NMR (400 MHz, DMSO-d6) δ ppm 8.38 (d, 1H) 7.34 (d, 2H) 7.08 (d, 1H) 6.83 - 6.95 (m, 2H) 5.45 (s, 2H) 3.73 - 3.80 (m, 4H) 3.72 (s, 3H) 3.32 (br s, 4H) 1.58 (s, 9H).

### Example 30

### 3-tert-Butyl-6-[(4-chlorophenyl)methyl]-8-(morpholin-4-yl)pyrido[2,3-e][1,2,4]triazolo[4,3-c]pyrimidin-5(6H)-one

### Methyl 5-bromo-3-[(4-chlorophenyl)methylamino]pyridine-2-carboxylate (Intermediate 59)

To a mixture of methyl 3-amino-5-bromo-pyridine-2-carboxylate (0.6 g, 2.606 mmol), 4-chlorobenzaldehyde (0.73 g, 5.202 mmol) and 0.297 mL of acetic acid (0.312 g, 5.194 mmol) in 50 mL of DCM, sodium triacetoxyborohydride (2.75 g, 12.99 mmol) was added, and the resulting suspension was stirred at room temperature for 8 hours. The reaction was quenched with water, the two layers were separated and the aqueous layer was extracted with DCM. The combined organic phases were dried over anhydrous Na₂SO₄, filtered and evaporated at reduced pressure. The crude residue was purified by the Biotage Isolera One^{®} system, cartridge type SNAP100, eluting with PE : EtOAc using a gradient from 100:0 to 60:40. The collected fractions afforded 0.65 g of the title compound methyl 5-bromo-3-[(4-chlorophenyl)methylamino]pyridine-2-carboxylate (yield: 70.4%) as a pale yellow solid.

MS (ESI) m/z 354.89 [M +H]⁺

### 7-Bromo-1-[(4-chlorophenyl)methyl]pyrido[3,2-d]pyrimidine-2,4-dione (Intermediate 60)

To a stirred solution of methyl 5-bromo-3-[(4-chlorophenyl)methylamino]pyridine-2-carboxylate (Intermediate 59, 1.4 g, 3.94 mmol) in 40 mL of DCM was added 2,2,2-trichloroacetyl isocyanate (0.516 mL, 815.9 mg, 4.331 mmol) and the reaction was stirred at room temperature overnight. The solvent was evaporated and 9.00 mL of 25% sodium methoxide in methanol (39.37 mmol) were added and the suspension was heated at 60°C and stirred for 1 hour. After cooling at room temperature, the mixture was quenched with glacial acetic acid and water until complete dissolution (pH = 4-5). The aqueous layer was repeatedly extracted with DCM. The collected organic layers were dried over anhydrous Na₂SO₄, filtered and evaporated. The crude residue was purified via automated flash chromatography (Biotage Isolera One^{®}, SNAP25 cartridge) eluting with PE - EtOAc in gradient from 80:20 to 100:0.

The fractions collected by evaporation afforded 170 mg of the desired product, 7-bromo-1-[(4-chlorophenyl)methyl]pyrido[3,2-d]pyrimidine-2,4-dione, (yield: 11.8%) as a pale yellow solid.

MS (ESI) m/z 365.68 [M + H]⁺

### 7-Bromo-4-chloro-1-[(4-chlorophenyl)methyl]pyrido[3,2-d]pyrimidin-2-one (Intermediate 61)

To a suspension of 7-bromo-1-[(4-chlorophenyl)methyl]pyrido[3,2-d]pyrimidine-2,4-dione (Intermediate 60, 170 mg, 0.464 mmol) in 0.29 mL of DIPEA (213.7 mg,1.855 mmol) was added POCl₃ (1.15 mL, 1.92 g, 12.52 mmol) and the resulting mixture was stirred at 60°C for 2 hours. Then the reaction was cooled at r.t. and the solvent was evaporated. The residue was rinsed three times with 1,4-dioxane and evaporated to remove the residual POCl₃. The obtained crude was used for the next step without further purification.

MS (ESI) m/z 379.88 [M + H]⁺ (methanol derivative)

### 8- Bromo-3-tert-butyl-6-[(4-chlorophenyl)methyl]pyrido[2,3-e][1,2,4]triazolo[4,3-c]pyrimidin-5(6H)-one (Intermediate 62)

To a solution of 7-bromo-4-chloro-1-[(4-chlorophenyl)methyl]pyrido[3,2-d]pyrimidin-2-one (Intermediate 61, 0.53 g, 1.38 mmol) in 10 mL of dioxane was added 2,2-dimethylpropanehydrazide (0.24 g, 2.065 mmol). The reaction mixture was stirred for 20 min at r.t. and then refluxed for 20 min. The mixture was cooled to r.t. and the solvent was evaporated to dryness. The crude was purified twice via automated flash chromatography by Biotage-Isolera Four coupled with Dalton 2000^{®}, using SNAP50 and SNAP25 cartridges, eluting with a gradient of EtOAc - PE from 1:1 to 9: 1 affording 215 mg (35 % yield) of the title compound.

MS (ESI) m/z 445.98 [M + H]⁺

¹H-NMR (400 MHz, DMSO-d6) δ ppm 1.57 (s, 9H) 5.49 (s, 2H) 7.38 - 7.47 (m, 4H) 8.06 (s, 1H) 8.70 (s, 1H)

### 3-tert-Butyl-6-[(4-chlorophenyl)methyl]-8-(morpholin-4-yl)pyrido[2,3-e][1,2,4]triazolo[4,3-c]pyrimidin-5(6H)-one (Example 30)

In a microwave vial, a solution of 8-Bromo-3-tert-butyl-6-[(4-chlorophenyl)methyl]pyrido[2,3-e][1,2,4]triazolo[4,3-c]pyrimidin-5(6H)-one (Intermediate 62, 103 mg, 0.23 mmol) and morpholine (40.2 µL, 40.18 mg, 0.4612 mmol) in 4 mL of dioxane was purged with N₂. After 5 minutes, palladium (II) acetate (10.35 mg, 0.046 mmol), Xantphos (53.37 mg, 0.092 mmol) and cesium carbonate (150.3 mg, 0.4612 mmol) were added to the solution and the mixture was stirred in a microwave oven at 150°C for 2 hours. The reaction mixture was cooled to r.t., poured into water and extracted with EtOAc. The organic layer was washed with water, dried over anhydrous Na₂SO₄ and the solvent was evaporated to dryness. The crude was purified via automated flash chromatography (Biotage-Isolera Four coupled with Dalton 2000^{®}) using a SNAP25 Ultra cartridge, eluting with a gradient of EtOAc - PE from 8:2 to 10:0. The collected fractions gave 3.5 mg (yield: 3.4%) of the title compound.

HPLC purity = 97.4%

MS (ESI) m/z 453.31 [M + H]⁺

¹H NMR (400 MHz, CHLOROFORM-d) δ ppm 8.42 (d, 1H) 7.33 - 7.38 (m, 2H) 7.24 - 7.28 (m, 2H) 6.77 (d, 1H) 5.53 (s, 2H) 3.84 - 3.92 (m, 4H) 3.23-3.30 (m, 4H) 1.57 (s, 9H).

### Example 31

### 6-[(4-Bromophenyl)methyl]-3-tert-butyl-8-(morpholin-4-yl)pyrido[2,3-e][1,2,4]triazolo[4,3-c]pyrimidin-5(6H)-one

### Methyl 5-bromo-3-[(4-bromophenyl)methylamino]pyridine-2-carboxylate (Intermediate 63)

To a solution of methyl 5-bromo-3-fluoropyridine-2-carboxylate (500 mg, 2.14 mmol) in 10 mL of MeCN were added (4-bromophenyl)methanamine hydrochloride (523 mg, 2.35 mmol) and 0.93 mL of DIPEA (690 mg, 5.34 mmol). The solution was refluxed overnight. Then, the solvent was concentrated *in vacuo,* the residue was dissolved in EtOAc, washed with water, dried over anhydrous Na₂SO₄, filtered and concentrated under vacuum to obtain 600 mg of the title compound (yield: 56%) as a yellow solid, which was used in the next step without any further purification.

### 7-Bromo-1-[(4-bromophenyl)methyl]pyrido[3,2-d]pyrimidine-2,4-dione (Intermediate 64)

To a stirred solution of methyl 5-bromo-3-[(4-bromophenyl)methylamino]pyridine-2-carboxylate (Intermediate 63, 625 mg, 0.62 mmol) in 6 mL of DCM, was added dropwise trichloroacetyl isocyanate (0.22 mL, 353 mg, 1.87 mmol). The mixture was stirred at r.t. for 30 min, then the solvent was evaporated and the residue dissolved in 2 mL of MeOH. A solution of sodium methoxide 25% in MeOH (3.45 mL, 15 mmol) was added to the residue and the reaction was stirred at 60°C for 4 hours. The solvent was removed under vacuum. Then, the residue was diluted in water acidified until pH 4-5 and extracted with DCM. The organic layer was washed with brine, dried over anhydrous Na₂SO₄, filtered and concentrated under vacuum. During the evaporation a precipitate was formed, filtered and dried under vacuum to afford 250 mg of the desired compound (yield: 68 %) as off-white solid.

### 8-Bromo-6-[(4-bromophenyl)methyl]-3-tert-butylpyrido[2,3-e][1,2,4]triazolo[4,3-c]pyrimidin-5(6H)-one (Intermediate 65)

To a suspension of 7-bromo-1-[(4-bromophenyl)methyl]pyrido[3,2-d]pyrimidine-2,4-dione (Intermediate 64, 250 mg, 0.608 mmol) in 0.425 mL of DIPEA (314.4 mg, 2.433 mmol) was added POCl₃ (5.8 mL, 9.325 g, 60.82 mmol) and the resulting mixture was stirred at 60°C for 2 hours. Then the reaction was cooled at r.t. and the solvent was evaporated to dryness. The residue was rinsed and evaporated three times with dioxane to remove the residual POCl₃. The obtained crude was dissolved in 6 mL of dry 1,4-dioxane. Then, 2,2-dimethylpropanehydrazide (106 mg, 0.910 mmol) was added and the mixture was stirred for 2 hours at 50°C, then refluxed for 1 hour. The mixture was cooled down to r.t., diluted with aq. NaHCO₃ saturated solution and extracted with EtOAc. The organic phase was washed with brine, dried on anhydrous Na₂SO₄ and evaporated to dryness to obtain 800 mg of crude, which was purified by Biotage-Isolera^{®} system, (cartridge SNAP12 KP-C18-HS) eluting with (H₂O + 0.1% TFA) : (MeCN + 0.1% TFA) from 100:0 to 0:100. The fractions collected afforded 200 mg of the title compound (yield: 67%) as yellow solid.

¹H NMR (400 MHz, DMSO-d6) δ: 8.7 (s, 1H), 8.06 (s, 1H), 7.52 -7.54 (m, 2H), 7.37 - 7.39 (m, 2H), 5.47 (s, 2H), 1.56 (s, 9H).

### 6-[(4-Bromophenyl)methyl]-3-tert-butyl-8-(morpholin-4-yl)pyrido[2,3-e][1,2,4]triazolo[4,3-c]pyrimidin-5(6H)-one (Example 31)

In a vial equipped with a magnetic stirring were placed 8-bromo-6-[(4-bromophenyl)methyl]-3-*tert*-butylpyrido[2,3-*e*][1,2,4]triazolo[4,3-*c*]pyrimidin-5(6*H*)-one ( Intermediate 65, 50 mg, 0.100 mmol) and morpholine (13 µL, 0.150 mmol). Then, DABCO (25.12 mg, 0.220 mmol) and of 4,4'-di-*t*-butyl-2,2'-bipyridine)*bis*[3,5-difluoro-2-[5-trifluoromethyl-2-pyridinyl-kN)phenyl-kC]iridium(III) hexafluorophosphate (4.57 mg) followed by 203 µL of DMA. Afterwards, a solution of Nickel (II) chloride ethylene glycol dimethyl ether complex (1:2:1) (2.24 mg, 0.010 mmol) in 203 µL of DMA was added. The mixture was rigorously degassed. The vial was first filled with N₂, cooled at -78°C and degassed under vacuum, backfilled with N₂ and warmed to room temperature. This cycle was repeated 3 times. Then, the vial was sealed with a parafilm strip and subjected to 365 nm LED irradiation at the distance of 2 cm from 34 W blue LED lamp. The internal temperature upon irradiation reached approximately 55°C. The reaction was diluted with EtOAc and washed with water and brine. The organic phase was dried over anhydrous Na₂SO₄, filtered and evaporated to dryness. The crude was purified using a Biotage-Isolera^{®} purification system, cartridge SNAP KP-Sil 4g, eluting with PE : EtOAc in gradient from100:0 to 0:100. The fractions collected gave 3 mg of the title compound (yield: 5.9 %) as a yellow solid.

HPLC purity = 91.85 %

MS (ESI) m/z 497.23 [M + H]⁺

¹H NMR (400 MHz, DMSO-d6 + TFA) δ ppm 8.53 (br s, 1H), 7.52 (br d, J = 7.9 Hz, 2H), 7.37 (br d, J = 7.9 Hz, 2H), 7.03 (s, 1H), 5.55 (s, 2H), 3.73 (br s, 4H), 3.45 (br s, 4H), 1.55 (s, 9H).

### Example 32

### 6-(4-Chlorobenzyl)-3-(2-methoxypropan-2-yl)-8-(morpholin-4-yl)pyrido[2,3-e][1,2,4]triazolo[4,3-c]pyrimidin-5(6H)-one

### Methyl 2-methoxy-2-methyl-propanoate (Intermediate 79)

To a solution of 2-methoxy-2-methyl-propanoic acid (3 g, 25.396 mmol) in 30 mL of methanol was added sulfuric acid (2.03 mL, 3.736 g, 38.094 mmol) and the mixture was stirred at reflux for 8h. After cooling at room temperature, the mixture was used as it was in the next step.

MS (ESI) m/z 133.31 [M + H]⁺

### 2-Methoxy-2-methyl-propanehydrazide (Intermediate 80)

To a solution of methyl 2-methoxy-2-methyl-propanoate (Intermediate 79, 25.39 mmol) in 30 mL methanol in a microwave vial was added hydrazine hydrate (8.61 mL, 88 g, 177 mmol). The vial was sealed and the mixture was stirred under microwave irradiation at 100°C for 1 hour. After cooling at r.t., the solvent was removed under vacuum, water and DCM were added. The two phases were separated, the aqueous layer was extracted with DCM and the combined organic phases were dried over anhydrous Na₂SO₄, filtered and evaporated to afford 1.75 g (52.2% yield) of the title compound as a clear oil.

MS (ESI) m/z 133.10 [M + H]⁺

### 4-Chloro-1-[(4-chlorophenyl)methyl]-7-morpholino-pyrido[3,2-d]pyrimidin-2-one (Intermediate 66)

To a suspension of *1-[(4-chlorophenyl)methyl]-7-(morpholin-4-yl)pyrido[3,2-d]pyrimidine-2,4(1H,3H)-dione* (Intermediate 9, 105 mg, 0.2817 mmol) in 145 µL of DIPEA (109.2 mg, 0.845 mmol) was added POCl₃ (3 mL, 5 g, 32.7 mmol) and the resulting mixture was stirred at 50° C for 20 min. Then the reaction was cooled to room temperature. The solvent was evaporated and the crude rinsed with 1,4-dioxane and evaporated three times to remove the residual POCl₃. The obtained crude was used in the next step without further purification.

### N'-[(4Z)-1-[(4-chlorophenyl)methyl]-7-(morpholin-4-yl)-2-oxo-2,3-dihydropyrido[3, 2-d]pyrimidin-4(1H)-ylidene]-2-methoxy-2-methylpropanehydrazide (Intermediate 67)

To a solution of 4-chloro-1-[(4-chlorophenyl)methyl]-7-morpholino-pyrido[3,2-d]pyrimidin-2-one (Intermediate 66, 237 mg, 0.606 mmol) in 5 mL of anhydrous 1,4-dioxane was added 2-methoxy-2-methyl-propanehydrazide (Intermediate 80, 88 mg, 0.666 mmol) and the reaction mixture was stirred at r.t. for 1 hour. The reaction was poured into water and extracted with EtOAc. The organic phase was washed with water, dried over anhydrous Na₂SO₄ and the solvent was evaporated to dryness to yield mg 295 (91% yield) of the title compound used in the next step without further purification.

MS (ESI) m/z 487.17 [M + H]⁺

### 6-(4-Chlorobenzyl)-3-(2-methoxypropan-2-yl)-8-(morpholin-4-yl)pyrido[2,3-e][1,2,4]triazolo[4,3-c]pyrimidin-5(6H)-one (Example 32)

To a solution of N'-[(4Z)-1-[(4-chlorophenyl)methyl]-7-(morpholin-4-yl)-2-oxo-2,3-dihydropyrido[3,2-d]pyrimidin-4(1H)-ylidene]-2-methoxy-2-methylpropanehydrazide (Intermediate 67, 295 mg, 0.606 mmol) in 5 mL of anhydrous THF was added Burgess reagent ((methoxycarbonylsulfamoyl)triethylammonium hydroxide, inner salt, 216.5 mg, 0.908 mmol) and the reaction mixture was stirred at r.t. overnight. Then, it was poured into water and extracted with EtOAc. The organic phase was washed with water, dried over anhydrous Na₂SO₄, filtered and evaporated to dryness. The residue was purified via automated flash chromatography (Biotage Isolera Four coupled with Dalton 2000^{®}), SNAP25 Cartridge, eluting with EtOAc - MeOH gradient from 10:0 to 8:2. The collected fractions were concentrated and further purified by reverse phase using a SNAP30 KP-C18-HS cartridge, eluting with a mixture H₂O - ACN in gradient from 8:2 to 2:8. The collected fractions afforded 41 mg (14 % yield) of the title compound.

HPLC purity = 95%

MS (ESI) m/z 469.24 [M + H]⁺

¹H NMR (400 MHz, DMSO-d6) δ ppm 8.40 (d, 1H) 7.33 - 7.49 (m, 4H) 6.99 (d, 1H) 5.50 (s, 2H) 3.66 - 3.80 (m, 4H) 3.30 - 3.33 (m, 4H) 3.06 (s, 3H) 1.76 (s, 6H).

### Example 33

### 6-(4-Methoxybenzyl)-3-(2-methoxypropan-2-yl)-8-(morpholin-4-yl)pyrido[2,3-e][1,2,4]triazolo[4,3-c]pyrimidin-5(6H)-one

To a solution of 4-chloro-1-[(4-methoxyphenyl)methyl]-7-morpholino-pyrido[3,2-d]pyrimidin-2-one (Intermediate 58, 157 mg, 0.406 mmol) in 5 mL of anhydrous 1,4-dioxane was added 2-methoxy-2-methyl-propanehydrazide (53.64 mg, 0.406 mmol) and the mixture was stirred at room temperature for 30 min. The mixture was then heated at reflux for 12 hours. After cooling at room temperature, water and EtOAc were added and the two phases were separated. The aqueous layer was then extracted with EtOAc and the combined organic phases were dried over anhydrous Na₂SO₄, filtered and evaporated at reduced pressure. The crude residue was purified by Isolera One^{®} system, cartridge type SNAP25, using a gradient from EtOAc 100% to EtOAc : MeOH 80:20. The collected fractions were re-purified by Isolera One system, on a reverse phase cartridge SNAP30 KP-C18-HS, eluting with a gradient from NH₄HCO₃ buffer : ACN 85:15 to NH₄HCO₃ buffer : ACN 50:50. The collected fractions afforded 3 mg (1.6 % yield) of the title compound as light yellow solid.

HPLC purity = 94.4%

MS (ESI) m/z 465.32 [M + H]⁺

¹H NMR (400 MHz, CHLOROFORM-d) δ ppm 8.38 (s, 1H) 7.23 (br d, 2H) 6.90 (d, 2H) 6.85 (s, 1H) 5.42 (s, 2H) 3.85 - 3.95 (m, 4H) 3.81 (s, 3H) 3.23 - 3.30 (m, 4H) 3.22 (s, 3H) 1.94 (s, 6H).

### Example 34

### 8-(4-Acetylpiperazin-1-yl)-3-tert-butyl-6-[(4-chlorophenyl)methyl]pyrido[2,3-e][1,2,4]triazolo[4,3-c]pyrimidin-5(6H)-one

### tert-Butyl 4-(6-cyano-5-fluoro-3-pyridyl)piperazine-1-carboxylate (Intermediate 68)

To a solution of 3,5-difluoropyridine-2-carbonitrile (8 g, 57.106 mmol) in 20 mL of DMA were added 15.9 mL of TEA (11.557 g, 114.21 mmol) and tert-butyl piperazine-1-carboxylate (10.636 g, 57.106 mmol) and the mixture was stirred at room temperature. After 2 hours water was added (80 mL) and the white precipitate formed was filtered and washed with water (20 mL). The solid was dried in a oven at 40°C overnight to give 17.4 g of the title compound (yield: 99%) as white powder, which was used in the next step without any further purification.

MS (ESI) m/z 307.18 [M + H]⁺

### tert-Butyl 4-(6-carbamoyl-5-fluoro-3-pyridyl)piperazine-1-carboxylate (Intermediate 69)

To a solution of tert-butyl 4-(6-cyano-5-fluoro-3-pyridyl)piperazine-1-carboxylate (Intermediate 68, 17.05 g, 55.66 mmol) in 50 mL of DMSO were added NaOH 1N (33.40 mL, 33.40 mmol) and 30% hydrogen peroxide aqueous solution (6.823 mL, 7.574 g, 66.80 mmol). The mixture was stirred at r.t. for 3 hours. The so formed white precipitate was collected, washed with water and dried in a oven at 40°C to give 13.7 g of the title compound (yield: 76%) as white powder which was used in the next step without any further purification.

MS (ESI) m/z 325.24 [M + H]⁺

### tert-Butyl 4-[6-carbamoyl-5-[(4-chlorophenyl)methylamino]-3-pyridyl]piperazine-1-carboxylate (Intermediate 70)

To a solution of tert-butyl 4-(6-carbamoyl-5-fluoro-3-pyridyl)piperazine-1-carboxylate (Intermediate 69, 3.5 g, 11 mmol) in 12 mL of DMA were added 3.7 mL of N-ethyl-N-isopropyl-propan-2-amine (2.8 g, 22 mmol) and (4-chlorophenyl)methanamine (2.0 mL, 2.3 g, 16 mmol).). The mixture was heated at 160°C in a microwave oven for 2 hours. The solution was cooled and poured into water. A white precipitate was formed, filtered, washed with water and dried in a oven at 40°C, to give 3.6 g of the title compound (yield: 75%) as off-white solid, which was used in the next step without any further purification.

MS (ESI) m/z 446.14 [M + H]⁺

### tert-Butyl 4-(1-[(4-chlorophenyl)methyl]-2,4-dioxo-pyrido[3,2-d]pyrimidin-7-yl]piperazine-1-carboxylate (Intermediate 71)

To a solution cooled at 0-5°C of tert-butyl 4-[6-carbamoyl-5-[(4-chlorophenyl)methylamino]-3-pyridyl]piperazine-1-carboxylate (Intermediate 70, 3.67 g of 8.23 mmol) in 100 mL of dry DMF were added sodium hydride 60% oil dispersion (1.975 g, 49.38 mmol). The mixture was stirred 15 minutes at 0-5°C and then di(imidazol-1-yl)methanone (CDI, 8.007 g, 49.38 mmol) was added portion-wise in 15 minutes. The mixture was stirred at 0-5°C for 30 min, then warmed up to r.t. and stirred overnight. The reaction was cooled at 0-5°C, the pH was adjusted to 5 by adding acetic acid; then, water was added (200 mL). The formed yellow precipitate was filtered, dried in an oven at 50°C for 4 hours to give 4.18 g (97 % yield) of the desired compound as orange powder, which was used in the next step without further purification.

MS (ESI) m/z 472.27 [M + H]⁺

### tert-Butyl 4-[1-[(4-chlorophenyl)methyl]-2-oxo-4-thioxo-pyrido[3,2-d]pyrimidin-7-yl]piperazine-1-carboxylate (Intermediate 72)

To a solution of tert-butyl 4-[1-[(4-chlorophenyl)methyl]-2,4-dioxo-pyrido[3,2-d]pyrimidin-7-yl]piperazine-1-carboxylate (Intermediate 71, 4.18 g, 8.90 mmol) in 100 mL of pyridine, Lawesson's reagent (14.4 g, 35.60 mmol) was added and the mixture was stirred at 110°C for 8 hours. The mixture was cooled at 0-5°C and water (200 mL) was added. A yellow precipitate was formed, filtered and dried in a oven under vacuum to obtain 3.68 g of the title compound (yield: 82.9%) as yellow powder. The product was used in the next step without any further purification.

MS (ESI) m/z 488.21 [M + H]⁺

### 1-[(4-chlorophenyl)methyl]-7-(piperazin-1-yl)-4-sulfanylidene-3,4-dihydropyrido[3,2-d]pyrimidin-2(1H)-one hydrochloride (Intermediate 73)

To a solution cooled at 0-5°C of tert-butyl 4-[1-[(4-chlorophenyl)methyl]-2-oxo-4-thioxo-pyrido[3,2-d]pyrimidin-7-yl]piperazine-1-carboxylate (Intermediate 72, 3.68 g, 7.541 mmol) in 75 mL of DCM, 18.85 mL of a 2M hydrogen chloride solution in diethyl ether (37.70 mmol) were added and the mixture was stirred at r.t. overnight. The solvent was removed under reduced pressure. 3.2 g (99% yield) of the title compound were collected and used in the next step without any further purification.

MS (ESI) m/z 387.95 [M + H]⁺

### 7-(4-Acetylpiperazin-1-yl)-1-[(4-chlorophenyl)methyl]-4-thioxo-pyrido[3,2-d]pyrimidin-2-one (Intermediate 74)

To a suspension of 1-[(4-chlorophenyl)methyl]-7-(piperazin-1-yl)-4-sulfanylidene-3,4-dihydropyrido[3,2-d]pyrimidin-2(1H)-one hydrochloride (Intermediate 73, 3.2 g, 7.540 mmol) in 100 mL of DCM cooled at 0-5°C were added 5.25 mL of TEA (3.815 g, 37.70 mmol). The mixture was stirred 15 minutes at 0-5°C, then keeping the temperature at 0-5°C, a solution of acetyl chloride (0.536 mL, 0.592 g, 7.54 mmol) in 10 mL of DCM was dripped. The reaction was warmed up to room temperature and stirred overnight. Then water and 100 ml of DCM were added. and the formed precipitate was filtered. The two phases were separated and the organic layer was washed with brine, dried over anhydrous Na₂SO₄, filtered and evaporated. The residue was combined with the precipitate to give 3.13 g of the title compound (yield: 96.6%) as light-yellow powder which was used in the next step without any further purification.

MS (ESI) m/z 430.04 [M + H]⁺

### 8-(4-Acetylpiperazin-1-yl)-3-tert-butyl-6-[(4-chlorophenyl)methyl]pyrido[2,3-e][1,2,4]triazolo[4,3-c]pyrimidin-5(6H)-one (Example 34)

To a solution of 7-(4-acetylpiperazin-1-yl)-1-[(4-chlorophenyl)methyl]-4-thioxo-pyrido[3,2-d]pyrimidin-2-one (Intermediate 74, 3.13 g, 7.281 mmol) in 100 mL of pyridine, 2,2-dimethylpropanehydrazide (2.537 g, 21.84 mmol) was added and the mixture was stirred at 110°C for 10 hours. After cooling at room temperature, the solvent was removed under vacuum, water and EtOAc were added, and the two phases were separated. The organic layer was washed with brine, dried over anhydrous Na₂SO₄, filtered and evaporated to dryness.

The crude material (3.5 g) was dissolved in 100 mL of THF and of Burgess reagent (5.2 g, 21.84 mmol) was added. The mixture was stirred at r.t. overnight. Then, additional Burgess reagent (5.2 g, 21.84 mmol) was added and the mixture was stirred at r.t. for 2h. The solvent was removed under reduced pressure and the crude was dissolved in EtOAc was washed with brine. The organic layer was dried over anhydrous Na₂SO₄, filtered and evaporated. The crude residue was purified by Biotage Selekt^{®} purification system, using a cartridge type SNAP50, and eluting with a gradient from EtOAc 100% to EtOAc : MeOH 80:20. The collected fractions gave 714 mg (yield: 19.8%) of the title compound as light brown powder.

HPLC purity 92.7%

MS (ESI) m/z 494.41 [M + H]⁺

¹H NMR (400 MHz, DMSO-d6) δ ppm 8.39 (d, 1H) 7.32 - 7.51 (m, 4H) 6.99 (d, 1H) 5.51 (s, 2H) 3.51 - 3.68 (m, 4H) 3.31 - 3.47 (m, 4H) 2.04 (s, 3H) 1.57 (s, 9H).

### Example 35

### 3-tert-Butyl-6-[(4-methoxyphenyl)methyl]-8-(piperazin-1-yl)pyrido[2,3-e][1,2,4]triazolo[4,3-c]pyrimidin-5(6H)-one

### Methyl 5-bromo-3-[(4-methoxyphenyl)methylamino]pyridine-2-carboxylate (Intermediate 75)

To a solution of (4-methoxyphenyl)methanamine (1.12 mL, 1.172 g, 8.55 mmol) in 42 mL of MeCN was added methyl 5-bromo-3-fluoropyridine-2-carboxylate (2 g, 8.55 mmol) and the mixture was stirred at reflux for 24 hours. The solvent was partially removed and the solid filtered to give 1.9 g of the title compound (yield: 63.3%) which was used in the next step without any further purification.

### 7-Bromo-1-[(4-methoxyphenyl)methyl]pyrido[3,2-d]pyrimidine-2,4(1H,3H)-dione (Intermediate 76)

To a stirred solution of methyl 5-bromo-3-[(4-chlorophenyl)methylamino]pyridine-2-carboxylate (Intermediate 75, 3 g, 8.44 mmol ) in 16 mL of DCM was added dropwise trichloroacetyl isocyanate (2.0 mL, 3.16 g, 16.87 mmol) and the reaction was stirred at r.t. for 1h. Then the solvent was evaporated and 23.15 mL of a 25% sodium methoxide solution in MeOH (101.23 mmol) were added and the reaction was stirred at 70°C overnight. After cooling, the mixture was diluted with water and acidified at pH 5-6 using 3N HCl. The precipitate was isolated by filtration and dried to afford 2.5 g (56.1%) of title compound as off-white powder.

### 8-Bromo-3-(tert-butyl)-6-(4-methoxybenzyl)pyrido[2,3-e][1,2,4]triazolo[4,3-c]pyrimidin-5(6H)-one (Intermediate 77)

In a round bottom flask phosphoryl chloride (29 mL, 46.4 g, 302.62 mmol) was mixed with 2.12 mL of DIPEA (1.565 g,12.11 mmol) and 7-bromo-1-[(4-methoxyphenyl)methyl]pyrido[3,2-*d*]pyrimidine-2,4(1*H*,3*H*)-dione (Intermediate 76, 1.1 g, 3.04 mmol). The mixture was stirred at 80°C for 3h. Then it was evaporated to dryness (using N₂ to remove vacuum from the rotavapor). The crude was then dissolved in 27 mL of dry 1,4-dioxane and 2,2-dimethylpropanehydrazide (0.53 g, 4.56 mmol) was added and the mixture stirred at 50°C for 45 min and then refluxed for 30 min. The mixture was cooled down, diluted with aq. NaHCOs saturated solution, and extracted with EtOAc. Organic layer was washed with brine, dried over anhydrous Na₂SO₄, filtered and evaporated to obtain 2.5 g of crude that was purified twice by Biotage-Isolera^{®}, Silicycle SiliaSep 120gHP cartridge eluting with PE : EtOAc from 80:20 to 0:100. The collected fractions gave 700 mg (41.7% yield) of the title compound.

In the same reaction were also isolated 150 mg (11%) of the thermodynamic product 8-bromo-2-(tert-butyl)-6-(4-methoxybenzyl)pyrido[2,3-e][1,2,4]triazolo[1,5-c]pyrimidin-5(6H)-one.

### tert-Butyl 4-(3-(tert-butyl)-6-(4-methoxybenzyl)-5-oxo-5,6- dihydropyrido[2,3-e][1,2,4]triazolo[4,3-c]pyrimidin-8-yl)piperazine-1-carboxylate (Intermediate 78)

A vial equipped with a magnetic stirring bar was charged with 8-bromo-3-(tert-butyl)-6-(4-methoxybenzyl)pyrido[2,3-e][1,2,4]triazolo[4,3-c]pyrimidin-5(6H)-one (Intermediate 77, 120 mg, 0.270 mmol), tert-butyl piperazine-1-carboxylate (75.8 mg, 0.410 mmol), DABCO (54.78 mg, 0.490 mmol), and 4,4'-Di-t-butyl-2,2'-bipyridine)bis[3,5-difluoro-2-[5-trifluoromethyl-2-pyridinyl-kN)phenyl-kC]iridium(III) hexafluorophosphate (6.09 mg, 0.010 mmol) then dissolved in 0.45 mL of DMA and a solution of Nickel(2+) chloride-1,2-dimethoxyethane (1:2:1) (2.98 mg, 0.010 mmol) dissolved in 0.45 mL of DMA was added. The mixture was rigorously degassed 3 times according to the following procedure: the vial is first filled with N₂, cooled at -78°C, degassed under vacuum, backfilled with N₂ and warmed to room temperature. Then, the vial was sealed with a parafilm strip and subjected to 365 nm LED irradiation (2 cm distance from 34 W blue LED lamp) for 1 hour. The internal temperature upon irradiation raised approximately to 55°C. The reaction was diluted with EtOAc and washed with water and brine. The organic phase was dried over anhydrous Na₂SO₄, filtered and concentrated *in vacuo* to afford a residue that was used in the next step without any further purification.

### 3-tert-Butyl-6-[(4-methoxyphenyl)methyl]-8-(piperazin-1-yl)pyrido[2,3-e][1,2,4]triazolo[4,3-c]pyrimidin-5(6H)-one (Example 35)

To a solution of tert-butyl 4-(3-(tert-butyl)-6-(4-methoxybenzyl)-5-oxo-5,6-dihydropyrido[2,3-e][1,2,4]triazolo[4,3-c]pyrimidin-8-yl)piperazine-1-carboxylate (Intermediate 78, 140 mg, 0.260 mmol) in 0.8 mL of DCM at r.t. was added TFA (0.2 mL, 29.2 mg, 0.260 mmol) and the solution was stirred 4h at r.t.. The solvent was removed under vacuum and the residue was purified by Biotage-Isolera^{®} system, SNAP KP-C18-HS 12 g cartridge, eluting with H₂O+0.1% TFA : MeCN+0.1% TFA from 100:0 to 0:100. The collected fractions gave 100 mg (yield: 87%) of the title compound as a brown solid.

HPLC purity = 91.56 %

MS (ESI) m/z 448.06 [M +H]⁺

¹H NMR (400 MHz, DMSO-d6 +TFA) δ = 8.89 (br d, J = 1.5 Hz, 2H), 8.52 (br s, 1H), 7.35 (br d, J = 7.9 Hz, 2H), 7.17 (br s, 1H), 6.89 (br d, J = 8.1 Hz, 2H), 5.50 (br s, 2H), 3.71 (s, 3H), 3.68 (s, 4H), 3.25 (s, 4H), 1.57 (s, 9H).

### Example 36

### 8-(4-Acetylpiperazin-1-yl)-3-tert-butyl-6-[(4-methoxyphenyl)methyl]pyrido[2,3-e][1,2,4]triazolo[4,3-c]pyrimidin-5(6H)-one

To a solution of 3-tert-butyl-6-[(4-methoxyphenyl)methyl]-8-(piperazin-1-yl)pyrido[2,3-e][1,2,4]triazolo[4,3-c]pyrimidin-5(6H)-one (Example 35, 80 mg, 0.180 mmol) in 0.5 mL of DCM and 37.37 µL of TEA (0.270 mmol) was added acetyl chloride (12.76 µL, 0.180 mmol). The solution was stirred at room temperature for 1h. The reaction was diluted with DCM and washed with water and brine. The organic phase was dried over anhydrous Na₂SO₄, filtered and evaporated *in vacuo* to afford a residue that was purified by Biotage-Isolera^{®} system, cartridge SNAP KP-C18-HS 4g, eluting using H₂O+0.1% TFA - MeCN+0.1% TFA in gradient from 100:0 to 0:100. The collected fractions gave 30 mg (34% yield) of the title compound.

HPLC purity = 90.3%

MS (ESI) m/z 490.33 [M + H]⁺

¹H NMR (400 MHz, DMSO-d6) δ = 8.51 (s, 1H), 7.37 (br d, J = 8.1 Hz, 2H), 7.07 (br s, 1H), 6.90 (br d, J = 8.3 Hz, 2H), 5.51 (br s, 2H), 3.70 (s, 3H), 3.59 (br s, 6H), 3.51 (br s, 2H), 2.04 (s, 3H), 1.57 (s, 9H).

### Example 37

### 3-tert-Butyl-6-[(4-methoxyphenyl)methyl]-8-(4-methoxypiperidin-1-yl)pyrido[2,3-e][1,2,4]triazolo[4,3-c]pyrimidin-5(6H)-one

In a vial equipped with a magnetic stirring bar 8-bromo-3-(tert-butyl)-6-(4-methoxybenzyl)pyrido[2,3-e][1,2,4]triazolo[4,3-c]pyrimidin-5(6H)-one (Intermediate 77, 70 mg, 0.160 mmol), 4,4'-Di-t-butyl-2,2'-bipyridine)bis[3,5-difluoro-2-[5-trifluoromethyl-2-pyridinylkN)phenyl-kC]iridium(III) hexafluorophosphate (3.55 mg), DABCO (31.95 mg, 0.280 mmol ) and 4-methoxypiperidine (29.4 µLµL, 27.34mg, 0.240 mmol) were dissolved in 0.5 mL of DMA. Then, Nickel (2+) chloride - 1,2-dimethoxyethane (1:2:1) (6.95 mg, 0.030 mmol) was added as a solution in 0.2 mL of DMA. The vial was then placed under N₂, cooled at -78°C and degassed, backfilled with N₂ and heated up slowly to r.t. The cycle was repeated 3 times, and then the reaction mixture under stirring was irradiated (blue light) for 3h. The reaction mixture was then diluted with water (20 mL) and extracted with 20 mL of EtOAc in 3 portions. The combined organic layers were dried over anhydrous Na₂SO₄, filtered and concentrated *in vacuo* to obtain a crude which was purified by reverse phase chromatography to yield 37 mg of desired product with a UPLC purity around 76%. This material was then further purified by FractionLynx recovering 13.8 mg as powder, containing the title product in presence of ~17% of the preferred thermodynamic isomer.

HPLC purity > 99% as a 83:17 mixture of the two isomer

MS (ESI) m/z 477.28 [M + H]⁺

¹H NMR (400 MHz, DMSO-d6) δ = 8.45 - 8.35 (m, 1H), 7.34 (br d, J = 7.5 Hz, 2H), 7.10 - 6.96 (m, 1H), 6.89 (br d, J = 7.5 Hz, 2H), 5.56 - 5.37 (m, 2H), 3.70 (br s, 5H), 3.41 (br s, 1H), 3.26 (s, 3H), 3.14 (br t, J = 9.8 Hz, 2H), 1.85 (br s, 2H), 1.57 (br s, 7H), 1.42 (br s, 4H).

### Example 38

### 3-tert-Butyl-6-[(4-methoxyphenyl)methyl]-8-(piperidin-1-yl)pyrido[2,3-e][1,2,4]triazolo[4,3-c]pyrimidin-5(6H)-one

In a vial equipped with a magnetic stirring 8-bromo-3-(tert-butyl)-6-(4-methoxybenzyl)pyrido[2,3-e][1,2,4]triazolo[4,3-c]pyrimidin-5(6H)-one (intermediate 77, 70 mg, 0.160 mmol), 4,4'-Di-t-butyl-2,2'-bipyridine)bis[3,5-difluoro-2-[5-trifluoromethyl-2-pyridinyl-kN)phenyl-kC]iridium(III) hexafluorophosphate (3.55 mg), DABCO (39.05 mg, 0.350 mmol) and Nickel(2+) chloride -1,2-dimethoxyethane (1:2:1) (1.74 mg, 0.010 mmol) were dissolved in 0.05 mL of DMA. Then piperidine (23.5 µL, 0.240 mmol) was added as a solution in 0.05 mL of DMA. The vial was then placed under N₂, cooled at -78°C and degassed, backfilled with N₂ and heated up slowly to r.t. This cycle was repeated 3 times, then the reaction mixture irradiated (blue light) while stirring for 3h. The reaction mixture was poured into water (20 mL) and extracted with EtOAc (60 mL). The combined organic layers were washed with brine (10 mL), dried over anhydrous Na₂SO₄, filtered and evaporated to get a crude which was purified by flash chromatography (25 g SiO2) eluting in gradient using PE : EtOAc from 50:50 to 0:100. The collected fractions gave 50 mg of a mixture of the title compound and the de-halogenated by-product. The mixture was further purified by reverse phase chromatography cartridge (5.5 g) eluting in gradient using as eluant H₂O 0.1% TFA - ACN 0.1% TFA from 100:0 to 0:100. The collected fractions gave 11.2 mg (13% yield) of the title compound.

HPLC purity = 97.4 %

MS (ESI) m/z 447.40 [M + H]⁺

¹H NMR (400 MHz, DMSO-d6) δ ppm 8.37 (br s, 1H), 7.39 - 7.29 (m, J = 8.1 Hz, 2H), 6.99 (br s, 1H), 6.94 - 6.85 (m, J = 8.1 Hz, 2H), 5.45 (br s, 2H), 3.71 (s, 3H), 3.37 (br s, 4H), 1.57 (s, 15H).

### Example 39

### 6-[(4-Chlorophenyl)methyl]-8-(4-oxopiperidin-1-yl)-3-(propan-2-yl)pyrido[2,3-e][1,2,4]triazolo[4,3-c]pyrimidin-5(6H)-one

### 8-Bromo-6-[(4-chlorophenyl)methyl]-3-(propan-2-yl)pyrido[2,3-e][1,2,4]triazolo[4,3-c]pyrimidin-5(6H)-one (Intermediate 81)

To a solution of 7-bromo-4-chloro-1-[(4-chlorophenyl)methyl]pyrido[3,2-d]pyrimidin-2-one (Intermediate 61, 250 mg, 0.6494 mmol) in 6 mL of anhydrous 1,4-dioxane, 2-methylpropanehydrazide (132.6 mg, 1.299 mmol) was added and the reaction mixture was stirred at 60°C for 2 hours. Then, the mixture was cooled to r.t., poured into water and extracted with EtOAc. The organic phase was washed with water, dried over anhydrous Na₂SO₄ and the solvent was evaporated to dryness to give a crude which was purified via automated flash chromatography (Biotage Isolera Four coupled with Dalton 2000^{®}), SNAP25 Cartridge, eluting with a EtOAc : PE gradient from 1:1 to 9:1 to afford 216 mg (yield: 77%) of the title compound.

MS (ESI) m/z 432.14 [M + H]⁺

### 6-[(4-Chlorophenyl)methyl]-8-(4-oxopiperidin-1-yl)-3-(propan-2-yl)pyrido[2,3-e][1,2,4]triazolo[4,3-c]pyrimidin-5(6H)-one (Example 39)

In a microwave vial, a solution of 8-bromo-6-[(4-chlorophenyl)methyl]-3-(propan-2-yl)pyrido[2,3-e][1,2,4]triazolo[4,3-c]pyrimidin-5(6H)-one (Intermediate 81, 135 mg, 0.312 mmol) and piperidin-4-one hydrochloride (71.89 mg, 0.4680 mmol) in 4 mL of anhydrous 1,4-dioxane was purged with N₂. After 5 minutes, palladium(II) acetate (14.01 mg, 0.0624 mmol), XantPhos (72.21 mg, 0.125 mmol) and caesium carbonate (305.0 mg, 0.9360 mmol) were added and the mixture was stirred in a microwave oven at 80°C for 3.5 hours. Then the mixture was cooled at r.t., poured into water and extracted with EtOAc. The organic layer was washed with water, dried over anhydrous Na₂SO₄ and the solvent was evaporated to dryness give a crude which was purified via automated flash chromatography (Biotage Isolera Four coupled with Dalton 2000^{®}), SNAP10 Cartridge, eluting in gradient with EtOAc -MeOH from 10:0 to 9:1 to give 10 mg (yield: 7%) of the title compound.

HPLC purity = 84.1%

MS (ESI) m/z 451.21 [M + H]⁺

¹H NMR (400 MHz, CHLOROFORM-d) δ ppm 8.42 (d, 1H) 7.33 - 7.39 (m, 2H) 7.26 (d, 2H) 6.77 (d, 1H) 5.54 (s, 2H) 3.72 (t, 4H) 3.29 - 3.45 (m, 1H) 2.61 (t, 4H) 1.51 (d, 6H).

### Example 40

### 8-(4-Aminopiperidin-1-yl)-3-tert-butyl-6-[(4-methoxyphenyl)methyl]pyrido[2,3-e][1,2,4]triazolo[4,3-c]pyrimidin-5(6H)-one

### 5-(1,4-Dioxa-8-azaspiro[4.5]decan-8-yl)-3-fluoro-pyridine-2-carbonitrile (Intermediate 82)

To a solution of 3,5-difluoropyridine-2-carbonitrile (1.5 g, 10.707 mmol) in 20 mL of DMA was added 1,4-dioxa-8-azaspiro[4.5]decane (1.373 mL, 1.533 g, 10.707 mmol) followed by 2.98 mL of TEA (2.1670 g, 21.415 mmol). The mixture was heated at 100°C for 3h. After cooling at r.t. water and EtOAc were added. The two phases were separated, the organic layer was washed with water, dried over anhydrous Na₂SO₄, filtered and evaporated *in vacuo.* The crude residue was purified by Biotage Selekt^{®} system, cartridge type SNAP50, eluting with PE : EtOAc in gradient from 8:2 to 3:7. The collected fractions gave 2.19 g (yield: 77.7%) of the title compound as a white powder.

MS (ESI) m/z 264.34 [M + H]⁺

### 5-(1,4-Dioxa-8-azaspiro[4.5]decan-8-yl)-3-[(4-methoxyphenyl)methylamino]pyridine-2-carbonitrile (Intermediate 83)

To a solution of 5-(1,4-dioxa-8-azaspiro[4.5]decan-8-yl)-3-fluoro-pyridine-2-carbonitrile (Intermediate 82, 2.19 g, 8.318 mmol) in 10 mL of NMP in a MW vial, (4-methoxyphenyl)methanamine (1.192 mL, 1.255 g, 9.149 mmol) and 1.71 of mL DIPEA (1.29 g, 9.981 mmol) were added. The mixture was stirred under MW irradiation at 130°C for 2.5 h. Then, additional 0.5 equivalents of (4-methoxyphenyl)methanamine were added and the mixture heated at 130°C under microwave irradiation for further 2h. After cooling at r.t., water and EtOAc were added, and the two phases were separated. The organic layer was washed with water and brine, dried over anhydrous Na₂SO₄, filtered and evaporated *in vacuo.* The crude residue was purified by means of a Biotage Isolera One^{®} system, cartridge type SNAP100, using a gradient from PE : EtOAc 75:25 to 30:70. The collected fractions gave 2.77 g (yield: 87.5%) of title compound as reddish powder.

MS (ESI) m/z 381.21 [M + H]⁺

### 5-(1,4-Dioxa-8-azaspiro[4.5]decan-8-yl)-3-[(4-methoxyphenyl)methylamino]pyridine-2-carboxamide (Intermediate 84)

To a solution cooled at 0-5°C of 5-(1,4-dioxa-8-azaspiro[4.5]decan-8-yl)-3-[(4-methoxyphenyl)methylamino]pyridine-2-carbonitrile (Intermediate 83, 2.77 g, 7.282 mmol) in 10 mL of DMSO were added 4.37 mL of a 1N NaOH aq. solution (4.37 mmol) and 30% hydrogen peroxide aq. solution (0.8926 mL, 990.8 mg, 8.738 mmol). The mixture was stirred at room temperature overnight. Then water and EtOAc were added to the DMSO and the two phases were separated. The organic layer was washed with water and brine, dried over anhydrous Na₂SO₄, filtered and evaporated *in vacuo* to obtain 2.7 g (yield: 93%) of the title compound as light-yellow powder, which was used in the next step without any further purification.

MS (ESI) m/z 399.23 [M + H]⁺

### 7-(1,4-Dioxa-8-azaspiro[4.5]decan-8-yl)-1-[(4-methoxyphenyl)methyl]pyrido[3,2-d]pyrimidine-2,4-dione (Intermediate 85)

To a solution of 5-(1,4-dioxa-8-azaspiro[4.5]decan-8-yl)-3-[(4-methoxyphenyl)methylamino]pyridine-2-carboxamide (Intermediate 84, 2.77 g, 6.775 mmol) in 50 mL of dry DMF stirred under nitrogen atmosphere at 0-5°C, 60% sodium hydride oil dispersion (271 mg, 6.775 mmol) was added and the mixture was stirred for 15 minutes. Then ethyl chloroformate (3.239 mL, 3676 mg, 33.88 mmol) was added dropwise. The reaction mixture was stirred at room temperature for 1h, then heated at 70° C for 6h to obtain the intermediate carbamate with mass [M+H]⁺ = 471 as main product. The mixture was cooled down to 0-5°C and two additional equivalents of 60% sodium hydride oil dispersion were added in two portions, refluxing for 8 h after each addition. The mixture was cooled at 0-5°C, the pH was cautiously adjusted at around 4-5 with glacial acetic acid, then water was slowly added. The aqueous layer was extracted with DCM, the combined organic phases were dried over anhydrous Na₂SO₄, filtered and evaporated. The crude residue was purified by means of a Biotage Isolera Four coupled with Dalton 2000^{®}, cartridge type SNAP100, using a gradient from EtOAc : MeOH 10:0 to 8:2. The collected fractions gave 570 mg (yield: 19.8%) of the title compound as light brown powder.

MS (ESI) m/z 425.01 [M + H]⁺

### 4-Chloro-7-(1,4-dioxa-8-azaspiro[4.5]decan-8-yl)-1-[(4-methoxyphenyl)methyl]pyrido[3,2-d]pyrimidin-2-one (Intermediate 86)

Under nitrogen atmosphere, a solution of 7-(1,4-dioxa-8-azaspiro[4.5]decan-8-yl)-1-[(4-methoxyphenyl)methyl]pyrido[3,2-d]pyrimidine-2,4-dione (Intermediate 85, 200 mg, 0.47 mmol), in 0.242 mL of DIPEA (182.7 mg, 1.414 mmol) and POCl₃ (3.03 mL, 5.06 mg, 32.99 mmol) was stirred at 50°C for 2h. After cooling at r.t., the reaction was concentrated under vacuum, and the residue was diluted with 1,4 dioxane. The mixture was evaporated *in vacuo* three times, each time taking up with 1,4-dioxane. The crude (208 mg) containing the title compound as dark thick oil, was used in the next step without any further purification.

MS (ESI) m/z 438.93 [M + H]⁺ (quenched with methanol and detected as methoxy derivative)

### 3-tert-Butyl-8-(1,4-dioxa-8-azaspiro[4.5]decan-8-yl)-6-[(4-methoxyphenyl)methyl]pyrido[2,3-e][1,2,4]triazolo[4,3-c]pyrimidin-5(6H)-one (Intermediate 87)

To a solution of crude 4-chloro-7-(1,4-dioxa-8-azaspiro[4.5]decan-8-yl)-1-[(4-methoxyphenyl)methyl]pyrido[3,2-d]pyrimidin-2-one (Intermediate 86, 208 mg, 0.4696 mmol) in 5 mL of anhydrous 1,4-dioxane, 2,2-dimethylpropanehydrazide (38.19 mg, 0.3287 mmol) was added and the mixture was stirred at room temperature for 30 min. The mixture was then heated at reflux for 5 hours. After cooling at room temperature, water and EtOAc were added and the two phases separated. The aqueous layer was then extracted with EtOAc and the combined organic phases were dried over anhydrous Na₂SO₄, filtered and evaporated. The crude residue was purified by means of a Biotage Isolera One^{®}, cartridge type SNAP25, using a gradient from EtOAc : MeOH 10:0 to 8:2. The collected fractions gave 52 mg (yield: 21.9%) of the title compound as yellow oil.

MS (ESI) m/z 504.94 [M + H]⁺

### 3-tert-Butyl-6-[(4-methoxyphenyl)methyl]-8-(4-oxopiperidin-1-yl)pyrido[2,3-e][1,2,4]triazolo[4,3-c]pyrimidin-5(6H)-one (Intermediate 88)

To a solution of 3-tert-butyl-8-(1,4-dioxa-8-azaspiro[4.5]decan-8-yl)-6-[(4-methoxyphenyl)methyl]pyrido[2,3-e][1,2,4]triazolo[4,3-c]pyrimidin-5(6H)-one (Intermediate 87, 60 mg, 0.1189 mmol) in 2 mL of 1,4-dioxane, 2 mL of 6N aqueous HCl solution were added and the mixture was stirred at room temperature overnight. Then it was basified with potassium carbonate, and the aqueous layer was extracted with EtOAc. The organic layer was dried over anhydrous Na₂SO₄, filtered and evaporated. The crude residue was purified by means of a Biotage Selekt^{®} instrument, cartridge type SNAP10, using as eluent EtOAc - MeOH in gradient from 100:0 to 85:15. The collected fractions gave 14 mg (25.6% yield) of the title compound as pale-yellow oil.

MS (ESI) m/z 461.06 [M + H]⁺

### 8-(4-Aminopiperidin-1-yl)-3-tert-butyl-6-[(4-methoxyphenyl)methyl]pyrido[2,3-e][1,2,4]triazolo[4,3-c]pyrimidin-5(6H)-one (Example 40)

To a solution of 3-tert-butyl-6-[(4-methoxyphenyl)methyl]-8-(4-oxopiperidin-1-yl)pyrido[2,3-e][1,2,4]triazolo[4,3-c]pyrimidin-5(6H)-one (Intermediate 88, 14 mg, 0.0304 mmol) in 1 mL of MeOH were added ammonium acetate (23.43 mg, 0.3040 mmol) followed by sodium cyanoborohydride (2.866 mg, 0.0456 mmol) and the resulting mixture was stirred at r.t. overnight. The solvent was concentrated *in vacuo,* water and DCM were added, the two phases were separated. The aqueous layer was extracted with DCM (3x), and the combined organic phases were dried over anhydrous Na₂SO₄, filtered and evaporated. The crude residue was purified by means of a Biotage Selekt^{®} system, cartridge type SNAP10, using a gradient from DCM : 1.4 N ammonia sol. in MeOH 10:0 to 5:5. The collected fractions gave 2 mg (14.2% yield) of the title compound as off-white powder.

HPLC purity = 80.5%

MS (ESI) m/z 462.47 [M + H]⁺

¹H NMR (400 MHz, CHLOROFORM-d) δ ppm 8.47 - 8.84 (m, 2H) 8.08 - 8.47 (m, 1H) 7.21 (br d, 1H) 6.85 - 6.98 (m, 1H) 6.65 - 6.85 (m, 2H) 5.14 - 5.53 (m, 1H) 3.75 - 4.08 (m, 2H) 3.48 - 3.75 (m, 4H) 2.84 - 3.07 (m, 1H) 1.88 - 2.44 (m, 5H) 1.69 (br s, 9H) 1.62 (br s, 10H)

### Example 41

### 3-tert-Butyl-6-[(4-chlorophenyl)methyl]-8-[4-(hydroxymethyl)piperidin-1-yl]pyrido[2,3-e][1,2,4]triazolo[4,3-c]pyrimidin-5(6H)-one

In a vial equipped with a magnetic stirring bar of 8-bromo-3-(tert-butyl)-6-(4-chlorobenzyl)pyrido[2,3-e][1,2,4]triazolo[4,3-c]pyrimidin-5(6H)-one (Intermediate 62, 50 mg, 0.110 mmol), 4,4'-Di-t-butyl-2,2'-bipyridine)bis[3,5-difluoro-2-[5-trifluoromethyl-2-pyridinyl-kN)phenyl-kC]iridium(III) hexafluorophosphate (2.51 mg, cat.), DABCO (22.6 mg, 0.200 mmol) and 4-piperidylmethanol (20.35 mg, 0.170 mmol) were diluted in 0.25 mL DMA. Then 1.23 mg of Nickel(2+) chloride - 1,2-dimethoxyethane (1:2:1) (0.010 mmol) were added as a solution in 0.25 mL of DMA. The vial was then placed under N₂, cooled at -78°C and degassed, backfilled with N₂ and heated up slowly to r.t. The degassing cycle was repeated 3 times, then the reaction mixture under stirring was irradiated (blue light, 365 nm LED, 2 cm distance, 34 W, no fan cooling) for 3h. The crude was diluted with EtOAc washed with water and dried over anhydrous Na₂SO₄. The organic layer was filtered, and solvent concentrated *in vacuo.* This material was then further purified in reverse phase by FractionLynx. The collected fractions were basified (pH ca 8) with DIPEA and the solvent was removed. Then the residual water was then extracted with EtOAc, dried over anhydrous Na₂SO₄, filtered and solvent removed *in vacuo.* The product was solved in DCM and filtered through a 100 mg C18 cartridge eluting with 6 column volumes of water and 2 column volumes of MeCN, to remove DIPEA and concentrated *in vacuo.* The final product was finally dissolved in MeCN/water and lyophilized to obtain 1.36 mg (2.7%) of the title compound.

HPLC purity = 97%

MS (ESI) m/z 477 [M + H]⁺

¹H NMR (400 MHz, DMSO-d6) δ ppm 1.10 (br d, J=12.06 Hz, 2H) 1.56 (s, 10H) 1.70 (br d, J=12.93 Hz, 2H) 2.82 (br t, J=12.50 Hz, 2H) 3.24 (br t, J=5.48 Hz, 2H) 3.91 (br d, J=12.28 Hz, 2H) 4.47 (br t, J=4.93 Hz, 1H) 5.50 (s, 2H) 6.91 (s, 1H) 7.41 (q, J=8.33 Hz, 4H) 8.36 (s, 1H).

### Example 42

### 3-tert-Butyl-6-[(4-fluoro-3-methoxyphenyl)methyl]-8-(morpholin-4-yl)pyrido[2,3-e][1,2,4]triazolo[4,3-c]pyrimidin-5(6H)-one

### Methyl 5-bromo-3-[(4-fluoro-3-methoxy-phenyl)methylamino]pyridine-2-carboxylate (Intermediate 89)

To a solution of methyl 3-amino-5-bromo-pyridine-2-carboxylate (520 mg, 2.25 mmol), 4-fluoro-3-methoxy-benzaldehyde (694 mg of 4.50 mmol)) and acetic acid (0.257 mL, 270.3 mg, 4.501 mmol) in 20 mL of DCM, was added of sodium triacetoxy borohydride (1.43 g, 6.7518 mmol) in three portions, each one after 8 hours of stirring at r.t. Then, the mixture was washed with water, dried over anhydrous Na₂SO₄ and the solvent was evaporated to dryness. The obtained crude was purified via automated flash chromatography (Biotage Isolera Four coupled with Dalton 2000^{®}), SNAP25 cartridge, eluting with EtOAc : PE in gradient from 1:9 to 1:1 to afford 800 mg (yield: 96%) of the title compound.

MS (ESI) m/z 369.28 [M + H]⁺

### 7-bromo-1-[(4-fluoro-3-methoxyphenyl)methyl]pyrido[3,2-d]pyrimidine-2,4(1H,3H)-dione (Intermediate 90)

To a stirred solution of methyl 5-bromo-3-[(4-fluoro-3-methoxyphenyl)methylamino]pyridine-2-carboxylate (Intermediate 89, 800 mg, 2.167 mmol) in 20 mL of DCM, 2,2,2-trichloroacetyl isocyanate (0.7746 mL, 1.225 g, 6.501 mmol) was added and the reaction mixture was stirred at r.t. overnight. The solvent was evaporated and 4.95 mL of 25% sodium methoxide solution in methanol (4.682 g, 21.67 mmol) were added. The suspension was heated at 60°C for 4h and cooled to r.t. Water and aqueous 2N HCl solution were added till pH = 5 and the mixture was extracted with DCM. The organic phase was washed with water, dried over anhydrous Na₂SO₄ and the solvent was evaporated to dryness. The crude was purified via automated flash chromatography (Biotage Isolera Four coupled with Dalton 2000^{®}), SNAP25 cartridge, eluting in gradient with EtOAc - PE from 8:2 to 10:0. The collected fractions gave 181 mg (22% yield) of the title compound.

MS (ESI) m/z 379.99 [M + H]⁺

### 7-bromo-4-chloro-1-[(4-fluoro-3-methoxyphenyl)methyl]pyrido[3,2-d]pyrimidin-2(1H)-one (Intermediate 91)

To a suspension of 7-bromo-1-[(4-fluoro-3-methoxyphenyl)methyl]pyrido[3,2-d]pyrimidine-2,4(1H,3H)-dione (Intermediate 90,180 mg, 0.4734 mmol) in 10 mL of DIPEA (7.4 g, 64 mmol) was added phosphoryl trichloride (1 mL, 1.64 g, 10.7 mmol) and the resulting mixture was stirred at 50°C for 3 hours. The reaction was cooled to r.t. and concentrated under vacuum. The crude was rinsed with 1,4-dioxane and evaporated for 3 times, each time taking up with 1,4-dioxane. 180 mg (95% yield) of the title compound were used for the next step without further purification.

MS (ESI) m/z 397.97 [M + H]⁺

### 8-Bromo-3-tert-butyl-6-[(4-fluoro-3-methoxyphenyl)methyl]pyrido[2,3-e][1,2,4]triazolo[4,3-c]pyrimidin-5(6H)-one (Intermediate 92)

To a solution of 7-bromo-4-chloro-1-[(4-fluoro-3-methoxyphenyl)methyl]pyrido[3,2-d]pyrimidin-2(1H)-one (Intermediate 91, 180 mg, 0.4516 mmol) in 10 mL of 1,4-dioxane 2,2-dimethylpropanehydrazide (157.4 mg, 1.355 mmol) was added and the reaction mixture was stirred at 60°C for 6 hours. The reaction mixture was cooled to r.t., poured into water and extracted with EtOAc. The organic phase was washed with water, dried over anhydrous Na₂SO₄ and the solvent was evaporated to dryness. The crude obtained was purified via automated flash chromatography (Biotage Isolera Four coupled with Dalton 2000^{®}), SNAP10 cartridge, eluting in gradient with EtOAc - PE from 1:1 to 9:1 to give 80 mg (39% yield) of the title compound.

MS (ESI) m/z 460.19 [M + H]⁺

### 3-tert-Butyl-6-[(4-fluoro-3-methoxyphenyl)methyl]-8-(morpholin-4-yl)pyrido[2,3-e][1,2,4]triazolo[4,3-c]pyrimidin-5(6H)-one (Example 42)

In a microwave vial, a solution of 8-Bromo-3-tert-butyl-6-[(4-fluoro-3-methoxyphenyl)methyl]pyrido[2,3-e][1,2,4]triazolo[4,3-c]pyrimidin-5(6H)-one (Intermediate 92, 80 mg, 0.1738 mmol) and 30.3 µL of morpholine (30.28 mg, 0.3476 mmol) in 4 mL of anhydrous 1,4-dioxane was purged with N₂. After 5 minutes palladium(II) acetate (7.804 mg, 0.03476 mmol), XantPhos (40.22 mg, 0.06952 mmol) and caesium carbonate (113.3 mg, 0.3476 mmol) were added and the mixture was stirred in microwave oven at 150°C for 2 hours. The reaction was cooled to r.t., poured into water and extracted with EtOAc. The organic layer was washed with water, dried over anhydrous Na₂SO₄ and the solvent was evaporated to dryness. The obtained crude was purified via automated flash chromatography (Biotage Isolera Four coupled with Dalton 2000^{®}), SNAP25 cartridge, eluting in gradient with EtOAc : PE from 8:2 to 10:0 to give 10 mg (12% yield) of the title compound.

HPLC purity = 98.3%

MS (ESI) m/z 467.26 [M + H]⁺

¹H NMR (400 MHz, DMSO-d6) δ ppm 8.45 (d, 1H) 7.32 (dd, 1H) 7.13 (dd, 1H) 7.07 (d, 1H) 6.88 - 6.96 (m, 1H) 5.52 (s, 2H) 3.81 (s, 3H) 3.70 - 3.78 (m, 4H) 3.32 - 3.37 (m, 4H) 1.44 (s, 9H).

### Example 43

### 3-tert-Butyl-6-[(4-chlorophenyl)methyl]-8-[(3R)-3-hydroxypyrrolidin-1-yl]pyrido[2,3-e][1,2,4]triazolo[4,3-c]pyrimidin-5(6H)-one

### (R)-3-(tert-Butyl)-8-(3-((tert-butyldimethylsilyl)oxy)pyrrolidinl-yl)-6-(4-chlorobenzyl)pyrido[2,3-e][1,2,4]triazolo[4,3- c]pyrimidin-5(6H)-one (Intermediate 93)

In a vial equipped with a magnetic stirring bar 8-bromo-3-(tert-butyl)-6-(4-chlorobenzyl)pyrido[2,3-e][1,2,4]triazolo[4,3-c]pyrimidin-5(6H)-one (Intermediate 62, 100 mg, 0.220 mmol), 4,4'-Di-t-butyl-2,2'-bipyridine)bis[3,5-difluoro-2-[5-trifluoromethyl-2-pyridinyl-kN)phenyl-kC]iridium(III)hexafluorophosphate (5.02 mg), DABCO (45.2 mg, 0.400 mmol) and tert-butyl-dimethyl-[(3R)-pyrrolidin-3-yl]oxy-silane (112.7 mg, 0.340 mmol) were solved in 0.3 mL of DMA. Then Nickel(2+) chloride - 1,2-dimethoxyethane (1:2:1) (9.84 mg, 0.040 mmol) was added as a solution in 0.3 mL of DMA. The vial was then placed under N₂, cooled at -78°C and degassed, backfilled with N₂ and heated up slowly to room temperature. The purge cycle was repeated 3 times. Then the stirred mixture was irradiated (blue light, 365nm LED, 2 cm distance from 34 W no fan cooling) for 4h. The mixture was diluted with EtOAc and washed with water. The organic layer was dried over anhydrous Na₂SO₄, filtered and concentrated to dryness. The obtained crude was purified by Biotage-Isolera^{®}, Luknova Supersep 5.5 g eluting in gradient with H₂O : MeCN from 100:0 to 0:100. The collected fractions gave 100 mg (47% yield) of the title compound.

MS (ESI) m/z 567.07 [M + H]⁺

### 3-tert-Butyl-6-[(4-chlorophenyl)methyl]-8-[(3R)-3-hydroxypyrrolidin-1-yl]pyrido[2,3-e][1,2,4]triazolo[4,3-c]pyrimidin-5(6H)-one (Example 43)

In a round bottom flask (R)-3-(tert-butyl)-8-(3-((tert-butyldimethylsilyl)oxy)pyrrolidin-1-yl)-6-(4-chlorobenzyl)pyrido[2,3-e][1,2,4]triazolo[4,3-c]pyrimidin-5(6H)-one (Intermediate 93, 100 mg, 0.110 mmol) was solved in 0.8 mL of DCM. Then 200 µL of TFA (0.003 mol) were added and the mixture was stirred for 4h. The crude was diluted with DCM and basified (pH 8) with DIPEA. The solvent was removed *in vacuo.* The crude was purified by reverse phase HPLC using a FractionLynx system. Collected fractions gave 10.5 mg (yield: 21.7%) of (R)-3-(tertbutyl)-6-(4-chlorobenzyl)-8-(3-hydroxypyrrolidin-1-yl)pyrido[2,3-e][1,2,4]triazolo[4,3-c]pyrimidin-5(6H)-one as white powder.

HPLC purity = 99%

MS (ESI) m/z 453 [M + H]⁺

¹H NMR (400 MHz, DMSO-d6) δ ppm 8.20 (s, 1H), 7.55 - 7.32 (m, 4H), 6.59 (br s, 1H), 5.58 (s, 2H), 4.44 (br d, J = 1.8 Hz, 1H), 3.64 - 3.13 (m, 5H), 2.13 - 1.87 (m, 2H), 1.56 (s, 9H).

### Example 44

### 3-tert-Butyl-6-[(4-chlorophenyl)methyl]-8-(morpholin-4-yl)[1,2,4]triazolo[4',3':1,6]pyrimido[5,4-c]pyridazin-5(6H)-one

The compound of Example 44, corresponding to the Example 191 of the Patent US 2021/0246139, was prepared by the procedures described for the Example 180 but replacing (4-methoxyphenyl)methanamine with (4-chlorophenyl)methanamine at Step 1.

An alternative preparation pathway is described below:

To a solution of 5-[(4-chlorophenyl)methyl]-3-morpholino-8-thioxo-pyrimido[5,4-c]pyridazin-6-one (Intermediate 38, 1.76 g, 4.514 mmol) in 30 mL of pyridine, 2,2-dimethylpropanehydrazide (629.2 mg, 5.417 mmol) was added and the mixture was stirred at 120°C for 5h. After cooling at r.t., pyridine was removed under vacuum. The crude residue was dissolved in EtOAc. The organic layer was washed with water, brine and dried over anhydrous Na₂SO₄ and evaporated under vacuum. The crude dark oil (3 g) was solved in 30 mL of anhydrous THF and Burgess reagent (2.151 g, 9.028 mmol) was added. The mixture was stirred at room temperature for 2 days. The solvent was removed under vacuum and the residue dissolved in DCM. The organic layer was washed with water, brine and dried over anhydrous Na₂SO₄, filtered, and evaporated *in vacuo.* The crude residue was purified by chromatography using a Biotage Selekt^{®} instrument, cartridge type SNAP25, with a gradient from EtOAc 100% to EtOAc : MeOH 90:10. The fraction collected gave 490 mg (yield: 23.9 %) of title compound as a light brown powder.

HPLC purity = 97.81 %

MS (ESI) m/z 453.98 [M + H]⁺

¹H NMR (400 MHz, DMSO-d6 ) δ ppm 7.43 - 7.50 (m, 2H) 7.33 - 7.42 (m, 2H) 6.76 (s, 1H) 5.44 (s, 2H) 3.70 - 3.82 (m, 4H) 3.59 - 3.70 (m, 4H) 1.55 (s, 9H).

### Example 45

### tert-Butyl 4-{6-[(4-chlorophenyl)methyl]-3-(2-methoxypropan-2-yl)-5-oxo-5,6-dihydro[1,2,4]triazolo[4',3':1,6]pyrimido[5,4-c]pyridazin-8-yl}piperazine-1-carboxylate

### 3-Chloro-5-[(4-chlorophenyl)methyl]pyrimido[5,4-c]pyridazine-6,8-dione (Intermediate 94)

To a cooled solution of 6-chloro-4-[(4-chlorophenyl)methylamino]pyridazine-3-carboxamide (Intermediate 34, 1 g, 3.366 mmol) in 15 mL of dry DMF, 60% sodium hydride oil dispersion (0.807 g, 20.20 mmol) and di(imidazol-1-yl)methanone (3.275 g, 20.20 mmol) were added. The mixture was stirred at room temperature for 30 min. Then 20 mL of acetic were added until pH 5 and ater was added (200 mL). The white precipitate was filtered, collected and dried in oven at 50°C for 4 hours. The product was used in the next step without any further purification.

MS (ESI) m/z 322.97 [M + H]+

### tert-Butyl 4-[5-[(4-chlorophenyl)methyl]-6,8-dioxo-pyrimido[5,4-c]pyridazin-3-yl]piperazine-1-carboxylate (Intermediate 95)

In a screw cap vial 3-chloro-5-[(4-chlorophenyl)methyl]pyrimido[5,4-c]pyridazine-6,8-dione (Intermediate 94, 0.32 g, 0.9904 mmol) and tert-butyl piperazine-1-carboxylate (0.5534 g, 2.971 mmol) and 5 mL of DMAC were added. The reaction vial was placed into a microwave oven (Biotage Smith Creator) and heated at 100°C for 1 hour. The solution was cooled at room temperature, diluted with ethyl acetate and washed with water. The organic phase was dried over anhydrous sodium sulphate, filtered and evaporated to dryness. The resulting white-off powder was used in the next step without any further purification (0.46 g, 98% yield).

MS (ESI) m/z 473.06 [M + H]⁺

### tert-Butyl 4-{5-[(4-chlorophenyl)methyl]-6-oxo-8-sulfanylidene-5,6,7,8-tetrahydropyrimido[5,4-c]pyridazin-3-yl}piperazine-1-carboxylate (Intermediate 96)

To a solution of tert-butyl 4-[5-[(4-chlorophenyl)methyl]-6,8-dioxo-pyrimido[5,4-c]pyridazin-3-yl]piperazine-1-carboxylate (Intermediate 95, 0.4 g, 0.85 mmol) in 12 mL of pyridine, Lawesson's reagent (1.369 g, 3.383 mmol) was added and the mixture was stirred at 120°C. After 4 h the reaction mixture was cooled with an ice bath, then 40 mL of water were added until a yellow/brown precipitate occurred. The solid was collected via filtration, washed with water and dried in a oven at 35°C overnight. The desired product (370 mg, 89% yield) was used in the next step without any further purification.

MS (ESI) m/z 489.21 [M + H]⁺

### tert-Butyl 4-{6-[(4-chlorophenyl)methyl]-3-(2-methoxypropan-2-yl)-5-oxo-5,6-dihydro[1,2,4]triazolo[4',3':1,6]pyrimido[5,4-c]pyridazin-8-yl}piperazine-1-carboxylate

### (Example 45)

To a solution of tert-butyl 4-{5-[(4-chlorophenyl)methyl]-6-oxo-8-sulfanylidene-5,6,7,8-tetrahydropyrimido[5,4-c]pyridazin-3-yl}piperazine-1-carboxylate (Intermediate 96, 360 mg, 0.736 mmol) in 10 mL of pyridine, 2-methoxy-2-methyl-propanehydrazide (389 mg, 2.94 mmol) was added and the mixture was stirred at 120°C. After 1 hour the reaction was cooled at r.t. and evaporated under vacuum. The residue was dissolved in DCM and washed with water and brine. The organic phase was dried over anhydrous sodium sulphate and evaporated to dryness. The crude was dissolved in 12 mL of anhydrous THF and Burgess reagent (701 mg, 2.94 mmol) was added and the mixture was stirred at r.t. overnight. Then, the solvent was removed under vacuum and the residue was dissolved in DCM and washed with water and brine and dried over anhydrous sodium sulphate. The solvent was evaporated and the crude was purified via automated flash chromatography (Biotage Isolera One^{®}) using a gradient from DCM 100% to DCM : MeOH 95:5. The fractions collected gave 160 mg (yield: 38%) of the title compound as a yellow solid.

HPLC purity = 99.06%

MS (ESI) m/z 569.44 [M + H]⁺

¹H-NMR (DMSO-d6) δ ppm 7.43-7.50 (m, 2H), 7.36-7.42 (m, 2H), 6.76 (s, 1H), 5.44 (s, 2H), 3.65-3.79 (m, 4H), 3.40-3.53 (m, 4H), 3.07 (s, 3H), 1.75 (s, 6H), 1.44 (s, 9H)

### Example 46

### 6-[(4-Chlorophenyl)methyl]-3-(2-methoxypropan-2-yl)-8-(piperazin-1-yl)[1,2,4]triazolo[4',3':1,6]pyrimido[5,4-c]pyridazin-5(6H)-one

To a solution of tert-butyl 4-{6-[(4-chlorophenyl)methyl]-3-(2-methoxypropan-2-yl)-5-oxo-5,6-dihydro[1,2,4]triazolo[4',3':1,6]pyrimido[5,4-c]pyridazin-8-yl}piperazine-1-carboxylate (Example 45, 150 mg, 0.2636 mmol) in 2 mL of DCM was added a 4M hydrogen chloride sol. in 1,4-dioxane (0.3295 mL, 1.318 mmol). The mixture was stirred for 1 hour at room temperature. The solvent was evaporated to dryness *in vacuo.* The title compound was obtained (120 mg, 97% yield) as yellow/brown solid.

HPLC purity = 95.55%

MS (ESI) m/z 469.32 [M + H]⁺

¹H NMR (DMSO-d6) δ ppm 9.36 (br s, 2H), 7.43-7.52 (m, 2H), 7.35-7.42 (m, 2H), 6.91 (s, 1H), 5.46 (s, 2H), 3.88-4.01 (m, 4H), 3.16-3.32 (m, 4H), 3.07 (s, 3H), 1.75 (s, 6H)

### Example 47

### 8-(4-Acetylpiperazin-1-yl)-6-[(4-chlorophenyl)methyl]-3-(2-methoxypropan-2-yl)[1,2,4]triazolo[4',3':1,6]pyrimido[5,4-c]pyridazin-5(6H)-one

To a solution of 6-[(4-chlorophenyl)methyl]-3-(2-methoxypropan-2-yl)-8-(piperazin-1-yl)[1,2,4]triazolo[4',3':1,6]pyrimido[5,4-c]pyridazin-5(6H)-one (Example 46, 60 mg, 0.1187 mmol) and 36.4 µL of TEA (0.26 mmol) in 2 mL of anhydrous THF was added dropwise acetyl chloride (10.13 µL, 0.1425 mmol) and the mixture was stirred at r.t. for 30 min. The solvent was removed *in vacuo.* The crude was dissolved in DCM which was washed with water, brine and dried over anhydrous sodium sulphate and evaporated to dryness to obtain 51.8 mg (yield: 85%) of the title compound.

HPLC purity = 95.84%

MS (ESI) m/z 511.26 [M + H]⁺

¹H NMR (400 MHz, DMSO-d6) δ ppm 7.42 - 7.51 (m, 2H) 7.37 - 7.42 (m, 2H) 6.77 (s, 1H) 5.45 (s, 2H) 3.64 - 3.86 (m, 4H) 3.59 (br d, 4H) 3.07 (s, 3H) 2.06 (s, 3H) 1.75 (s, 6H).

### Example 48

### 6-[(4-Chlorophenyl)methyl]-3-(2-methoxypropan-2-yl-8-(morpholin-4-yl)[1,2,4]triazolo[4',3':1,6]pyrimido[5,4-c]pyridazin-5(6H)-one

To a solution of 5-[(4-chlorophenyl)methyl]-3-(morpholin-4-yl)-8-sulfanylidene-7,8-dihydropyrimido[5,4-c]pyridazin-6(5H)-one (Intermediate 38, 300 mg, 0.7694 mmol) in 7 mL of pyridine 2-methoxy-2-methyl-propanehydrazide (406.8 mg, 3.078 mmol) was added and the reaction mixture stirred at 120°C. After 1 hour, the reaction was cooled to room temperature and evaporated under vacuum. The residue was dissolved in DCM which was washed with water and brine. The organic phase was dried over anhydrous sodium sulphate and evaporated to dryness. The crude was dissolved in 7 mL of THF and Burgess reagent (733.4 mg, 3.078 mmol) was added. After overnight stirring, further Burgess reagent (733.4 mg. 3.078 mmol) was added portionwise and the mixture stirred at r.t. for further 12 hours. The solvent was removed under vacuum; then the residue was dissolved in DCM, which was washed with water and brine. The organic phase was dried over anhydrous sodium sulphate and evaporated to dryness. The crude was purified via automated flash chromatography (Biotage Isolera One^{®}) eluting with DCM : MeOH using a gradient from 100:0 to 97:3. The fractions collected afforded 160 mg (50% yield) of the title compound as a yellowish solid.

HPLC purity = 92.38%

MS (ESI) m/z 470.30 [M + H]⁺

¹H NMR (400 MHz, DMSO-d6) δ ppm 7.42 - 7.59 (m, 1H) 7.32 - 7.42 (m, 1H) 6.77 (s, 1H) 5.44 (s, 1H) 3.70 - 3.86 (m, 4H) 3.55 - 3.70 (m, 4H) 3.07 (s, 2H) 1.75 (s, 3H)

### Example 49

### 6- [(4-Chlorophenyl)methyl]-3-(2,2-dimethylpropyl)-8-(morpholin-4-yl)[1,2,4]triazolo[4',3':1,6]pyrimido[5,4-c]pyridazin-5(6H)-one

To a solution of 5-[(4-chlorophenyl)methyl]-3-(morpholin-4-yl)-8-sulfanylidene-7,8-dihydropyrimido[5,4-c]pyridazin-6(5H)-one (Intermediate 38, 124 mg, 0.3180 mmol) in 5 mL of pyridine, 3,3-dimethylbutanehydrazide (165.6 mg, 1.272 mmol) was added and the mixture was stirred at 120°C for 1 hour. After cooling at room temperature, pyridine was removed under vacuum. The crude was dissolved in EtOAc and washed with water and brine. The organic layer was dried over Na₂SO₄, filtered and evaporated. The crude residue (570 mg) was dissolved in 5 mL of THF and Burgess reagent was added (303.1 mg, 1.272 mmol). The mixture was stirred at room temperature for 15 min then warmed up to 50°C. The reaction was stirred at the same temperature for two days adding further Burgess reagent amounts until complete conversion. The solvent was removed under reduced pressure and the crude dissolved in EtOAc, which was washed with brine. The organic layer was dried over anhydrous Na₂SO₄, filtered and evaporated. The final residue was purified by using a Biotage Selekt^{®} instrument, cartridge type SNAP10, eluting with a gradient from EtOAc 100% to EtOAc : MeOH 90: 10. The collected fractions gave 40 mg (27% yield) of the title compound as a yellow solid.

HPLC purity = 93.66%

MS (ESI) m/z 468,09 [M + H]⁺

¹H NMR (400 MHz, DMSO-d6) δ ppm 7.41 - 7.49 (m, 2H) 7.33 - 7.40 (m, 2H) 6.78 (s, 1H) 5.42 (s, 2H) 3.58 - 3.80 (m, 8H) 3.26 (s, 2H) 1.04 (s, 9H)

### Example 50

### 6-[(4-Chlorophenyl)methyl]-3-(dimethylamino)-8-(morpholin-4-yl)[1,2,4]triazolo[4',3':1,6]pyrimido[5,4-c]pyridazin-5(6H)-one

### 5-[(4-Chlorophenyl)methyl]-8-hydrazinyl-3-(morpholin-4yl)pyrimido[5,4-c]pyridazin-6(5H)-one (Intermediate 98)

To a solution of 5-[(4-chlorophenyl)methyl]-3-(morpholin-4-yl)-8-sulfanylidene-7,8-dihydropyrimido[5,4-c]pyridazin-6(5H)-one (Intermediate 38, 200 mg, 0.5130 mmol) in 1.5 mL EtOH in a microwave vial was added hydrazine hydrate (0.598 mL 616.3 mg, 12.31 mmol) and the solution was heated under microwave irradiation at 80°C for 45 min. After cooling at room temperature, the solvent was removed under vacuum. 196 mg (98% yield) of 5-[(4-chlorophenyl)methyl]-8-hydrazinyl-3-(morpholin-4-yl)pyrimido[5,4-c]pyridazin-6(5H)-one as light yellow powder were recovered and used in the next step without any further purification.

MS (ESI) m/z 388.14 [M + H]⁺

### 2-{5-[(4-Chlorophenyl)methyl]-3-(morpholin-4-yl)-6-oxo-5,6-dihydropyrimido[5,4-c]pyridazin-8-yl}-N,N-dimethylhydrazine-1-carboxamide (Intermediate 99)

To a suspension of 5-[(4-chlorophenyl)methyl]-8-hydrazinyl-3-(morpholin-4-yl)pyrimido[5,4-c]pyridazin-6(5H)-one (Intermediate 98, 196 mg, 0.50 mmol) in 10 mL of DCM under stirring at 0-5°C were added 0.141 µL of TEA (102 mg, 1.01 mmol) followed by N,N-dimethylcarbamoyl chloride (69.8 0 µL, 81.53 mg, 0.76 mmol). The mixture was warmed up to room temperature and stirred overnight, then heated at 50°C for 8h. After cooling at room temperature, water and EtOAc were added, the two phases separated and the organic layer was washed with brine, dried over Na₂SO₄, filtered, and evaporated *in vacuo.* The crude oil was taken up with diethyl ether and the obtained solid was filtered and dried under vacuum to obtain 90 mg of 2-{5-[(4-chlorophenyl)methyl]-3-(morpholin-4-yl)-6-oxo-5,6-dihydropyrimido[5,4-c]pyridazin-8-yl}-N,N-dimethylhydrazine-1-carboxamide (yield: 38.8%) as light yellow powder, which was used in the next step without any further purification.

MS (ESI) m/z 459.04 [M + H]⁺

### 6-[(4-Chlorophenyl)methyl]-3-(dimethylamino)-8-(morpholin-4-yl)[1,2,4]triazolo[4',3':1,6]pyrimido[5,4-c]pyridazin-5(6H)-one (Example 50)

To a solution of 2-{5-[(4-chlorophenyl)methyl]-3-(morpholin-4-yl)-6-oxo-5,6-dihydropyrimido[5,4-c]pyridazin-8-yl}-N,N-dimethylhydrazine-1-carboxamide (Intermediate 99, 35 mg, 0.076 mmol), triphenylphosphine (40.01 mg, 0.1525 mmol) and TEA (0.042 mL, 30.87 mg, 0.3051 mmol) in 10 mL of anhydrous THF cooled at 0-5°C, was added 1,1,1,2,2,2-hexachloroethane (36.11 mg, 0.1525 mmol) and the resulting suspension was stirred at room temperature overnight. The reaction was concentrated under vacuum. The crude was purified by Biotage Isolera One^{®}, cartridge type SNAP50, using a gradient EtOAc : MeOH from 99:1 to 85:15. The collected fractions afforded 11 mg (32.7% yield) of the title compound.

HPLC purity = 87.08%

MS (ESI) m/z 441.14 [M + H]⁺

¹H NMR (400 MHz, DMSO-d6) δ ppm 7.42 - 7.52 (m, 2H) 7.31 - 7.41 (m, 2H) 6.71 (s, 1H) 5.36 (s, 2H) 3.67 - 3.78 (m, 4H) 3.55 - 3.66 (m, 4H) 2.97 (s, 6H)

### Example 51

### 6-[(4-Methoxyphenyl)methyl]-2-(2-methylpropyl)-8-(morpholin-4-yl)pyrido[2,3-e][1,2,4]triazolo[1,5-c)pyrimidin-5(6H)-one

Prepared following the procedure described in Patent US 2021/0246139 for Example 96 and characterized as Example 156.

### Example 52

### 2-tert-Butyl-6-[(4-chlorophenyl)methyl]-8-(3-hydroxypiperidin-1-yl)pyrido[2,3-e][1,2,4]triazolo[1,5-c]pyrimidin-5(6H)-one

### 8-Bromo-2-(tert-butyl)-6-(4-chlorobenzyl)pyrido[2,3-e][1,2,4]triazolo[4,3-c]pyrimidin-5(6H)-one (Intermediate 100)

The title compound was prepared following the procedure described for the synthesis of Intermediate 62. During the purification carried out using a Biotage-Isolera^{®} instrument, equipped with Silicycle SiliaSep 120g HP cartridge and eluting with a PE : EtOAc from 80:20 to 0:100, were isolated 563 mg (46 % yield) of the kinetic isomer8-bromo-3-tert-butyl-6-[(4-chlorophenyl)methyl]pyrido[2,3-e][1,2,4]triazolo[4,3-c]pyrimidin-5(6H)-one (Intermediate 62) and 118 mg (10% yield) of thermodynamic compound 8-bromo-2-(tert-butyl)-6-(4-chlorobenzyl)pyrido[2,3-e][1,2,4]triazolo[4,3-c]pyrimidin-5(6H)-one (Intermediate 100).

¹HMR (400 MHz, DMSO- 1 d6) δ ppm 1.45 (s, 10H) 5.56 (s, 2H) 7.41 (q, J=8.77 Hz, 4H) 8.19 (s, 1H) 8.76 (s, 1H)

### 2-tert-Butyl-6-[(4-chlorophenyl)methyl]-8-(3-hydroxypiperidin-1-yl)pyrido[2,3-e][1,2,4]triazolo[1,5-c]pyrimidin-5(6H)-one (Example 52)

In a vial equipped with a magnetic stirring bar, 8-bromo-2-(tert-butyl)-6-(4-chlorobenzyl)pyrido[2,3-e][1,2,4]triazolo[4,3-c]pyrimidin-5(6H)-one (Intermediate 100, 70 mg, 0.160 mmol), 4,4'-di-t-butyl-2,2'-bipyridine)bis[3,5-difluoro-2-[5-trifluoromethyl-2-pyridinyl-kN)phenylkC]iridium(III) hexafluorophosphate (3.52 mg), DABCO (31.64 mg 0.280 mmol) and piperidin-3-ol (25.02 mg, 0.240 mmol) were dissolved in 0.3 mL of DMA. Then Nickel (2+) chloride - 1,2-dimethoxyethane (1:2:1) (6.89 mg, 0.030 mmol) was added as a solution in 0.3 mL of DMA. The vial was then placed under N₂, cooled at -78°C and degassed, backfilled with N₂ and warmed up slowly to r.t. The cycle was repeated 3 times, then the mixture irradiated (blue light, 365nm LED, 2 cm distance from 34 W, no fan cooling) while stirring for 3h. The mixture was diluted with EtOAc and washed with water. The organic layer was dried over anhydrous Na₂SO₄, filtered and concentrated *in vacuo.* The crude was purified by Biotage-Isolera^{®} instrument, equipped with Silicycle SiliaSep 4g cartridge, eluting in gradient with a mixture of PE : EtOAc from 100:0 to 0:100. The crude (40 mg) was further purified by reverse phase HPLC by Fraction Lynx afforded 7 mg (yield: 7.6%) of the title compound.

HPLC purity = 80%

MS (ESI) m/z 467.31 [M + H]⁺

¹H NMR (DMSO-d6) δ ppm 8.40 (s, 1H), 7.40-7.50 (m, 2H), 7.34-7.40 (m, 2H), 6.95 (s, 1H), 5.55 (s, 2H), 4.88 (d, 1H), 3.66-3.79 (m, 1H), 3.50-3.64 (m, 2H), 2.97-3.12 (m, 1H), 2.81-2.97 (m, 1H), 1.81-1.94 (m, 1H), 1.66-1.80 (m, 1H), 1.42 (s, 9H), 1.30-1.48 (m, 2H)

### Example 53

### 9-tert-Butyl-5-(4-chlorobenzyl)-3-(4-hydroxypiperidin-1-yl)[1,2,4]triazolo[1',5':1,6]pyrimido[5,4-c]pyridazin-6(5H)-one and

### Example 55

### 3-tert-Butyl-6-[(4-chlorophenyl)methyl]-8-(4-hydroxypiperidin-1-yl)[1,2,4]triazolo[4',3':1,6]pyrimido[5,4-c]pyridazin-5(6H)-one

### 1-[5-(4-Chlorobenzyl)-6,8-dioxo-5,6,7,8-tetrahydropyrimido[5,4-c]pyridazin-3-yl]piperidin-4-yl acetate (Intermediate 101)

To a screwcap vial were added 90% 4-piperidyl acetate (1.284 g, 8.072 mmol), and 3-chloro-5-[(4-chlorophenyl)methyl]pyrimido[5,4-c]pyridazine-6,8-dione (Intermediate 94, 438 mg, 1.345 mmol,) in 12 mL of DMA. The reaction vial was placed into the microwave oven (Biotage Smith Creator^{®}) and heated at 100 °C for 1h. The mixture was diluted with 100 mL of water and extracted with DCM. The organic phase was dried over anhydrous sodium sulphate and evaporated to dryness. The residue which was containing residual DMA was diluted with ethyl acetate and the organic phase was washed with water, dried over anhydrous sodium sulphate and evaporated to dryness affording 450 mg (yield: 77.8%) of the desired product as an off-white solid.

MS (ESI) m/z 429.88 [M + H]⁺

### 1-[5-(4-Chlorobenzyl)-6-oxo-8-sulfanylidene-5,6,7,8-tetrahydropyrimido[5,4-c]pyridazin-3-yl]piperidin-4-yl acetate (Intermediate 102)

To a solution of [1-[5-[(4-chlorophenyl)methyl]-6,8-dioxo-pyrimido[5,4-c]pyridazin-3-yl]-4-piperidyl] acetate (Intermediate 101, 383 mg, 0.89 mmol) in 12 mL of pyridine Lawesson's reagent (2.523 g, 6.236 mmol) was added portionwise and the mixture was stirred at 120°C for 12 hours. The mixture was cooled with an ice bath, then 60 mL of water were added until a yellow precipitate was formed. The yellow solid was filtered, washed with water and dried in an oven at 50°C for 4 hours. The desired product (324 mg, 81.5% yield) was used in the next step without any further purification.

MS (ESI) m/z 445.86 [M + H]⁺

### 1-[3-tert-Butyl-6-(4-chlorobenzyl)-5-oxo-5,6-dihydro[1,2,4]triazolo[4',3':1,6]pyrimido[5,4-c]pyridazin-8-yl]piperidin-4-yl acetate (Intermediate 103)

To a solution of 1-[5-(4-chlorobenzyl)-6-oxo-8-sulfanylidene-5,6,7,8-tetrahydropyrimido[5,4-c]pyridazin-3-yl]piperidin-4-yl acetate (Intermediate 102, 324 mg, 0.7266 mmol) in 12 mL of pyridine, 2,2-dimethylpropanehydrazide (337.6 mg, 2.906 mmol) was added and the mixture was stirred at 120°C for 1 hour. UPLC-MS showed the complete conversion of the starting material into the not isolated intermediate with MW 527. The reaction was cooled and the solvent evaporated under vacuum. The residue was dissolved in ethyl acetate and washed with water and brine. The organic phase was dried over anhydrous sodium sulphate and evaporated to dryness. The crude was dissolved in 12 mL of anhydrous THF and Burgess reagent (692.6 mg, 2.906 mmol) was added. The mixture was stirred at r.t. overnight. The solvent was removed under vacuum, then the residue was redissolved in DCM and washed with water and brine. The organic phase was dried over anhydrous sodium sulphate and evaporated to dryness. The crude was purified via automated flash chromatography (Biotage Isolera One^{®} instrument) using a gradient from DCM 100% to DCM : MeOH 95:5. The collected fractions afforded 125 mg (yield: 33.7%) of a yellowish solid.

MS (ESI) m/z 509.92 [M +H]⁺

### 9-tert-Butyl-5-(4-chlorobenzyl)-3-(4-hydroxypiperidin-1-yl)[1,2,4]triazolo[1',5':1,6]pyrimido[5,4-c]pyridazin-6(5H)-one (Example 53) and

### 3-tert-butyl-6-[(4-chlorophenyl)methyl]-8-(4-hydroxypiperidin-1-yl)[1,2,4]triazolo[4',3':1,6]pyrimido[5,4-c]pyridazin-5(6H)-one (Example 55).

To a suspension of 1-[3-tert-butyl-6-(4-chlorobenzyl)-5-oxo-5,6-dihydro[1,2,4]triazolo[4',3':1,6]pyrimido[5,4-c]pyridazin-8-yl]piperidin-4-yl acetate (Intermediate 103, 102 mg, 0.20 mmol) in 2 mL of MeOH, acetyl chloride (284.4 µL, 314 mg, 4.00 mmol) was added. The mixture was stirred at r.t. for 1hour. The crude was purified via automated flash chromatography (Biotage Isolera One^{®} instrument) using a gradient of DCM : MeOH from 100:0 to 90:10. The fractions collected afforded 18 mg (yield: 19%) of the title compound, 9-tert-butyl-5-(4-chlorobenzyl)-3-(4-hydroxypiperidin-1-yl)[1,2,4]triazolo[1',5':1,6]pyrimido[5,4-c]pyridazin-6(5H)-one (Example 53), which is the thermodynamic product and 76 mg of the kinetic product 3-tert-butyl-6-[(4-chlorophenyl)methyl]-8-(4-hydroxypiperidin-1-yl)[1,2,4]triazolo[4',3':1,6]pyrimido[5,4-c]pyridazin-5(6H)-one (Example 55).

### Example 53

HPLC purity 95.28 %

MS (ESI) m/z 468.34 [M +H]⁺

¹H NMR (DMSO-d6) δ ppm 7.42-7.54 (m, 2H), 7.31-7.42 (m, 2H), 6.78 (s, 1H), 5.49 (s, 2H), 4.08-4.22 (m, 2H), 3.67-3.83 (m, 1H), 3.29-3.46 (m, 2H), 1.69-1.91 (m, 2H), 1.44 (s, 9H), 1.22-1.40 (m, 2H)

### Example 55

HPLC purity 65.37 % containing the 17.13 % of Example 53

MS (ESI) m/z 468.41 [M +H]⁺

¹H NMR (DMSO-d6) δ ppm 7.43-7.48 (m, 2H), 7.34-7.42 (m, 2H), 6.74 (s, 1H), 5.45 (s, 2H), 4.07-4.20 (m, 2H), 3.71-3.82 (m, 1H), 3.25-3.46 (m, 2H), 1.71-1.89 (m, 3H), 1.55 (s, 9H), 1.38-1.46 (m, 1H)

### Example 54

### 5-[(4-Chlorophenyl)methyl]-9-(2,2-dimethylpropyl)-3-(morpholin-4-yl)[1,2,4]triazolo[1',5':1,6]pyrimido[5,4-c]pyridazin-6(5H)-one

### 8-Chloro-5-[(4-chlorophenyl)methyl]-3-morpholino-pyrimido[5,4-c]pyridazin-6-one (Intermediate 104)

To a suspension of 5-[(4-chlorophenyl)methyl]-3-morpholino-pyrimido[5,4-c]pyridazine-6,8(5H,7H)-dione (Intermediate 37, 150 mg, 0.40 mmol)) in 210 µL of DIPEA (155.6 mg, 1.204 mmol) stirred under nitrogen atmosphere, phosphoryl trichloride (2.992 mL, 4.922 g, 32.10 mmol) was added dropwise and the mixture was stirred at 50°C for 30 min. Then, it was cooled down to room temperature and POCl₃ was evaporated under reduced pressure. The residue was diluted with 1,4-dioxane for 3 times and concentrated *in vacuo* to completely remove the POCl₃. This mixture was used in the next step without any further purification.

### Methyl 3,3-dimethylbutanoate (Intermediate 105)

Prepared following the procedure described for the Intermediate 79 and using 3,3-dimethylbutanoic acid instead of 2-methoxy-2-methyl-propanoic acid. The crude product was used in the next step without further purification.

### 3,3-Dimethylbutanehydrazide (Intermediate 106)

Prepared following the procedure described for the Intermediate 80 but using methyl 3,3-dimethylbutanoate (Intermediate 105) instead of methyl 2-methoxy-2-methyl-propanoate (14.9% yield).

MS (ESI) m/z 131.18 [M + H]⁺

### 5-[(4-Chlorophenyl)methyl]-9-(2,2-dimethylpropyl)-3-(morpholin-4-yl)[1,2,4]triazolo[1',5':1,6]pyrimido[5,4-c]pyridazin-6(5H)-one (Example 54)

To a solution of 8-chloro-5-[(4-chlorophenyl)methyl]-3-morpholino-pyrimido[5,4-c]pyridazin-6-one (Intermediate 104, 157 mg, 0.40 mmol) in 5 mL of anhydrous 1,4-dioxane, 3,3-dimethylbutanehydrazide (Intermediate 106, 208.5 mg, 1.601 mmol) was added and the mixture was stirred at room temperature for 30 min. Then, the mixture was stirred at 100°C for 8 hours. After cooling at room temperature, water and EtOAc were added, the two phases were separated and the aqueous layer was extracted with EtOAc. The combined organic layers were dried over anhydrous Na₂SO₄, filtered and evaporated *in vacuo.* The crude residue was purified by means of a Biotage Selekt^{®} instrument, cartridge type SNAP25, using a gradient of PE : EtOAc from 50:50 to 0:100. The collected fractions gave 11 mg (5.87% yield) of the title compound as white powder.

HPLC purity = 98.32%

MS (ESI) m/z 468.36 [M + H]⁺

¹H NMR (400 MHz, DMSO-d6) δ ppm 7.42 - 7.48 (m, 2H) 7.35 - 7.42 (m, 2H) 6.83 (s, 1H) 5.49 (s, 2H) 3.71 - 3.78 (m, 4H) 3.65 - 3.71 (m, 4H) 2.77 (s, 2H) 1.06 (s, 9H).

### Example 56

### Biological Assay: Calcium Fluorescence Measurements

### Determination of human P2X3 receptor activity inhibition

The assay for the human purinergic P2RX3 receptor has been adapted to the internal screening instrumentation using the FLEXSTATION as reader and a photoprotein as luminescent readout. The assay consists of CHO-K1 cells recombinantly co-expressing the human P2rx3 receptor and a Ca²⁺ sensitive photoprotein. Cells are maintained in DMEM F-12 (1:1) mixture (LONZA cat. n° BE04-687F/U1) supplemented with 9 mL of 100 mM Sodium Pyruvate (LONZA cat. n° BE13-115E), 29 mL of 7.5% Sodium Bicarbonate (LONZA cat. n° BE17-613E), 5.5 mL of 1 M Hepes (LONZA cat. n° BE17-737E), 5 mL of 100X Penicillin/Streptomycin mixture (LONZA cat. n° DE17-602E) and 50 mL of Fetal Bovine Serum (Sigma cat. n° F7524), 1 mg/mL G418 disulfate salt (Sigma cat. n° G8168) and 5 µg/ml Puromycin dihydrochloride (Sigma cat. n° P9620). Standard propagation conditions consist of seeding in a P75 flask twice a week, recovering about 20×10⁶ cells, corresponding to about 80% confluency. Cells were seeded into black-walled clear bottom 96 well plates at a density of 40000 cells/well in a complete medium. After 24 h the culture medium was replaced with 200 µL/well of coelenterazine in Tyrode's buffer (Standard Tyrode's buffer: in house solution, 130 mM NaCl, 5 mM KCl, 2 mM CaCl2, 5 mM NaHCO3, 1 mM MgCl2, 20 mM HEPES, pH 7.4). Coelenterazine was purchased from BIOSYNTH (cat. n° C-7001). 10 mM stock solution was prepared in DMSO and glutathione and stored at -20° C. The plates were incubated for 4 hours at 37°C, and injected with 10 µL/well of test compounds at 25X concentration in Tyrode's buffer. After 4 minutes a second injection of 50 µL/well of 5X α,β-Met-ATP in Tyrode's buffer (α,β-Met-ATP: from Tocris (cat. n° 3209), was dissolved at 100 mM in water and stored in aliquots at -20°C) was performed and the signal of the emitted luminescence was recorded by FLEXSTATION III (Molecular Devices). For example, the tested compounds showed an antagonism versus human P2X₃ receptor between 1 nM and 10 µM.

### Determination of human P2X2/3 receptor activity inhibition

The assay for the human P2RX2/X3 has been evaluated by the screening instrumentation using the FLIPRTETRA as reader and the FLUO8-NoWash dye as fluorescent readout for calcium detection.

CHO-K1 cells recombinantly expressing the human P2RX2/X3 channel are maintained in DMEM F-12 (1:1) mixture (LONZA cat. n°: BE04-687F/U1) supplemented with 5 mL of 100 mM Sodium Pyruvate (LONZA cat. n° BE13-115E), 25 mL of 7.5% Sodium Bicarbonate (LONZA cat. n° BE17-613E), 6.5 mL of 1M Hepes (LONZA cat. n° BE17-737E), 5 mL of 100X Penicillin/Streptomycin mixture (LONZA cat. n° DE17-602E) and 50 mL of Fetal Bovine Serum (Sigma cat. n° F7524), 5 µg/mL Puromycin dihydrochloride (Sigma cat. n° P9620) and 1 mg/ml G418 disulfate salt (Sigma cat. n° G8168).

Cells were seeded into black-walled clear bottom 384 Multiwell plates at a density of 10.000 cells/well in complete medium. After 24 h the culture medium was replaced with 20 µL/well of Fluo-8 NoWash calcium sensitive dye (AAT Bioquest cat. n° 36316) diluted in Tyrode's buffer (Standard Tyrode's buffer: in house solution, 130 mM NaCl, 5 mM KCl, 2 mM CaCl2, 5 mM NaHCO3, 1 mM MgCl2, 20 mM HEPES, pH 7.4).

The plates were incubated for 1 hours at 37°C and injected with 10 µL/well of test compounds at 3X concentration in Tyrode's buffer with the FLIPRTETRA. After 5 minutes a second injection of 15 µL/well of 10µM α,β-Met-ATP in Tyrode's buffer (α,β-Met-ATP: from Tocris cat. n° 3209) was performed and the signal of the emitted fluorescence was recorded by FLIPRTETRA (Molecular Devices).

The antagonism for some of the compounds of interest, prepared according to the selected invention, was expressed as IC₅₀ (nM) for human P2X₃ and P2X_{2/3} receptors, and shown below in **Tables 8-13.** The selectivity is expressed as the ratio of IC₅₀ of P2X_{2/3}/ P2X₃ receptors.

**Table 8: Human P2X₃ and h-P2X_{2/3} receptor antagonist activity for selected compounds of the invention.**

| | *h*-P2X₃ | *h*-P2X_{2/3} | *Selectivity* |
|---|---|---|---|
| **Example** | **IC₅₀ nM** | **IC₅₀ nM** | |
| **1** | 703.2 | // | // |
| **2** | 1007 | // | // |
| **3** | 27.3 | // | // |
| **4** | 181.2 | // | // |
| **5** | 111.5 | 19000 | 170 |
| **6** | 33.5 | 221 | 6.5 |
| **7** | 642.1 | // | // |
| **8** | 1379 | // | // |
| **9** | 166.6 | 7410 | 44 |
| **10** | 1082 | // | // |
| **11** | 113.3 | // | // |
| **12** | 54 | // | // |
| **13** | 864.6 | // | // |
| **14** | 822.0 | // | // |
| **15** | 11770 | // | // |
| **16** | 871.7 | // | // |

**Table 9: Human P2X₃ and h-P2X_{2/3} receptor antagonist activity for selected compounds of the invention.**

| | *h*-P2X₃ | *h*-P2X_{2/3} | *Selectivity* |
|---|---|---|---|
| **Example** | **IC₅₀ nM** | **IC₅₀ nM** | |
| **17** | 41.5 | >30000 | >722 |
| **18** | 46.4 | >30000 | >646 |
| **19** | 31.2 | >30000 | >961 |

**Table 10: Human P2X₃ and h-P2X_{2/3} receptor antagonist activity for selected compounds of the invention.**

| | *h*-P2X₃ | *h*-P2X_{2/3} | *Selectivity* |
|---|---|---|---|
| **Example** | **IC₅₀ nM** | **IC₅₀ nM** | |
| **20** | 114.3 | 28500 | 249 |
| **21** | 67 | 3430 | 51 |
| **22** | 36.8 | 18800 | 510 |
| **23** | 64.7 | // | // |
| **24** | 56.2 | // | // |
| **25** | 123 | // | // |
| **26** | 115.27 | >30000 | >260 |

**Table 11: Human P2X₃ and h-P2X_{2/3} receptor antagonist activity for selected compounds of the invention.**

| **Example** | *h*-P2X₃ | *h*-P2X_{2/3} | *Selectivity* |
|---|---|---|---|
| | **IC₅₀ nM** | **IC₅₀ nM** | |
| **28** | 16.6 | >30000 | >1800 |
| **29** | 34.8 | 19700 | 566 |
| **30** | 64.3 | >30000 | >466 |
| **31** | 7.2 | 8150 | >1000 |
| **32** | 21.0 | >30000 | >1000 |
| **33** | 301 | // | // |
| **34** | 43.0 | >30000 | >697 |
| **35** | 330.0 | >30000 | >90 |
| **36** | 136.4 | >30000 | >119 |
| **37** | 338.7 | // | // |
| **38** | 50.6 | 19868 | >392 |
| **39** | 1260 | // | // |
| **40** | 491.3 | // | // |
| **41** | 9.5 | // | // |
| **42** | 1173 | // | // |
| **43** | 67.6 | >30000 | >444 |

**Table 12: Human P2X₃ and h-P2X_{2/3} receptor antagonist activity for selected compounds of the invention.**

| **Example** | *h*-P2X₃ | *h*-P2X_{2/3} | *Selectivity* |
|---|---|---|---|
| | **IC₅₀ nM** | **IC₅₀ nM** | |
| **44** | 6.73 | 3900 | 582 |
| **45** | 2554 | // | // |
| **46** | 322.1 | // | // |
| **47** | 241.9 | // | // |
| **48** | 24.9 | >30000 | >1000 |
| **49** | 19.5 | >30000 | >1500 |
| **50** | 98.6 | >30000 | >304 |
| **55** | 25 | >30000 | >1200 |

**Table 13: Human P2X₃ and h-P2X_{2/3} receptor antagonist activity for selected compounds of the invention.**

| **Example** | *h*-P2X₃ | *h*-P2X_{2/3} | *Selectivity* |
|---|---|---|---|
| | **IC₅₀ nM** | **IC₅₀ nM** | |
| **51** | 607.8 | // | // |
| **52** | 215 | // | // |
| **53** | 74. 7 | // | // |
| **54** | 24.9 | // | // |

### Statistical analysis.

The inhibition curves of the tested compounds at cloned P2X₃ receptor were determined by nonlinear regression analysis using software Prism 5.0 (Graphpad, San Diego, CA). The IC₅₀ values and pseudo-Hill slope coefficients were estimated by the program.

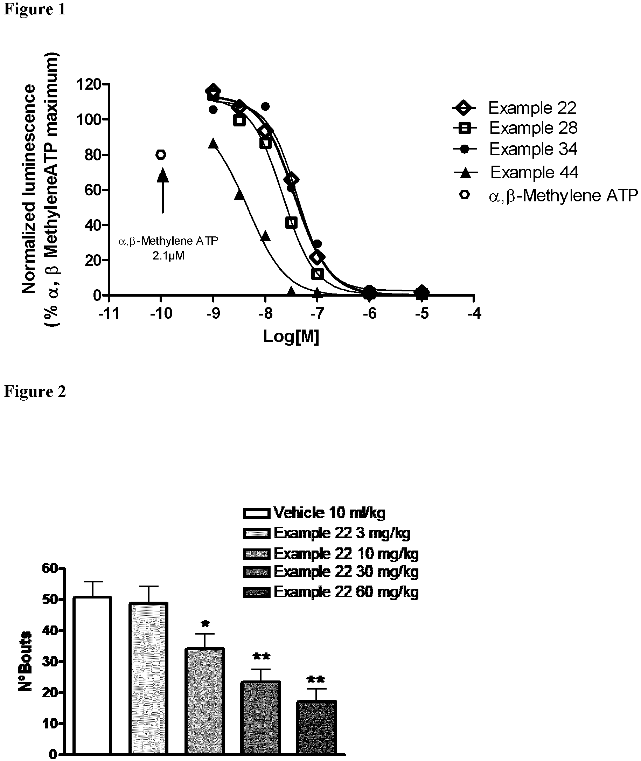

### Figure 1: Antagonism of P2X₃ receptors induced by α,β-methylene ATP.

### Example 57

### In vivo models for determination of P2X3 antagonist activities

C57BL/6J mice, Hartley Guinea pigs and Sprague-Dawley Rats from Charles River Italy were used in the experiments. Animals were housed with free access to food and water and maintained on a forced 12 hr light-dark cycle at 22-24°C for at least one week before the experiments were carried out. The animals were handled according to internationally accepted principles for care of laboratory animals (Directive 2010/63/EU of the European Parliament and of the Council of 22 September 2010).

### "Scratchng model" - Inhibition of ATP induced pruritus in mouse

Mice were shaved at the rostral back the day before injection. The day of the experiment, an intradermal injection of the irritant agent α,β-meATP 200 µg/50 µL (Sigma, Italy) into the skin was performed. The hind limb scratching behavior directed toward the injection site, was recorded. One bout was defined as the lifting of the hindlimb toward the injection site and then placing the limb back on the floor. The number of bouts were counted for a period of 30 minutes. The Example 22 compound investigated was injected intraperitoneally 30 min before the administration of the irritant agent. (**Fig. 2**)

In the scratching model the results are presented as mean ± SEM of the values. Statistical analysis was performed using one-way ANOVA and Dunnet's test for multiple comparisons. The ED35 of the tested compounds was determined by linear regression analysis of bouts (Graphpad v5).

### Figure 2: Example 22 antagonism of α,β-methylene ATP induced itch in mice "Scratching model" *p<0.05, **p<0.01vs vehicle group (One Way ANOVA, Dunnet's test)

The compound of Example 22 was able to reduce the number of bouts significantly and in a dose-related manner. The ED₃₅ values (dose of compound inhibiting the 35% of number of bouts) for Example 22, 28, 34 and 44 are reported in the following table:

**Table 14: ED₃₅ value in mice "Scratching model"**

| **Example** | ED₃₅ (mg/kg) |
|---|---|
| **22** | 10.62 |
| **34** | 12.42 |
| **44** | 9.06 |
| **28** | 22.54 |

### "Cough model" - Inhibition of citric acid induced cough in guinea pig

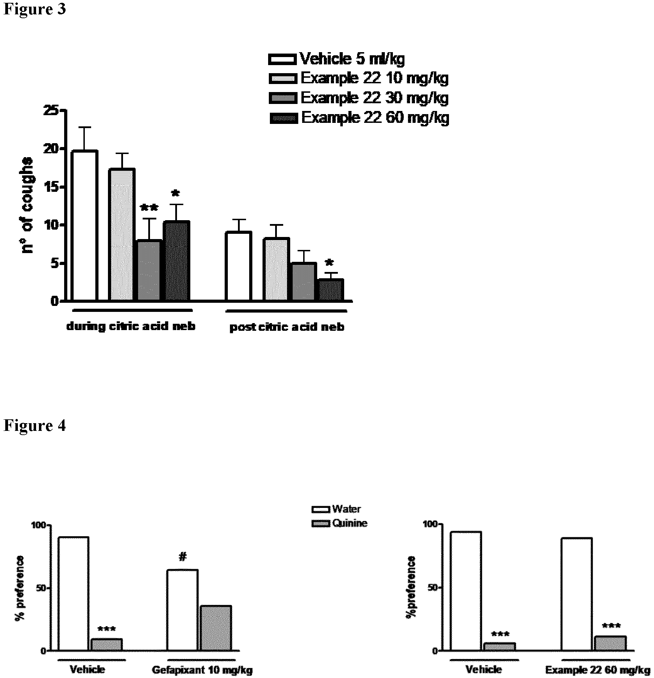

Conscious guinea pigs were placed in the transparent chamber of the whole-body plethysmograph (EMKA) and allowed to move freely. The number of coughs was registered by the IOX2 software for 10 minutes during and 10 minutes after the nebulization of citric acid 0.4 M. Example 22 was injected intraperitoneally 30 min before the nebulization of citric acid. (see **Fig. 3**)
In cough model the results are presented as mean ± SEM of the values. Statistical analysis was performed using one-way ANOVA and Dunnet's test for multiple comparisons.

**Figure 3****:** Example 22 antagonism of citric acid induced cough in guinea pig. *p<0.05, **p<0.01vs each vehicle group (One Way ANOVA, Dunnet's test)

The compound of Example 22 significantly reduced the number of coughs both during and after the nebulization of citric acid compared with vehicle. (Fig 3)
"Two bottle taste test" in rats - Dysgeusia effect of P2X3 antagonists

Rats were water fasted overnight. The day of the experiment the animals were treated with tested compounds and 30 minutes later two bottles were presented for 15 minutes, the first one containing water and the second bottle containing Quinine, a substance showing a bitter taste. The volume of liquid drunk in 15 minutes from each bottle was recorded and expressed as percentage of preference (Fig.4). Statistical analysis was performed on absolute values (mean ± SEM) using one-way ANOVA and Tukey's test for multiple comparisons.

**Figure 4****:** Two bottle taste test in rats for Gefapixant and Example 22. ***p<0.001vs each water group; #p<0.05 vs veh water group (One Way ANOVA, Tukey's test)

Animals treated with compound of Example 22 showed a clear preference for water in comparison to Quinine. On the contrary rats treated with Gefapixant, showed altered taste function and poor preference between water and Quinine bottles (Fig.4).

In formula I: each R₁ may represent hydrogen.
In formula I: each R₁ may represent hydrogen or a halogen atom.
In formula I: each R₁ may represent hydrogen or hydroxy, carbonyl, carboxyl.
In formula I: each R₁ may represent hydrogen amino or amido.
In formula I: each R₁ may represent hydrogen or C₁-C₆ alkyl or C₁-C₆ alkoxy group.
In formula I: each R₁ may represent hydrogen or mono-, bi- or tricyclic C₆-C₁₄ aryl group.
In formula I: each R₁ may represent hydrogen or optionally substituted, mono-, bi- or tricyclic. C₁-C₁₃ heterocyclic group containing from 1 to 5 N heteroatoms.
In formula I: each R₂ may represent hydrogen.
In formula I: R₂ may be absent.
In formula I: each R₂ may represent, C₁-C₆ alkyl group, C₁-C₆ alkoxy group or an C₄-C₁₄ arylalkyl group.

## Claims

1. A compound according to formula I: 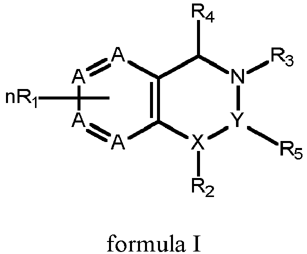 or an enantiomer, diastereomer, N-oxide, or a pharmaceutically acceptable salt or combinations thereof, wherein:
each A independently represents an atom selected from C, N, S or O;
X and Y are selected from C and N atoms, wherein the unit X-Y represents either a N-C group, or a C=N group respectively;
each R₁ independently represents hydrogen, a halogen atom, or an, optionally substituted, hydroxy, carbonyl, carboxyl, amino, amido, C₁-C₆ alkyl or C₁-C₆ alkoxy group, an, optionally substituted, mono-, bi- or tricyclic C₆-C₁₄ aryl group or an, optionally substituted, mono-, bi- or tricyclic C₁-C₁₃ heterocyclic group containing from 1 to 5 heteroatoms selected from N, O or S;
R₂ is absent or represents hydrogen or an, optionally substituted, C₁-C₆ alkyl group, C₁-C₆ alkoxy group, C₄-C₁₄ arylalkyl group, C₄-C₁₄ heteroarylalkyl group, C₃-C₇ cycloalkyl group, a mono-, bi- or tricyclic C₆-C₁₄ aryl group or a mono-, bi- or tricyclic C₁-C₁₃ heterocyclic group containing from 1 to 5 heteroatoms selected from N, O or S;
groups R₃ and R₄, or alternatively groups R₃ and R₅, are linked to each other to form a five- or six-membered heterocyclic ring containing from 2 to 3 heteroatoms atoms selected from N, O and S, optionally substituted with one or more groups nR₆, with the proviso that the remainder of R₄ or R₅ not linked with group R₃ to form the heterocyclic ring is absent, or is an atom independently selected from N, O or S which is double-bonded directly to the X-Y containing ring;
each R₆ independently represents hydrogen, a halogen atom selected from F, Cl, Br or I; or an, optionally substituted, carbonyl, oxo, C₁-C₆ alkyl, C₁-C₆ alkoxy or C₃-C₇ cycloalkyl group, an, optionally substituted, mono-, bi- or tricyclic C₆-C₁₄ aryl group or an, optionally substituted, mono-, bi- or tricyclic C₁-C₁₃ heterocyclic group containing from 1 to 5 heteroatoms selected from N, O or S or alternatively, two R₆ groups are linked to each other to form a group of the formula -(Zp)- wherein p is an integer of from 3 to 5 and each Z independently represents an oxygen atom or an optionally substituted methylene group, provided that no two adjacent Y moieties represent oxygen atoms; and
n is an integer independently selected from 0 to 3.

2. A compound according to claim 1 wherein the optional substituents are independently selected from the group consisting of halogen atoms, C₁-C₆ alkyl, C₁-C₆ alkoxy, hydroxy, mercapto, nitro, cyano, oxo, halo(C₁-C₆)alkyl, halo(C₁-C₆)alkoxy, C₁-C₆ alkylthio, C₁-C₆ alkylsulphonyl, C₁-C₆ alkylcarbonyl, sulphamoyl, C₁-C₆ alkylsulphamoyl, di(Ci-C₆)alkylsulphamoyl, (C₁-C₆)alkoxycarbonyl and (C₁-C₆)alkylcarbonyl(C₁-C₆)alkyl groups, and from groups of the formulae -NR*R*, -C(=O)-NR*R*, -D, -O-D, -C(=O)-D, -(CH₂)q-D, -NR**-D, -C(=O)-NR**-D, -NR**C(=O)-D and -O-C(=O)-D wherein each R* independently represents a hydrogen atom or a C₁-C₆ alkyl, C₁-C₆ alkoxy, C₁-C₆ alkylcarbonyl, phenyl or benzyl group, R** represents a hydrogen atom or a C₁-C₆ alkyl group, q is an integer from 1 to 6 and D represents a phenyl group or a Ci-Cs heterocyclic group containing from 1 to 3 heteroatoms selected from N, O and S; a C₁-C₆ cycloalkyl group; each group D being further optionally substituted with from 1 to 3 groups independently selected from halo, hydroxy, cyano, nitro and C₁-C₆ alkyl, preferably wherein the optional substituents are selected from the groups consisting of halogen atoms and C₁-C₆ alkyl groups.

3. A compound according to claim 1 or claim 2, wherein group X-Y represents a N-C group, groups R₃ and R₄ are linked to each other to form a five- or six-membered heterocyclic ring containing from 2 to 3 nitrogen heteroatoms atoms, optionally substituted with one or more groups nR₆, and R₅ is a carbonyl group.

4. A compound according to claim 1 or claim 2, wherein group X-Y represents a N-C group, groups R₃ and R₅ are linked to each other to form a five-membered heterocyclic ring containing from 2 to 3 nitrogen heteroatoms atoms, optionally substituted with one or more groups nR₆, and R₄ is a carbonyl group.

5. A compound according to claim 1 or claim 2, wherein group X-Y represents a C=N group, groups R₃ and R₄ are linked to each other to form a five- or six-membered heterocyclic ring containing from 2 to 3 nitrogen heteroatoms atoms, optionally substituted with one or more groups nR₆, and R₅ is absent.

6. A compound according to any preceding claim, wherein R₁ is selected from the group comprising H, Br, hydroxy, carboxyl, methoxy, methoxyethylamino, 2-hydroxyethylamino, tertiarybutoxycarbonylamino, 2-hydroxyethylaminocarbonyl, an optionally substituted azetidinyl, morpholinyl, oxetanyl, piperazinyl, piperidinyl, pyranyl or pyrrolidinyl moiety or derivative thereof, or an optionally substituted, spiro-fused bi- or tricyclic C₁-C₁₃ heterocyclic group containing from 1 to 5 heteroatoms selected from N, O or S.

7. A compound according to claim 6, wherein R₁ is selected from the group comprising 2-oxa-6-azaspiro[3.3]heptan-6-yl, 3-methoxymethylazetidin-1-yl, 3-methoxypyrrolidin-1-yl, 4-acetylpiperazin-1-yl, 4-aminopiperidin-1-yl, 4-hydroxypiperidin-1-yl, 4-hydroxypiperidin-1-yl-carbonyl, 4-methoxypiperidin-1-yl, 4-morpholinyl, dimethylaminopiperidin-1-yl, hydroxymethylpiperidin-1-yl, morpholin-4-ylcarbonyl, tetrahydro-2H-pyran-4-ylamino or tetrahydro-2H-pyran-4-ylaminocarbonyl.

8. A compound according to any preceding claim, wherein R₂ is a hydrogen atom or an optionally substituted benzyl group or derivative thereof.

9. A compound according to claim 8, wherein R₂ is a hydrogen atom, or is selected from the group comprising 3,5-dimethoxybenzyl, 4-methoxybenzyl, 4-methylbenzyl, 4-chlorobenzyl or 4-chloro-2,6-difluorobenzyl.

10. A compound according to any preceding claim, wherein R₆ is selected from the group comprising phenyl, (1-phenyl)ethyl, 1-ethyl-1H-pyrazol-3-yl, 1-ethyl-1H-pyrazol-5-yl, (tetrahydro-2H-pyran-4-yl)methyl, (tetrahydro-2H-pyran-4-yloxy)methyl, (tetrahydro-2H-pyran-4-yl)ethyl, 3,5-dimethyl-1,2oxazol-4-yl, 2-hydroxypyridin-3-yl, 2-methylpyridin-3-yl, morpholin-4-yl-carbonyl, pyridin-3-yl-methyl, oxo, methyl, ethyl, iso-propyl, tertiary-butyl, methylpropyl, 2,2-dimethylpropyl, 3-methylbutyl, 2,2,2-trifluoroethyl, methoxymethyl, methoxyethyl, (propan-2-yloxy)methyl, tertiary-butoxymethyl, prop-1-en-2-yl, propan-2-yl-acetamide, cyclopropyl, cyclobutyl, cyclohexyl, 1-methylcyclopropyl.

11. A compound according to claim 1, wherein -(Zp)- represents a group selected from -O-(CH₂)₂-O-, -O-(CH₂)₃-O-, -O-(CH₂)₂-, -O-(CH₂)₃-, -CH₂-O-CH₂- or -(CH₂)₂-O-(CH₂)₂.

12. A compound according to any preceding claim, wherein the compound has a structure in accordance with one of the following formulae 1a to 1f: 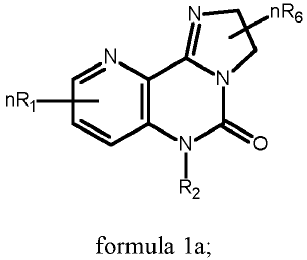 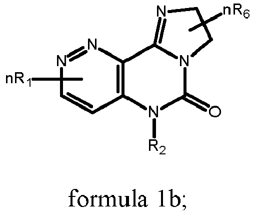 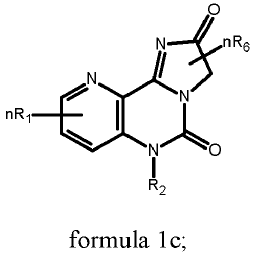 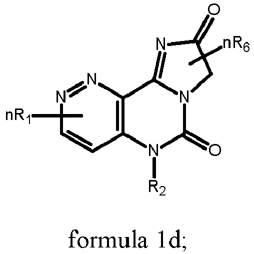 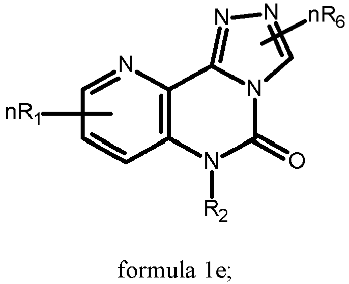 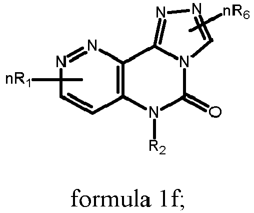 wherein groups R₁, R₂, R₆ and n are as defined in one or more preceding claim.

13. A compound according to any of claims 1 to 11, wherein the compound has a structure in accordance with one of the following formulae 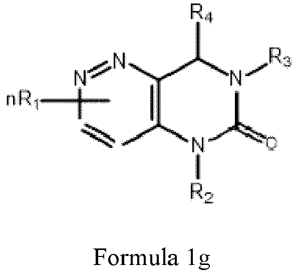 wherein groups R₁, R₂, R₃, R₄, R₅, R₆ and n are as defined in one or more preceding claim.

14. A compound according to claim 13, wherein the compound has a structure in accordance with one or more of the following formulae:

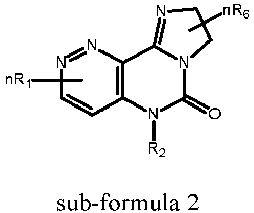

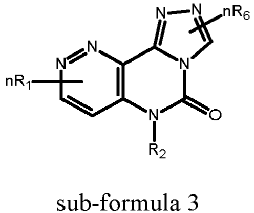

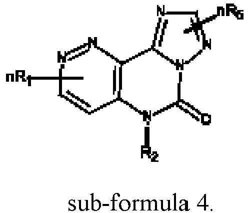

15. A compound according to claim 1, the compound being selected from the group consisting of:
6-(4-Chlorobenzyl)-8-(morpholin-4-yl)-3-(propan-2-yl)-2,6-dihydroimidazo[1,2-c]pyrido[2,3-e]pyrimidin-5(3H)-one
6-(4-Chlorobenzyl)-2,2-dimethyl-8-(morpholin-4-yl)-2,6-dihydroimidazo[1,2-c]pyrido[2,3-e]pyrimidin-5(3H)-one
2-tert-Butyl-6-(3,5-dimethoxybenzyl)-8-(morpholin-4-yl)-2,6-dihydroimidazo[1,2-c]pyrido[2,3-e]pyrimidin-5(3H)-one
2-tert-Butyl-6-(4-chlorobenzyl)-8-(morpholin-4-yl)-2,6-dihydroimidazo[1,2-c]pyrido[2,3-e]pyrimidin-5(3H)-one
6-(4-Chlorobenzyl)-2-(2-methoxypropan-2-yl)-8-(morpholin-4-yl)-2,6-dihydroimidazo[1,2-c]pyrido[2,3-e]pyrimidin-5(3H)-one
N-(5-Fluoropyridin-2-yl)-2-[8-(morpholin-4-yl)-5-oxo-2-(propan-2-yl)-2,3-dihydroimidazo[1,2-c]pyrido[2,3-e]pyrimidin-6(5H)-yl]acetamide
8-(4-Aminopiperidin-1-yl)-6-[(4-chlorophenyl)methyl]-2-(propan-2-yl)-2,6-dihydroimidazo[1,2-c]pyrido[2,3-e]pyrimidin-5(3H)-one
6-[(4-Chlorophenyl)methyl]-8-(piperazin-1-yl)-2-(propan-2-yl)-2,6-dihydroimidazo[1,2-c]pyrido[2,3-e]pyrimidin-5(3H)-one
6-[(4-Chlorophenyl)methyl]-8-(morpholin-4-yl)-2-(propan-2-yl)-2,6-dihydroimidazo[1,2-c]pyrido[2,3-e]pyrimidin-5(3H)-one
(2S)-6-(4-Chlorobenzyl)-8-(morpholin-4-yl)-2-(propan-2-yl)-2,6-dihydroimidazo[1,2-c]pyrido[2,3-e]pyrimidin-5(3H)-one
(2R)-6-(4-Chlorobenzyl)-8-(morpholin-4-yl)-2-(propan-2-yl)-2,6-dihydroimidazo[1,2-c]pyrido[2,3-e]pyrimidin-5(3H)-one
6-(4-Chlorobenzyl)-2-(1-hydroxy-2-methylpropan-2-yl)-8-(morpholin-4-yl)-2,6-dihydroimidazo[1,2-c]pyrido[2,3-e]pyrimidin-5(3H)-one
6-[(5-Chloropyridin-2-yl)methyl]-8-(morpholin-4-yl)-2-(propan-2-yl)-2,6-dihydroimidazo[1,2-c]pyrido[2,3-e]pyrimidin-5(3H)-one
6-[(5-Methoxypyridin-2-yl)methyl]-8-(morpholin-4-yl)-2-(propan-2-yl)-2,6-dihydroimidazo[1,2-c]pyrido[2,3-e]pyrimidin-5(3H)-one
6-[(4-Chlorophenyl)methyl]-8-[(1,3-dihydroxypropan-2-yl)amino]-2-(propan-2-yl)-2,6-dihydroimidazo[1.2-c]pyrido[2.3-e]pyrimidin-5(3H)-one
6-[(4-Chlorophenyl)methyl]-8-[(oxan-4-yl)amino]-2-(propan-2-yl)-2,6-dihydroimidazo[1,2-c]pyrido[2,3-e]pyrimidin-5(3H)-one
5-[(4-Chlorophenyl)methyl]-9-(1-hydroxy-2-methylpropan-2-yl)-3-(morpholin-4-yl)-8,9-dihydroimidazo[1',2':1,6]pyrimido[5,4-c]pyridazin-6(5H)-one
5-[(4-Chlorophenyl)methyl]-9-(2-methoxypropan-2-yl)-3-(morpholin-4-yl)-8,9-dihydroimidazo[1',2':1,6]pyrimido[5,4-c]pyridazin-6(5H)-one
5-[(4-Chlorophenyl)methyl]-9-(2-hydroxypropan-2-yl)-3-(morpholin-4-yl)-8,9-dihydroimidazo[1',2':1,6]pyrimido[5,4-c]pyridazin-6(5H)-one
3-tert-Butyl-6-[(4-methoxyphenyl)methyl]-8-(morpholin-4-yl)imidazo[1,2-c]pyrido[2,3-e]pyrimidine-2,5(3H,6H)-dione
3-tert-Butyl-6-[(4-chlorophenylmethyl]-8-(morpholin-4-yl)imidazo[1,2-c]pyrido[2,3-e]pyrimidine-2,5(3H,6H)-dione
6-[(4-Chlorophenyl)methyl]-8-(morpholin-4-yl)-3-(propan-2-yl)imidazo[1,2-c]pyrido[2,3-e]pyrimidine-2,5(3H,6H)-dione
(R or S)-6-[(4-Chlorophenyl)methyl]-8-(morpholin-4-yl)-3-(propan-2-yl)imidazo[1,2-c]pyrido[2,3-e]pyrimidine-2,5(3H,6H)-dione
(S or R)-6-[(4-Chlorophenyl)methyl]-8-(morpholin-4-yl)-3-(propan-2-yl)imidazo[1,2-c]pyrido[2,3-e]pyrimidine-2,5(3H,6H)-dione
6-[(4-Chlorophenyl)methyl]-8-(4-oxopiperidin-1-yl)-3-(propan-2-yl)imidazo[1,2-c]pyrido[2,3-e]pyrimidine-2,5(3H,6H)-dione
6-[(4-Chlorophenyl)methyl]-8-(4-hydroxypiperidin-1-yl)-3-(propan-2-yl)imidazo[1,2-c]pyrido[2,3-e]pyrimidine-2,5(3H,6H)-dione
5-[(4-Chlorophenyl)methyl1-8-(2-methoxypropan-2-yl)-3-(morpholin-4-yl)imidazo[1',2':1,6]pyrimido[5,4-c]pyridazine-6,9(5H,8H)-dione
5-[(4-Chlorophenyl)methyl]-3-(morpholin-4-yl)-8-(propan-2-yl)imidazo[1',2':1,6]pyrimido[5.4-c]pyridazine-6,9(5H,8H)-dione
3-tert-Butyl-6-(4-methoxybenzyl)-8-(morpholin-4-yl)pyrido[2,3-e][1,2,4]triazolo[4,3-c] pyrimidin-5 (6H) -one
3-tert-Butyl-6-[(4-chlorophenyl)methyl]-8-(morpholin-4-yl)pyrido[2,3-e][1,2,4]triazolo[4,3-c]pyrimidin-5(6H)-one
6-[(4-Bromophenyl)methyl]-3-tert-butyl-8-(morpholin-4-yl)pyrido[2,3-e][1,2,4]triazolo[4,3-c]pyrimidin-5(6H)-one
6-(4-Chlorobenzyl)-3-(2-methoxypropan-2-yl)-8-(morpholin-4-yl)pyrido[2,3-e][1,2,4]triazolo[4,3-c]pyrimidin-5(6H)-one
6-(4-Methoxybenzyl)-3-(2-methoxypropan-2-yl)-8-(morpholin-4-yl)pyrido[2,3-e][1,2,4]triazolo[4,3-c]pyrimidin-5(6H)-one
8-(4-Acetylpiperazin-1-yl)-3-tert-butyl-6-[(4-chlorophenyl)methyl]pyrido[2,3-e][1,2,4]triazolo[4,3-c]pyrimidin-5(6H)-one
3-tert-Butyl-6-[(4-methoxyphenyl)methyl]-8-(piperazin-1-yl)pyrido[2,3-e][1,2,4]triazolo[4,3-c]pyrimidin-5(6H)-one
8-(4-Acetylpiperazin-1-yl)-3-tert-butyl-6-[(4-methoxyphenyl)methyl]pyrido[2,3-e][1,2,4]triazolo[4,3-c]pyrimidin-5(6H)-one
3-tert-Butyl-6-[(4-methoxyphenyl)methyl]-8-(4-methoxypiperidin-1-yl)pyrido[2,3-e][1,2,4]triazolo[4,3-c]pyrimidin-5(6H)-one
3-tert-Butyl-6-[(4-methoxyphenyl)methyl]-8-(piperidin-1-yl)pyrido[2,3-e][1,2,4]triazolo[4,3-c]pyrimidin-5(6H)-one
6-[(4-Chlorophenyl)methyl]-8-(4-oxopiperidin-1-yl)-3-(propan-2-yl)pyrido[2,3-e][1,2,4]triazolo[4,3-c]pyrimidin-5(6H)-one
8-(4-Aminopiperidin-1-yl)-3-tert-butyl-6-[(4-methoxyphenyl)methyl]pyrido[2,3-e][1,2,4]triazolo[4,3-c]pyrimidin-5(6H)-one
3-tert-Butyl-6-[(4-chlorophenyl)methyl]-8-[4-(hydroxymethyl)piperidin-1-yl]pyrido[2,3-e][1,2,4]triazolo[4,3-c]pyrimidin-5(6H)-one
3-tert-Butyl-6-[(4-fluoro-3-methoxyphenyl)methyl]-8-(morpholin-4-yl)pyrido[2,3-e][1,2,4]triazolo[4,3-c]pyrimidin-5(6H)-one
3-tert-Butyl-6-[(4-chlorophenyl)methyl]-8-[(3R)-3-hydroxypyrrolidin-1-yl]pyrido[2,3-e][1,2,4]triazolo[4,3-c]pyrimidin-5(6H)-one
3-tert-Butyl-6-[(4-chlorophenyl)methyl1-8-(morpholin-4-yl)[1,2,4]triazolo[4',3':1,6]pyrimido[5,4-c]pyridazin-5(6H)-one
tert-Butyl 4-{6-[(4-chlorophenyl)methyl]-3-(2-methoxypropan-2-yl)-5-oxo-5,6-dihydro[1,2,4]triazolo[4',3':1,6]pyrimido[5,4-c]pyridazin-8-yl}piperazine-1-carboxylate
6-[(4-Chlorophenyl)methyl]-3-(2-methoxypropan-2-yl)-8-(piperazin-1-yl)[1,2,4]triazolo[4',3':1,6]pyrimido[5,4-c]pyridazin-5(6H)-one
8-(4-Acetylpiperazin-1-yl)-6-[(4-chlorophenyl)methyl]-3-(2-methoxypropan-2-yl)[1,2,4]triazolo[4',3':1,6]pyrimido[5,4-c]pyridazin-5(6H)-one
6-[(4-Chlorophenyl)methyl]-3-(2-methoxypropan-2-yl)-8-(morpholin-4-yl)[1,2,4]triazolo[4',3':1,6]pyrimido[5,4-c]pyridazin-5(6H)-one
6-[(4-Chlorophenyl)methyl]-3-(2,2-dimethylpropyl)-8-(morpholin-4-yl)[1,2,4]triazolo[4',3':1,6]pyrimido[5,4-c]pyridazin-5(6H)-one
6-[(4-Chlorophenyl)methyl1-3-(dimethylamino)-8-(morpholin-4-yl)[1,2,4]triazolo[4',3':1,6]pyrimido[5,4-c]pyridazin-5(6H)-one
6-[(4-Methoxyphenyl)methyl]-2-(2-methylpropyl)-8-(morpholin-4-yl)pyrido[2,3-e][1,2,4]triazolo[1,5-c]pyrimidin-5(6H)-one
2-tert-Butyl-6-[(4-chlorophenyl)methyl]-8-(3-hydroxypiperidin-1-yl)pyrido[2,3-e][1,2,4]triazolo[1,5-c]pyrimidin-5(6H)-one
9-tert-Butyl-5-[(4-chlorophenyl)methyl]-3-(4-hydroxypiperidin-1-yl)[1,2,4]triazolo[1',5':1,6]pyrimido[5,4-c]pyridazin-6(5H)-one
5-[(4-Chlorophenyl)methyl]-9-(2,2-dimethylpropyl)-3-(morpholin-4-yl)[1,2,4]triazolo[1',5':1,6]pyrimido[5,4-c]pyridazin-6(5H)-one
3-tert-Butyl-6-[(4-chlorophenyl)methyl]-8-(4-hydroxypiperidin-1-yl)[1,2,4]triazolo[4',3':1,6]pyrimido[5,4-c]pyridazin-5(6H)-one

16. A pharmaceutical composition comprising a compound according to any one of claims 1 to 12 and a pharmaceutically acceptable carrier or diluent.

17. A compound for use in the treatment or prevention of pain, chronic pain and tolerance to analgesic, respiratory disorders and dysfunctions, overactive bladder, bladder pain syndrome, dysuria and in general in genitourinary diseases, cardiovascular disorders, oesophageal hypersensitivity and more in general for the potential treatment of visceral organ diseases and disorders **characterized by** the involvement of P2X₃ and P₂X₂/₃ comprising administering to a subject in need of treatment an effective amount of a pharmaceutical composition according to claim 14 and /or
A compound for use in the treatment or prevention of itch and pruritus resulting from or associated with: atopic dermatitis, psoriasis or lupus erythematosus, or pruritus derived by viral infection or cancer (leukaemia or lymphomas) and more in general for the potential treatment of visceral organ diseases and disorders **characterized by** the involvement of P2X3 and P2X2/3 comprising administering to a subject in need of treatment an effective amount of a pharmaceutical composition according to claim 14.
